# EUROPEAN PATENT APPLICATION

(11) **EP 4 825 859 A1**
(43) Date of publication of application: **21.10.2026**
(21) Application number: 24902972.9
(22) Date of filing: 13.12.2024
(51) Int. Cl.: C07D 495/04, C07D 519/00, A61K 31/519

(54) **DEUTERATED HETEROCYCLIC COMPOUND ACTING AS PDE4B INHIBITOR AND USE THEREOF**

(30) Priority: 15.12.2023 CN 202311744747; 17.01.2024 CN 202410070286; 24.05.2024 CN 202410658874; 05.09.2024 CN 202411247213; 02.12.2024 CN 202411758940
(71) Applicant: Hubei Bio-Pharmaceutical Industrial Technological Institute Inc., Wuhan, Hubei 430075 (CN)
(72) Inventor: ZHANG, Xuejun, Wuhan, Hubei 430075 (CN); JIA, Yimin, Wuhan, Hubei 430075 (CN); LI, Jinping, Wuhan, Hubei 430075 (CN); LI, Qun, Wuhan, Hubei 430075 (CN); ZHANG, Xinli, Wuhan, Hubei 430075 (CN); TANG, Yamin, Wuhan, Hubei 430075 (CN); DENG, Jianwen, Wuhan, Hubei 430075 (CN); SONG, Xiaobo, Wuhan, Hubei 430075 (CN); SUN, Hongna, Wuhan, Hubei 430075 (CN); WANG, Junnan, Wuhan, Hubei 430075 (CN)
(74) Representative: M. Zardi & Co S.A.
(86) International application number: PCT/CN2024/139216
(87) International publication number: WO 2025/124557

(57) **Abstract**

The present disclosure belongs to the field of medicine. Specifically, the present disclosure relates to a deuterated heterocyclic compound acting as a PDE4B inhibitor and the use thereof. Specifically, the present disclosure relates to a compound represented by formula I, or a tautomer, stereoisomer, hydrate, solvate, pharmaceutically acceptable salt, or prodrug thereof.

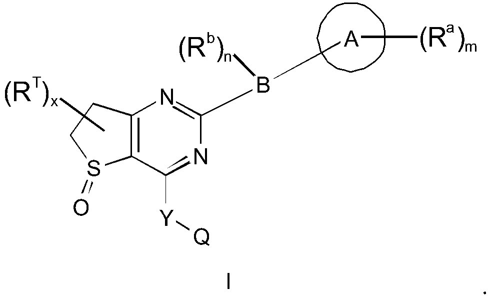

## Description

### PRIORITY INFORMATION

This application claims priority and benefits of Chinese Patent Application No. 202311744747.3, filed with China National Intellectual Property Administration on December 15, 2023; Chinese Patent Application No. 202410070286.4, filed with China National Intellectual Property Administration on January 17, 2024; Chinese Patent Application No. 202410658874.X, filed with China National Intellectual Property Administration on May 24, 2024; Chinese Patent Application No. 202411247213.4, filed with China National Intellectual Property Administration on September 05, 2024; and Chinese Patent Application No. 202411758940.7, filed with China National Intellectual Property Administration on December 02, 2024, the entire disclosures of which are incorporated herein by reference.

### FIELD

The present disclosure belongs to the field of medicine. Particularly, the present disclosure relates to a class of deuterated heterocyclic compound acting as a PDE4B inhibitor and use thereof. More particularly, the present disclosure relates to a compound represented by Formula I, or a tautomer, stereoisomer, hydrate, solvate, pharmaceutically acceptable salt or prodrug thereof. The compound exhibits favorable phosphodiesterase 4B (PDE4B) inhibitory effect.

### BACKGROUND

PDE4 is a cyclic nucleotide phosphodiesterase that is abundantly expressed in most cells and hydrolyzes cyclic adenosine monophosphate (cAMP) with a micromolar Km value. PDE4 molecules are involved in a variety of physiological processes, including brain function, mononuclear macrophage activation, neutrophil infiltration, vascular smooth muscle proliferation, and myocardial contraction. It has been reported that PDE4 serves as a target for various inflammatory diseases, such as asthma, chronic obstructive pulmonary disease (COPD), and rheumatoid arthritis. PDE4 consists of four subtypes: PDE4A, PDE4B, PDE4C and PDE4D, which are located on chromosomes 19p13.2, 1p31, 19p13.11, and 5q12, respectively. The PDE4 molecules exist in long, short and ultra-short forms based on their molecular size. An X-ray structure of the PDE4 molecule reveals that an active site of the PDE4 molecule can be divided into three sub-pockets: a divalent metal pocket that interacts with a phosphate portion of cAMP; two Q pockets that form hydrogen bonds and hydrophobic interactions with an inhibitor; and a solvation pocket (S pocket). PDE4 inhibitors occupy an active site through multiple interactions, including hydrophobic interactions with conserved phenylalanine and isoleucine, and hydrogen bonding with invariant glutamine. The high conservation and structural homology of the PDE4 catalytic domain poses a challenge for the discovery of selective inhibitors of the PDE4 subtypes.

Clinical studies of the PDE4 inhibitors have been limited by side effects, including nausea and vomiting, which are believed to result from inhibition of the PDE4D subtypes. Likewise, therapeutic indexes of second-generation PDE4 inhibitors, cilomilast and roflumilast, have also been limited by the side effects. Selective inhibition of the PDE4B subtypes may provide a way to achieve therapeutic efficacy while potentially mitigating these adverse events.

Currently, no drugs targeting a PDE4B inhibitory pathway for the treatment of numerous diseases including fibrosis have been marketed. Therefore, the development of novel compounds capable of inhibiting PDE4B activity is of positive significance for the treatment of such diseases.

### SUMMARY

An object of the present disclosure is to provide a novel compound acting as a PDE4B inhibitor.

In a first aspect, the present disclosure provides a compound represented by Formula I, or a tautomer, stereoisomer, hydrate, solvate, pharmaceutically acceptable salt or prodrug thereof: 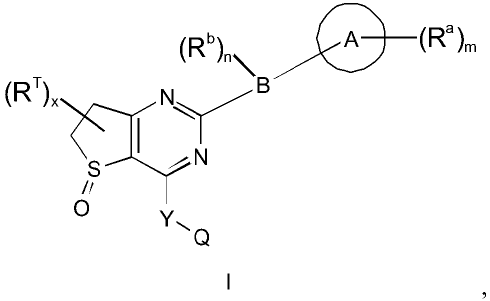 wherein:
ring A is a 5- to 10-membered aromatic ring or a 5- to 10-membered heteroaromatic ring;
each R^{T} is independently H, halogen, hydroxyl, deuterium, C₁₋₆ alkyl, C₁₋₆ alkoxy, C₃-₆ cycloalkyl, deuterated C₃-₆ cycloalkyl, deuterated C₁₋₆ alkyl, or deuterated C₁₋₆ alkoxy, wherein the C₁₋₆ alkyl, the C₁₋₆ alkoxy, the C₃-₆ cycloalkyl, the deuterated C₃-₆ cycloalkyl, the deuterated C₁₋₆ alkyl, and the deuterated C₁₋₆ alkoxy are optionally substituted with one or more substituents of halogen, hydroxyl, amino, nitro, cyano, or carbonyl, and wherein when a plurality of substituents are present, the plurality of substituents are identical or different;
B is 3- to 10-membered heterocycloalkyl, 3- to 10-membered heterocycloalkenyl, or 3- to 10-membered cycloalkyl;
R^{a} and R^{b} are each independently H, deuterium, halogen, hydroxyl, amino, nitro, cyano, carbonyl, oxo, carboxyl, C₁₋₆ alkyl, C₃₋₈ cycloalkyl, deuterated C₁₋₆ alkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, deuterated C₁₋₆ alkoxy, or 3- to 10-membered heterocycloalkyl, wherein the C₁₋₆ alkyl, the C₃₋₈ cycloalkyl, the deuterated C₁₋₆ alkyl, the C₂₋₆ alkenyl, the C₂₋₆ alkynyl, the C₁₋₆ haloalkyl, the C₃₋₈ halocycloalkyl, the C₁₋₆ hydroxyalkyl, the C₁₋₆ alkylcarbonyl, the C₁₋₆ alkoxy, and the C₁₋₆ haloalkoxy are optionally substituted with one or more R^{c}, wherein the one or more R^{c} are each deuterium, halogen, hydroxyl, amino, nitro, cyano, carbonyl, oxo, carboxyl, C₁₋₆ alkyl, deuterated C₁₋₆ alkyl, C₂₋₆ alkynyl, C₁₋₆ haloalkyl, C₁₋₆ hydroxyalkyl, C₁₋₆ alkylcarbonyl, C₁₋₆ alkoxy, or C₁₋₆ haloalkoxy, and wherein when a plurality of substituents R^{c} are present, the plurality of substituents R^{c} are identical or different;
Y is NR¹, O, S, or C(R¹)₂;
each R¹ is independently H, deuterium, C₁₋₁₀ alkyl, or C₂₋₆ alkenyl, wherein the C₁₋₁₀ alkyl and the C₂₋₆ alkenyl are optionally substituted with one or more R^{d}, wherein the one or more R^{d} are each deuterium, halogen, C₁-₃ haloalkyl, C₁₋₆ alkoxy, C₁₋₆ haloalkoxy, -COO-C₁-₆ alkyl, - C(O)NR^{f}R^{f}, 5- to 8-membered aromatic ring, -het¹, or monocyclic- or bicyclic- C₅₋₈-cycloalkyl, wherein each R^{f} is hydrogen or C₁₋₆ alkyl, and wherein het¹ represents a 5- to 8-membered monocyclic or bicyclic, saturated or unsaturated heterocyclic ring including one, two, three, or four heteroatoms independently selected from N, S and O;
Q is 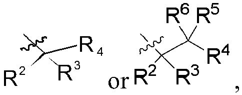 wherein:
   R² and R³ as a pair as well as R⁵ and R⁶ as a pair each independently forms, together with a carbon atom attached thereto, a saturated or partially saturated 3-, 4-, 5-, or 6-membered monocyclic ring, wherein the 3-, 4-, 5-, or 6-membered monocyclic ring includes zero, one, two, or three heteroatoms selected from N, O, or S, and, wherein the 3-, 4-, 5-, or 6-membered monocyclic ring is further substituted with g R²³, wherein the g R²³ are at least one of H, deuterium, halogen, hydroxyl, amino, cyano, carbonyl, oxo, carboxyl, C₁₋₆ alkyl, C₂₋₆ alkenyl, C₂₋₆ alkynyl, deuterated C₁₋₆ alkyl, C₁₋₆ haloalkyl, C₁₋₆ hydroxyalkyl, C₁₋₆ alkylcarbonyl, C₁₋₆ alkoxy, deuterated C₁₋₆ alkoxy, C₁₋₆ haloalkoxy, -COO-C₁₋₆ alkyl, or -C(O)NR^{f}R^{f}, wherein R^{f} is hydrogen or C₁₋₆ alkyl, or
   R⁵ and R⁶ are each independently H, deuterium, halogen, C₁₋₆ alkyl, deuterated C₁₋₆ alkyl, C₂₋₆ alkenyl, C₁₋₆ alkoxy, or deuterated C₁₋₆ alkoxy, wherein the C₁₋₆ alkyl, the deuterated C₁₋₆ alkyl, the C₂₋₆ alkenyl, the C₁₋₆ alkoxy, and the deuterated C₁₋₆ alkoxy are optionally substituted with one or more R^{d}, wherein the one or more R^{d} are each deuterium, halogen, C₁-₃ haloalkyl, C₁₋₆ alkoxy, C₁₋₆ haloalkoxy, -COO-C₁₋₆ alkyl, -C(O)NR^{f}R^{f}, -OC(O)NR^{f}R^{f}, 5- to 8-membered aromatic ring, -het², or monocyclic- or bicyclic-C₅₋₈-cycloalkyl, wherein R^{f} is hydrogen or C₁₋₆ alkyl, and wherein het² represents a 5- to 8-membered monocyclic or bicyclic, saturated or unsaturated heterocyclic ring including one, two, three, or four heteroatoms independently selected from N, S and O; and
   R⁴ is H, deuterium, halogen, hydroxyl, amino, nitro, cyano, carbonyl, oxo, carboxyl, C₁₋₆ alkyl, C₃₋₈ cycloalkyl, deuterated C₁₋₆ alkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, deuterated C₁₋₆ alkoxy, 3- to 10-membered heterocycloalkyl, wherein the C₁₋₆ alkyl, the C₃₋₈ cycloalkyl, the deuterated C₁₋₆ alkyl, the C₂₋₆ alkenyl, the C₂₋₆ alkynyl, the C₁₋₆ haloalkyl, the C₃₋₈ halocycloalkyl, the C₁₋₆ hydroxyalkyl, the C₁₋₆ alkylcarbonyl, the C₁₋₆ alkoxy, the deuterated C₁₋₆ alkoxy, the C₁₋₆ haloalkoxy, and the 3- to 10-membered heterocycloalkyl are optionally substituted with one or more R^{g}, wherein the one or more R^{g} are at least one of deuterium, halogen, hydroxyl, amino, nitro, cyano, carbonyl, oxo, carboxyl, C₁₋₆ alkyl, deuterated C₁₋₆ alkyl, C₂₋₆ alkynyl, C₁₋₆ haloalkyl, C₁₋₆ hydroxyalkyl, C₁₋₆ alkylcarbonyl, C₁₋₆ alkoxy, or C₁₋₆ haloalkoxy, and wherein when a plurality of substituents R^{g} are present, the plurality of substituents R^{g} are identical or different;
   x is 1, 2, 3, or 4;
   m and n are each independently 1, 2, 3, 4, 5, 6, 7, or 8;
   g is 1, 2, 3, 4, 5, 6, 7, or 8, and
   wherein the compound represented by Formula I satisfies the following condition:
      at least one of R^{T}, R^{a}, R^{b}, R¹, R², R³, R²³, R⁴, R⁵ , or R⁶ is deuterium, deuterated C₁₋₆ alkyl, or deuterated C₁₋₆ alkoxy.

According to an embodiment of the present disclosure, the present disclosure provides a compound represented by Formula I, or a tautomer, stereoisomer, hydrate, solvate, pharmaceutically acceptable salt or prodrug thereof: 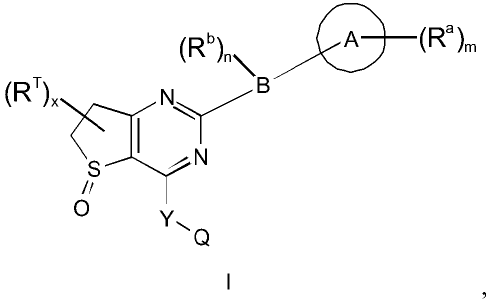 wherein:
ring A is a 5- to 10-membered aromatic ring or a 5- to 10-membered heteroaromatic ring;
each R^{T} is independently H, halogen, hydroxyl, deuterium, C₁₋₆ alkyl, C₁₋₆ alkoxy, C₃-₆ cycloalkyl, deuterated C₃-₆ cycloalkyl, deuterated C₁₋₆ alkyl, or deuterated C₁₋₆ alkoxy;
B is 3- to 10-membered heterocycloalkyl, 3- to 10-membered heterocycloalkenyl, or 3- to 10-membered cycloalkyl;
R^{a} and R^{b} are each independently H, deuterium, halogen, hydroxyl, amino, nitro, cyano, carbonyl, oxo, carboxyl, C₁₋₆ alkyl, C₃₋₈ cycloalkyl, deuterated C₁₋₆ alkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, deuterated C₁₋₆ alkoxy, or 3- to 10-membered heterocycloalkyl, wherein the C₁₋₆ alkyl, the C₃₋₈ cycloalkyl, the deuterated C₁₋₆ alkyl, the C₂₋₆ alkenyl, the C₂₋₆ alkynyl, the C₁₋₆ haloalkyl, the C₃₋₈ halocycloalkyl, the C₁₋₆ hydroxyalkyl, the C₁₋₆ alkylcarbonyl, the C₁₋₆ alkoxy, and the C₁₋₆ haloalkoxy are optionally substituted with one or more R^{c}, wherein the one or more R^{c} are each deuterium, halogen, hydroxyl, amino, nitro, cyano, carbonyl, oxo, carboxyl, C₁₋₆ alkyl, deuterated C₁₋₆ alkyl, C₂₋₆ alkynyl, C₁₋₆ haloalkyl, C₁₋₆ hydroxyalkyl, C₁₋₆ alkylcarbonyl, C₁₋₆ alkoxy, or C₁₋₆ haloalkoxy, and wherein when a plurality of substituents R^{c} are present, the plurality of substituents R^{c} are identical or different;
Y is NR¹, O, S, or C(R¹)₂;
each R¹ is independently H, deuterium, C₁₋₁₀ alkyl, or C₂₋₆ alkenyl, wherein the C₁₋₁₀ alkyl and the C₂₋₆ alkenyl are optionally substituted with one or more R^{d}, wherein the one or more R^{d} are each deuterium, halogen, C₁-₃ fluoroalkyl, C₁₋₆ alkoxy, C₁₋₆ haloalkoxy, -COO-C₁-₆ alkyl, - C(O)NR^{f}R^{f}, a 5- to 8-membered aromatic ring, -het¹, or monocyclic- or bicyclic- C₅₋₈-cycloalkyl, wherein R^{f} is hydrogen or C₁₋₆ alkyl, and wherein het¹ represents a 5- to 8-membered monocyclic or bicyclic, saturated or unsaturated heterocyclic ring including one, two, three, or four heteroatoms independently selected from N, S and O;
Q is 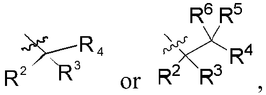 wherein:
   R² and R³ as a pair as well as R⁵ and R⁶ as a pair each independently forms, together with a carbon atom attached thereto, a saturated or partially saturated 3-, 4-, 5-, or 6-membered monocyclic ring, wherein the 3-, 4-, 5-, or 6-membered monocyclic ring includes zero, one, two, or three N atoms and zero, one, or two atoms of O or S, and, wherein the 3-, 4-, 5-, or 6-membered monocyclic ring is further substituted with g R²³, wherein the g R²³ are at least one of H, deuterium, halogen, hydroxyl, amino, cyano, carbonyl, oxo, carboxyl, C₁₋₆ alkyl, C₂₋₆ alkenyl, C₂₋₆ alkynyl, deuterated C₁₋₆ alkyl, C₁₋₆ haloalkyl, C₁₋₆ hydroxyalkyl, C₁₋₆ alkylcarbonyl, C₁₋₆ alkoxy, deuterated C₁₋₆ alkoxy, C₁₋₆ haloalkoxy, -COO-C₁₋₆ alkyl, or -C(O)NR^{f}R^{f}, wherein R^{f} is hydrogen or C₁₋₆ alkyl, or
   R⁵ and R⁶ are each independently H, deuterium, C₁₋₆ alkyl, deuterated C₁₋₆ alkyl, C₂₋₆ alkenyl, C₁₋₆ alkoxy, or deuterated C₁₋₆ alkoxy, wherein the C₁₋₆ alkyl and the C₂₋₆ alkenyl are optionally substituted with one or more R^{d}, wherein the one or more R^{d} are each deuterium, halogen, C₁-₃ fluoroalkyl, C₁₋₆ alkoxy, C₁₋₆ haloalkoxy, -COO-C₁₋₆ alkyl, -C(O)NR^{f}R^{f}, a 5- to 8-membered aromatic ring, -het², or monocyclic- or bicyclic-C₅₋₈-cycloalkyl, wherein R^{f} is hydrogen or C₁₋₆ alkyl, and wherein het² represents a 5- to 8-membered monocyclic or bicyclic, saturated or unsaturated heterocyclic ring including one, two, three, or four heteroatoms independently selected from N, S and O; and
   R⁴ is H, deuterium, halogen, hydroxyl, amino, nitro, cyano, carbonyl, oxo, carboxyl, C₁₋₆ alkyl, C₃₋₈ cycloalkyl, deuterated C₁₋₆ alkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, deuterated C₁₋₆ alkoxy, 3- to 10-membered heterocycloalkyl, wherein the C₁₋₆ alkyl, the C₃₋₈ cycloalkyl, the deuterated C₁₋₆ alkyl, the C₂₋₆ alkynyl, the C₁₋₆ haloalkyl, the C₃₋₈ halocycloalkyl, the C₁₋₆ hydroxyalkyl, the C₁₋₆ alkylcarbonyl, the C₁₋₆ alkoxy, the deuterated C₁₋₆ alkoxy, the C₁₋₆ haloalkoxy, and the 3- to 10-membered heterocycloalkyl are optionally substituted with one or more R^{g}, wherein the one or more R^{g} are at least one of deuterium, halogen, hydroxyl, amino, nitro, cyano, carbonyl, oxo, carboxyl, C₁₋₆ alkyl, deuterated C₁₋₆ alkyl, C₂₋₆ alkynyl, C₁₋₆ haloalkyl, C₁₋₆ hydroxyalkyl, C₁₋₆ alkylcarbonyl, C₁₋₆ alkoxy, or C₁₋₆ haloalkoxy, and wherein when a plurality of substituents R^{g} are present, the plurality of substituents R^{g} are identical or different;
   x is 1, 2, 3, or 4;
   m and n are each independently 1, 2, 3, 4, 5, 6, 7, or 8;
   g is 1, 2, 3, 4, 5, 6, 7, or 8, and
   wherein the compound represented by Formula I satisfies the following condition:
      at least one of R^{T}, R^{a}, R^{b}, R¹, R²³, R⁴, R⁵, or R⁶ is deuterium, deuterated C₁₋₆ alkyl, or deuterated C₁₋₆ alkoxy.

In some embodiments of the present disclosure, the ring A is selected from a 5- to 9-membered heteroaromatic ring.

In some embodiments of the present disclosure, the heteroaromatic ring includes one or two heteroatoms.

In some embodiments of the present disclosure, the heteroatom is selected from N or O.

In some embodiments of the present disclosure, the ring A is a pyridine ring, a pyrimidine ring, a pyrazine ring, a pyridazine ring, or benzoxazolyl.

In some embodiments of the present disclosure, the ring A is 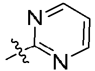 or

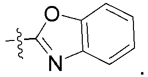

In some embodiments of the present disclosure, the structural unit 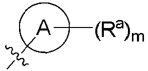 is

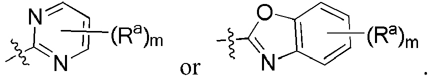

In some embodiments of the present disclosure, R^{a} is H, deuterium, F, Cl, C₁-₃ alkyl, deuterated C₁-₃ alkyl, or C₁-₃ haloalkyl.

In some embodiments of the present disclosure, R^{a} is H, deuterium, F, Cl, deuterated C₁₋₃ alkyl, or halogen-substituted C₁₋₃ alkyl,

In some embodiments of the present disclosure, R^{a} is H, deuterium, F, Cl, CH₃, CD₃, or halogen-substituted CH₃ .

In some embodiments of the present disclosure, R^{a} is H, deuterium, F, Cl, CH₃, CD₃, or F-substituted CH₃.

In some embodiments of the present disclosure, R^{a} is H, deuterium, CD₃, F, Cl, or CHF₂.

In some embodiments of the present disclosure, the structural unit 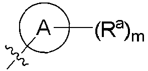 is

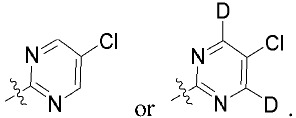

In some embodiments of the present disclosure, when Y is NR¹ or CHR¹, R¹ is selected from H, deuterium, or C₁₋₆ alkyl.

In some embodiments of the present disclosure, when Y is NR¹ or CHR¹, R¹ is selected from H or C₁₋₆ alkyl.

In some embodiments of the present disclosure, Y is -NH-.

In some embodiments of the present disclosure, B is selected from 3- to 10-membered heterocycloalkyl or 3- to 10-membered heterocycloalkenyl.

In some embodiments of the present disclosure, B is 3- to 10-membered heterocycloalkyl.

In some embodiments of the present disclosure, the 3- to 10-membered heterocycloalkyl is monocyclic, fused bicyclic, bridged bicyclic, or spiro bicyclic.

In some embodiments of the present disclosure, the 3- to 10-membered heterocycloalkyl further includes one to three heteroatoms selected from N, O, and S.

In some embodiments of the present disclosure, B is 3- to 10-membered heterocycloalkenyl.

In some embodiments of the present disclosure, the 3- to 10-membered heterocycloalkenyl is monocyclic or fused bicyclic; and/or

In some embodiments of the present disclosure, the 3- to 10-membered heterocycloalkenyl further includes one to three heteroatoms selected from N, O, and S.

In some embodiments of the present disclosure, B is 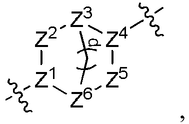 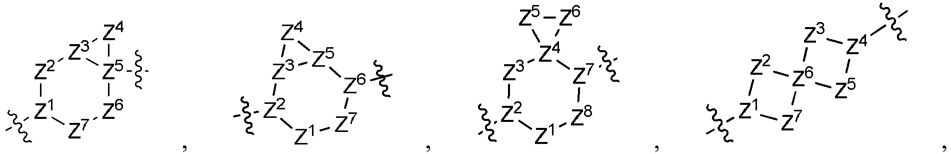 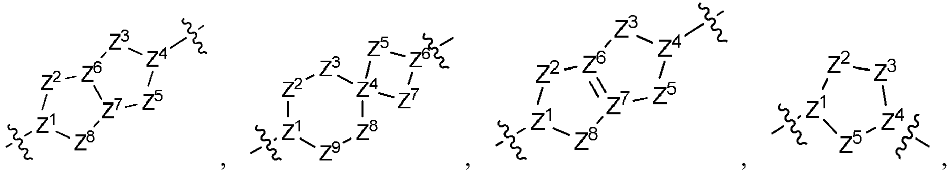 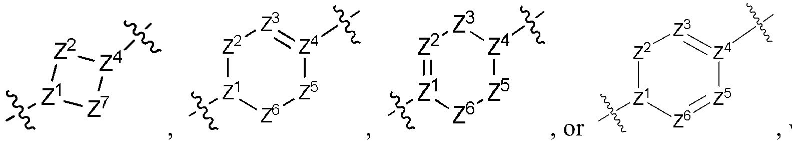 wherein
Z¹, Z², Z³, Z⁴, Z⁵, Z⁶, Z⁷, Z⁸, and Z⁹ are each independently N, NH, CH₂, CH, C, -NH-CH₂-, or -CH₂-CH₂-, and p is 0, 1, or 2.

In some embodiments of the present disclosure, 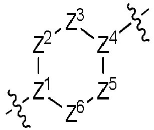 is 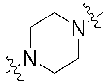 or

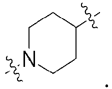

In some embodiments of the present disclosure, 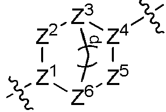 is

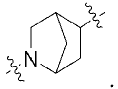

In some embodiments of the present disclosure, 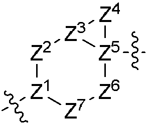 is

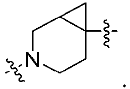

In some embodiments of the present disclosure, 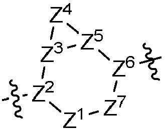 is

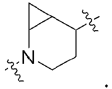

In some embodiments of the present disclosure, 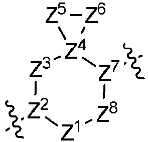 is

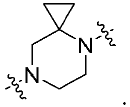

In some embodiments of the present disclosure, 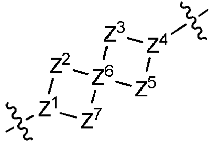 is

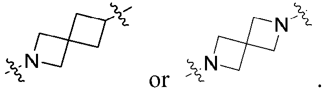

In some embodiments of the present disclosure, 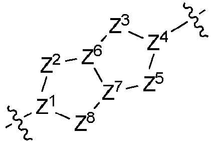 is

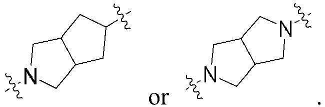

In some embodiments of the present disclosure, 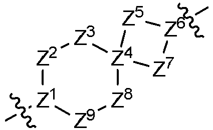 is

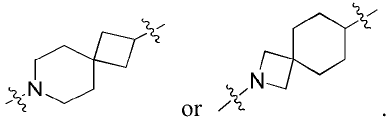

In some embodiments of the present disclosure, 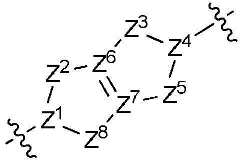 is

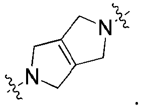

In some embodiments of the present disclosure, 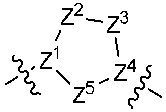 is

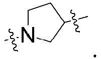

In some embodiments of the present disclosure, 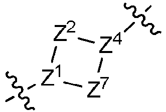 is

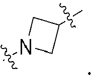

In some embodiments of the present disclosure, 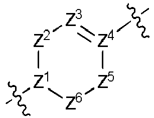 is

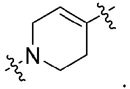

In some embodiments of the present disclosure, 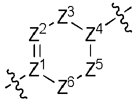 is

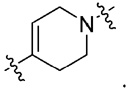

In some embodiments of the present disclosure, 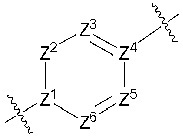 is

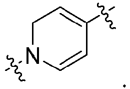

In some embodiments of the present disclosure, moiety 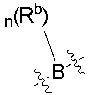 is

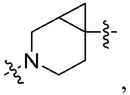

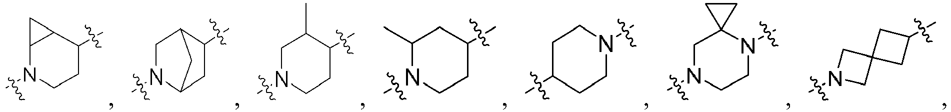

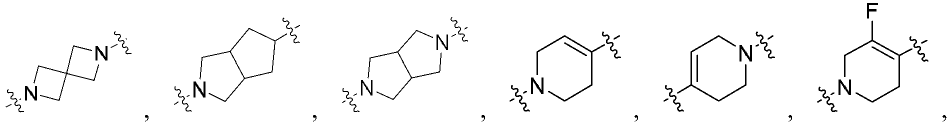

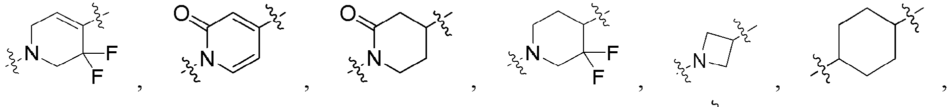

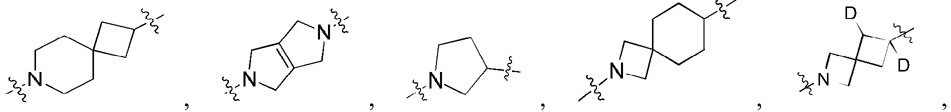

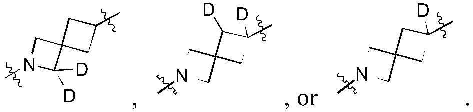

In some embodiments of the present disclosure, moiety 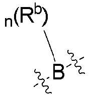 is

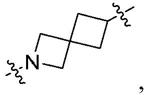

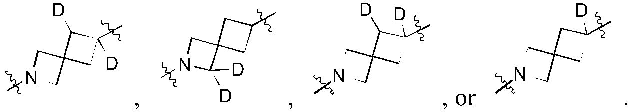

In some embodiments of the present disclosure, each R^{b} is independently H, deuterium, C₁₋₆ alkyl, deuterated C₁₋₆ alkyl, halogen, C₃₋₈ cycloalkyl, or oxo.

In some embodiments of the present disclosure, each R^{b} is independently H, deuterium, methyl, F, deuterated C₁-₃ alkyl, cycloethyl, or oxo.

In some embodiments of the present disclosure, each R^{b} is independently H, deuterium, methyl, F, cycloethyl, or oxo.

In some embodiments of the present disclosure, each R^{b} is independently H or deuterium.

In some embodiments of the present disclosure, the number of R^{c} is 0, 1, 2, or 3.

In some embodiments of the present disclosure, each R^{c} is independently deuterium, halogen, oxo, deuterated C₁₋₆ alkyl, C₁₋₆ alkyl, or C₁₋₆ haloalkyl.

In some embodiments of the present disclosure, the number of R⁸ is 0, 1, 2, or 3.

In some embodiments of the present disclosure, each R^{g} is independently deuterium, halogen, hydroxyl, cyano, deuterated C₁₋₆ alkyl, C₁₋₆ alkyl, or C₁₋₆ haloalkyl.

In some embodiments of the present disclosure, each R^{g} is independently deuterium, halogen, oxo, deuterated C₁-₃ alkyl, C₁-₃ alkyl, or C₁-₃ haloalkyl.

In some embodiments of the present disclosure, each R^{T} is independently H, deuterium, deuterated C₁-₃ alkyl, or deuterated C₁-₃ alkoxy.

In some embodiments of the present disclosure, each R^{T} is independently H or deuterium.

In some embodiments of the present disclosure, each R⁴ is independently H, deuterium, halogen, hydroxyl, amino, nitro, cyano, carboxyl, C₁₋₆ alkyl, C₃₋₈ cycloalkyl, deuterated C₁₋₆ alkyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, deuterated C₁₋₆ alkoxy, or 3- to 10-membered heterocycloalkyl, wherein the C₁₋₆ alkyl, the C₃₋₈ cycloalkyl, the deuterated C₁₋₆ alkyl, the C₁₋₆ haloalkyl, the C₃₋₈ halocycloalkyl, the C₁₋₆ hydroxyalkyl, the C₁₋₆ alkylcarbonyl, the C₁₋₆ alkoxy, the C₁₋₆ haloalkoxy, the deuterated C₁₋₆ alkoxy, and the 3- to 10-membered heterocycloalkyl are optionally substituted with one or more R^{g}, wherein the one or more R^{g} are at least one of deuterium, halogen, hydroxyl, amino, nitro, cyano, carbonyl, oxo, carboxyl, C₁₋₆ alkyl, deuterated C₁₋₆ alkyl, C₂₋₆ alkynyl, C₁₋₆ haloalkyl, C₁₋₆ hydroxyalkyl, C₁₋₆ alkylcarbonyl, C₁₋₆ alkoxy, or C₁₋₆ haloalkoxy, and wherein when a plurality of substituents R^{g} are present, the plurality of substituents R^{g} are identical or different.

In some embodiments of the present disclosure, each R⁴ is independently H, deuterium, hydroxyl, C₁₋₆ alkyl, C₃₋₈ cycloalkyl, deuterated C₁₋₆ alkyl, C₁₋₆ haloalkyl, C₃₋₈ halocycloalkyl, or C₁₋₆ hydroxyalkyl.

In some embodiments of the present disclosure, each R⁴ is independently H, deuterium, or hydroxyl.

In some embodiments of the present disclosure, R⁵ and R⁶ are each independently H, deuterium, C₁₋₆ alkyl, deuterated C₁₋₆ alkyl, C₂₋₆ alkenyl, C₁₋₆ alkoxy, or deuterated C₁₋₆ alkoxy, wherein the C₁₋₆ alkyl and the C₂₋₆ alkenyl are optionally substituted with one or more R^{d}, wherein the one or more R^{d} are each deuterium, halogen, C₁-₃ fluoroalkyl, C₁₋₆ alkoxy, C₁₋₆ haloalkoxy, or -COO-C₁₋₆ alkyl.

In some embodiments of the present disclosure, R⁵ and R⁶ are each independently H, deuterium, C₁₋₆ alkyl, or deuterated C₁₋₆ alkyl.

In some embodiments of the present disclosure, R⁵ and R⁶ are each independently H or deuterium.

In some embodiments of the present disclosure, Q is 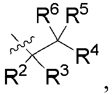 and R⁴ is hydroxyl.

In some embodiments of the present disclosure, Q is

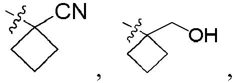

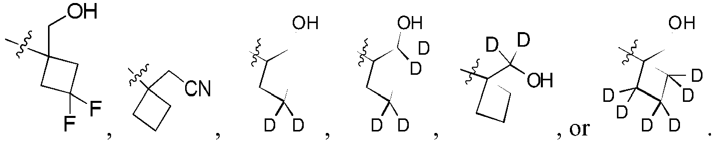

In some embodiments of the present disclosure, Q is

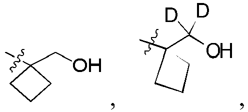

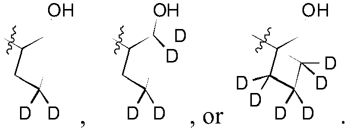

In some embodiments of the present disclosure, 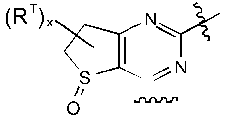 is 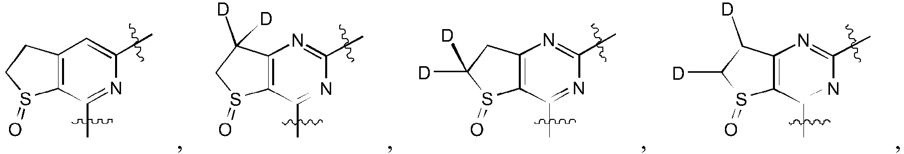 or

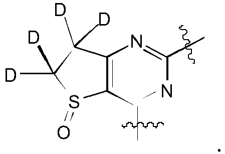

In some embodiments of the present disclosure, the compound has a structure represented by Formula I-A or Formula I-B: 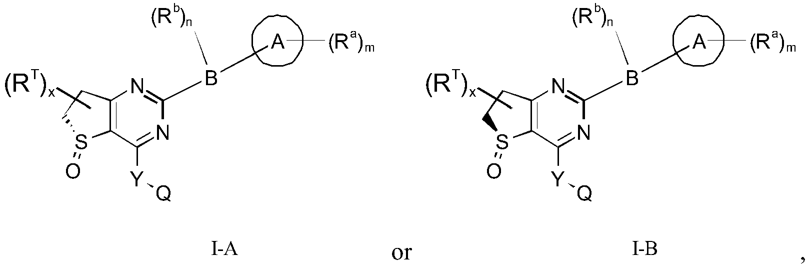 wherein ring A, B, R^{a}, R^{b}, R^{T}, Y, Q, m, n, and x are as defined in the present disclosure.

In some embodiments of the present disclosure, the compound has a structure represented by Formula I-1: 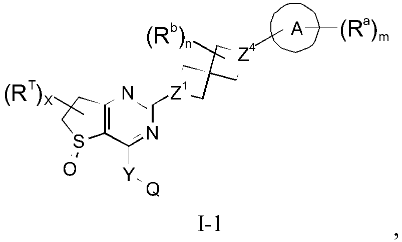 wherein:
Z¹ and Z⁴ are independently CH or N; and
ring A, R^{a}, R^{b}, R^{T}, Y, Q, m, n, and x are as defined in the present disclosure.

In some embodiments of the present disclosure, the compound has a structure represented by Formula I-1A: 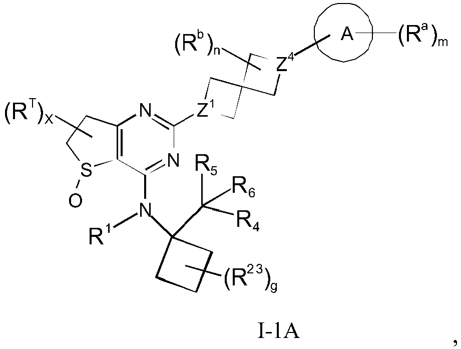 wherein:
Z¹ and Z² are independently CH or N; and
ring A, R^{a}, R^{b}, R^{T}, R¹, R²³, R⁴, R⁵, R⁶, m, n, and x are as defined in the present disclosure.

In some embodiments of the present disclosure, the compound has a structure represented by Formula I-1B: 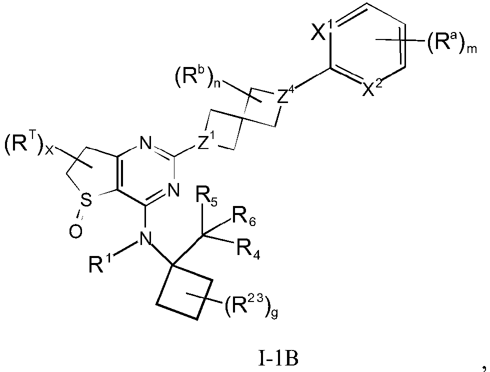 wherein:
X¹ and X² are each independently CH or N;
Z¹ and Z⁴ are each independently N or CH; and
R^{a}, R^{b}, R^{T}, R¹, R²³, R⁴, R⁵, R⁶, m, n, g, and x are as defined in the present disclosure.

In some embodiments of the present disclosure, in the structures represented by Formula I-1, Formula I-1A, or Formula I-1B, Z¹ is N, and Z⁴ is CH.

In some embodiments of the present disclosure, the compound has a structure represented by Formula I-1B1, Formula I-1B2, or Formula I-1B3: 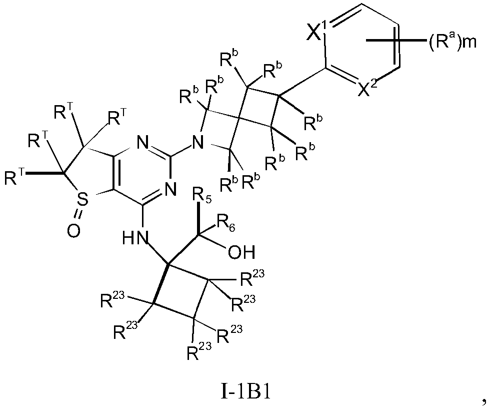 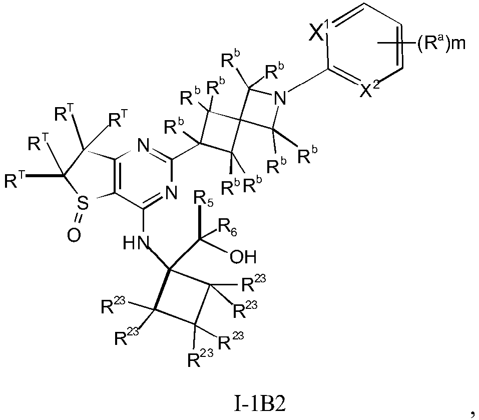 or 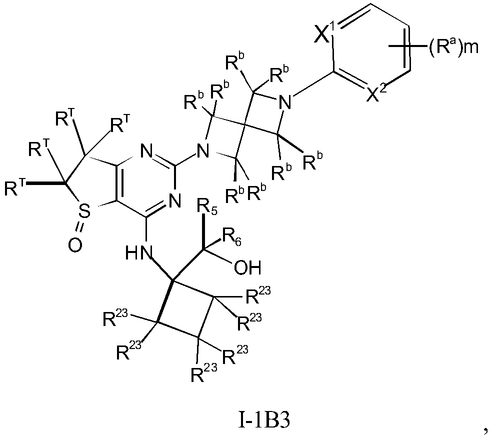 wherein:
X¹ and X² are each independently CH or N;
each R^{a} is independently H, halogen, hydroxyl, deuterium, C₁₋₆ alkyl, C₁₋₆ alkoxy, deuterated C₁₋₆ alkyl, or deuterated C₁₋₆ alkoxy;
each R^{T} is independently H, halogen, hydroxyl, deuterium, C₁₋₆ alkyl, C₁₋₆ alkoxy, deuterated C₁₋₆ alkyl, or deuterated C₁₋₆ alkoxy;
each R²³ is independently H, halogen, hydroxyl, deuterium, C₁₋₆ alkyl, C₁₋₆ alkoxy, deuterated C₁₋₆ alkyl, or deuterated C₁₋₆ alkoxy;
R⁵ and R⁶ are each independently H, deuterium, halogen, C₁₋₆ alkyl, deuterated C₁₋₆ alkyl, C₁₋₆ alkoxy, or deuterated C₁₋₆ alkoxy; and
each R^{b} is independently H, halogen, hydroxyl, deuterium, C₁₋₆ alkyl, C₁₋₆ alkoxy, deuterated C₁₋₆ alkyl, or deuterated C₁₋₆ alkoxy,
wherein at least one of R^{T}, R^{a}, R^{b}, R²³, R⁵, or R⁶ is deuterium, deuterated C₁₋₆ alkyl, or deuterated C₁₋₆ alkoxy.

In some embodiments of the present disclosure, in the structure represented by Formula I-1B1, Formula I-1B2, or Formula I-1B3, X¹ and X² are both N.

In some embodiments of the present disclosure, the compound has a structure represented by Formula I-1B4' or I-1B5': 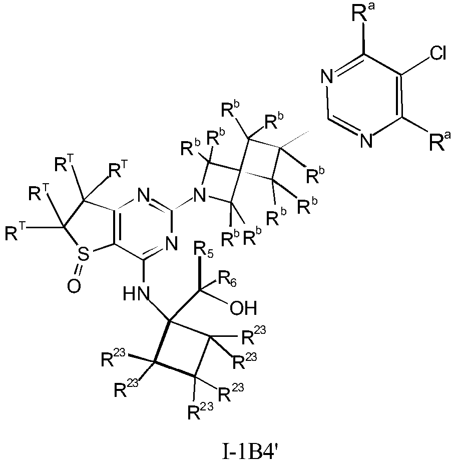 or 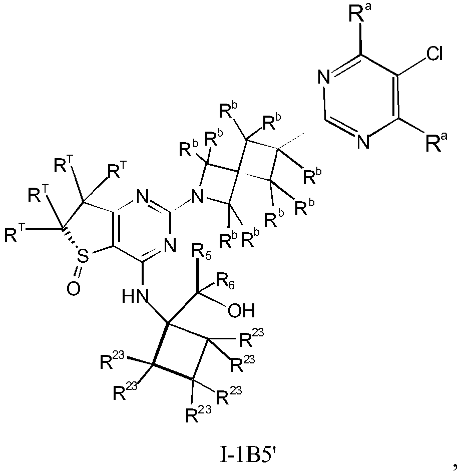 wherein:
each R^{a} is independently H, halogen, hydroxyl, deuterium, C₁₋₆ alkyl, C₁₋₆ alkoxy, deuterated C₁₋₆ alkyl, or deuterated C₁₋₆ alkoxy;
each R^{T} is independently H, halogen, hydroxyl, deuterium, C₁₋₆ alkyl, C₁₋₆ alkoxy, deuterated C₁₋₆ alkyl, or deuterated C₁₋₆ alkoxy;
each R²³ is independently H, halogen, hydroxyl, deuterium, C₁₋₆ alkyl, C₁₋₆ alkoxy, deuterated C₁₋₆ alkyl, or deuterated C₁₋₆ alkoxy;
R⁵ and R⁶ are each independently H, deuterium, halogen, C₁₋₆ alkyl, deuterated C₁₋₆ alkyl, C₁₋₆ alkoxy, deuterated C₁₋₆ alkoxy; and
each R^{b} is independently H, halogen, hydroxyl, deuterium, C₁₋₆ alkyl, C₁₋₆ alkoxy, deuterated C₁₋₆ alkyl, or deuterated C₁₋₆ alkoxy,
wherein at least one of R^{T}, R^{a}, R^{b}, R²³, R⁵, or R⁶ is deuterium, deuterated C₁₋₆ alkyl, or deuterated C₁₋₆ alkoxy.

In some embodiments of the present disclosure, the compound has a structure represented by Formula I-1B4: 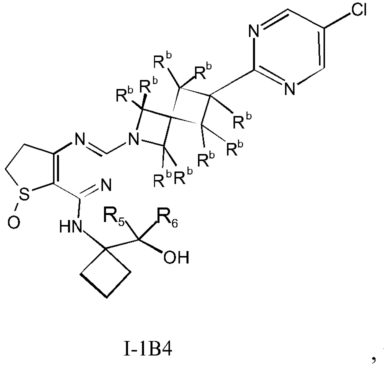 wherein:
R⁵ and R⁶ are each independently H, deuterium, halogen, C₁₋₆ alkyl, deuterated C₁₋₆ alkyl, C₁₋₆ alkoxy, or deuterated C₁₋₆ alkoxy; and
each R^{b} is independently H, halogen, hydroxyl, deuterium, C₁₋₆ alkyl, C₁₋₆ alkoxy, deuterated C₁₋₆ alkyl, or deuterated C₁₋₆ alkoxy,
wherein at least one of R^{b}, R⁵, and R⁶ is deuterium, deuterated C₁₋₆ alkyl, or deuterated C₁₋₆ alkoxy.

In some embodiments of the present disclosure, the compound has a structure represented by 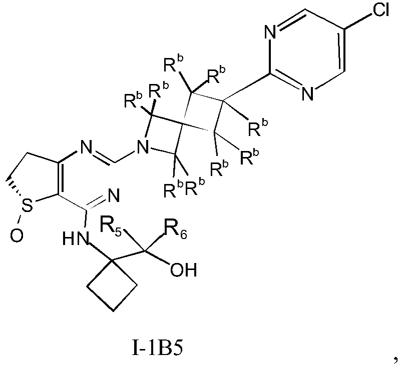 wherein:
R⁵ and R⁶ are each independently H, deuterium, halogen, C₁₋₆ alkyl, deuterated C₁₋₆ alkyl, C₁₋₆ alkoxy, or deuterated C₁₋₆ alkoxy; and
each R^{b} is independently H, halogen, hydroxyl, deuterium, C₁₋₆ alkyl, C₁₋₆ alkoxy, deuterated C₁₋₆ alkyl, or deuterated C₁₋₆ alkoxy,
wherein at least one of R^{b}, R⁵, and R⁶ is deuterium, deuterated C₁₋₆ alkyl, or deuterated C₁₋₆ alkoxy.

In some embodiments of the present disclosure, each R^{T} is independently H or deuterium.

In some embodiments of the present disclosure, at least one of R^{T} substituents is deuterium, and the remaining of R^{T} substituents are each H.

In some embodiments of the present disclosure, each R²³ is independently H or deuterium.

In some embodiments of the present disclosure, at least one of R²³ substituents is deuterium, and the remaining of R²³ substituents are each H.

In some embodiments of the present disclosure, R⁵ and R⁶ are independently H or deuterium.

In some embodiments of the present disclosure, at least one of R⁵ and R⁶ is deuterium, and the other one of R⁵ and R⁶ is H or deuterium.

In some embodiments of the present disclosure, R⁵ is H.

In some embodiments of the present disclosure, R⁵ is deuterium.

In some embodiments of the present disclosure, R⁶ is H.

In some embodiments of the present disclosure, R⁶ is deuterium.

In some embodiments of the present disclosure, each R^{b} is independently H or deuterium.

In some embodiments of the present disclosure, at least one of R^{b} is deuterium, and the remainder of R^{b} are each H.

In some embodiments of the present disclosure, each R^{a} is independently H, deuterium, Cl or F.

In some embodiments of the present disclosure, at least one of R^{a} is deuterium, and the remainder of R^{a} are each H, Cl, or F.

In some embodiments of the present disclosure, the compound has any one of the following structures: 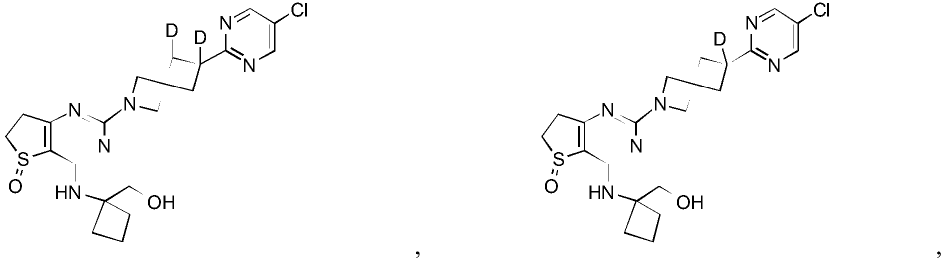 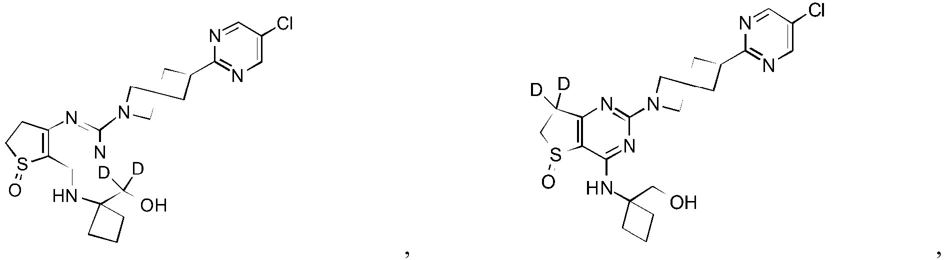 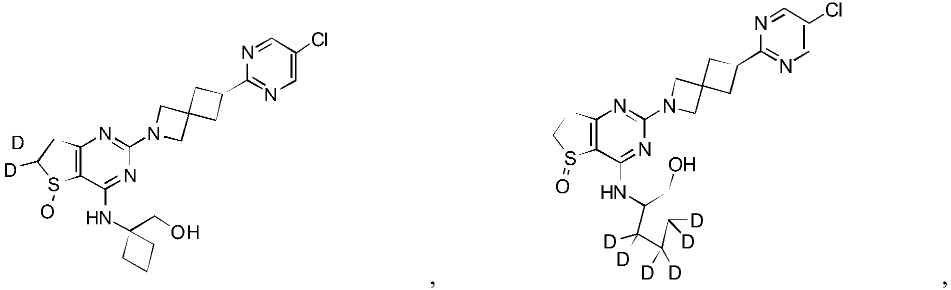 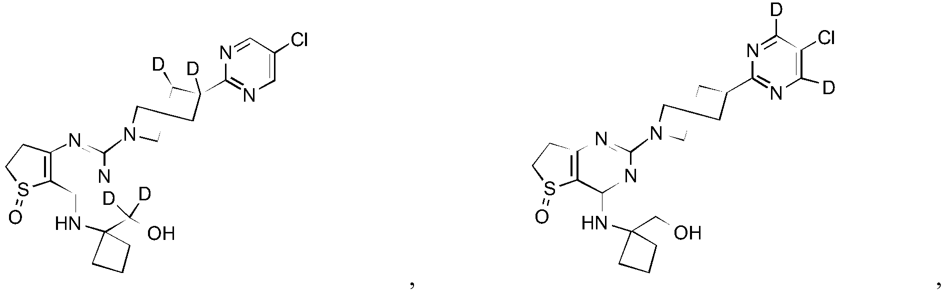 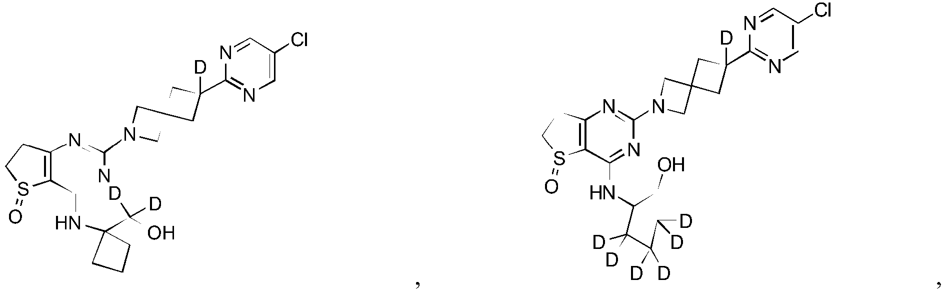 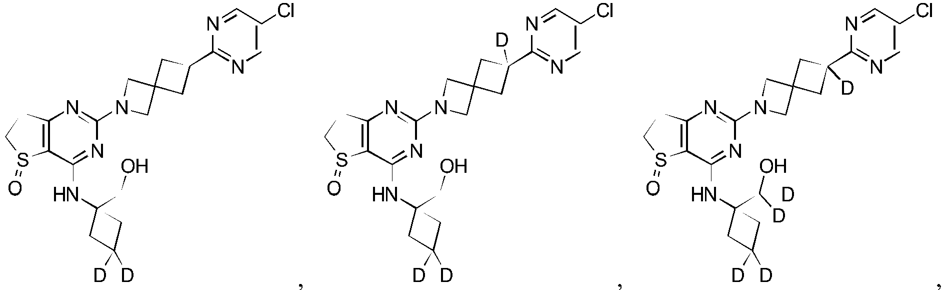 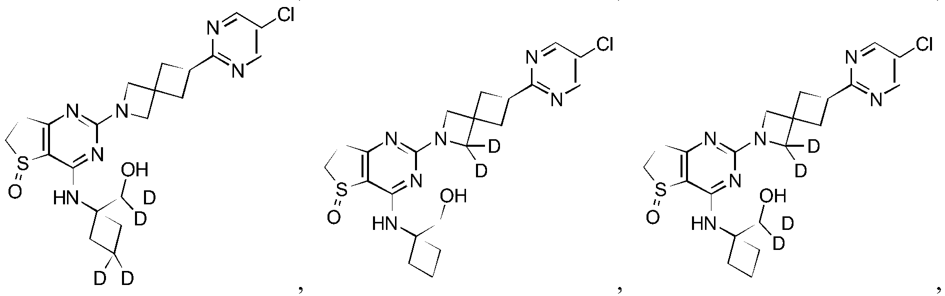 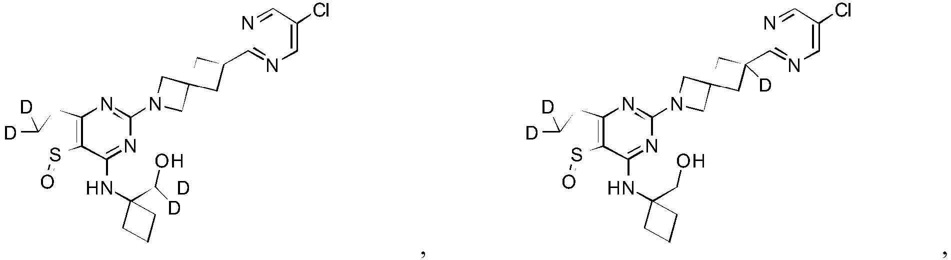 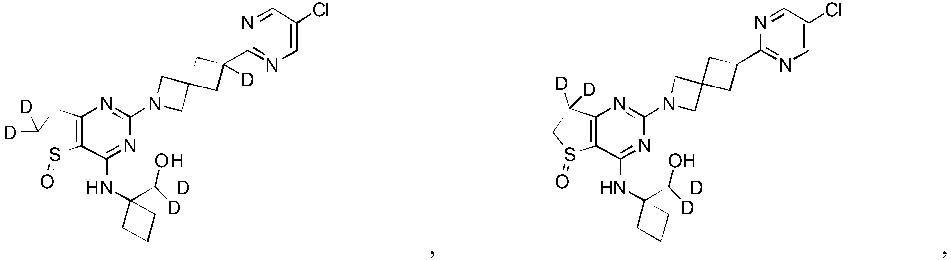 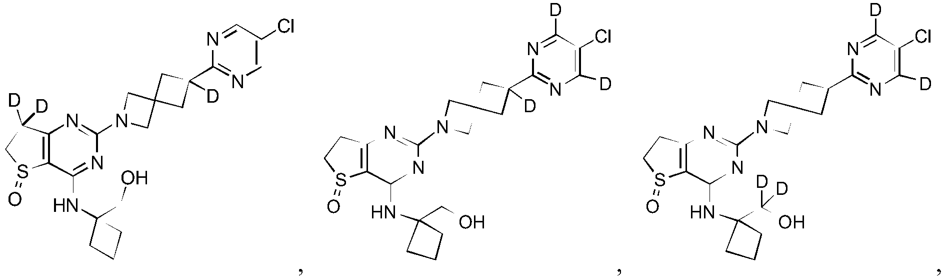 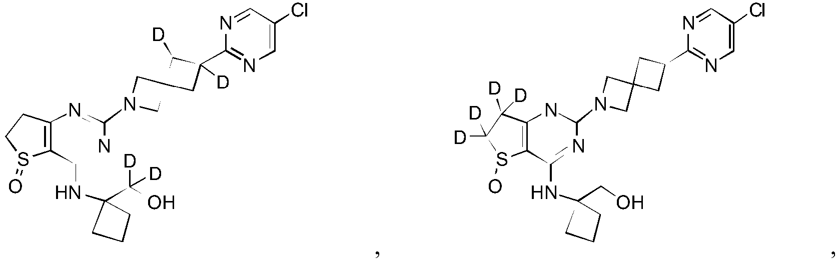 and

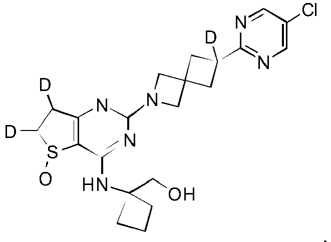

In some embodiments of the present disclosure, the compound has any one of the following structures:

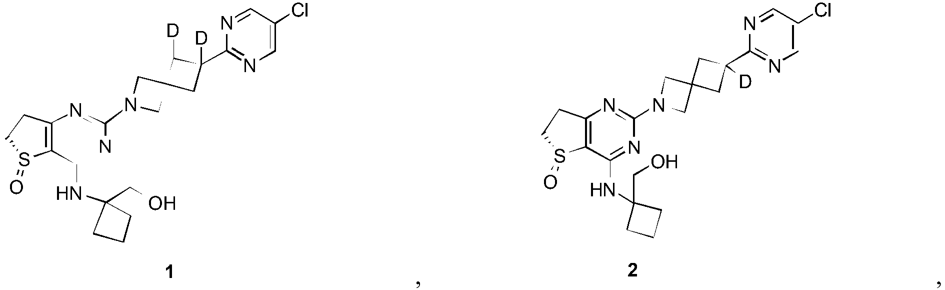

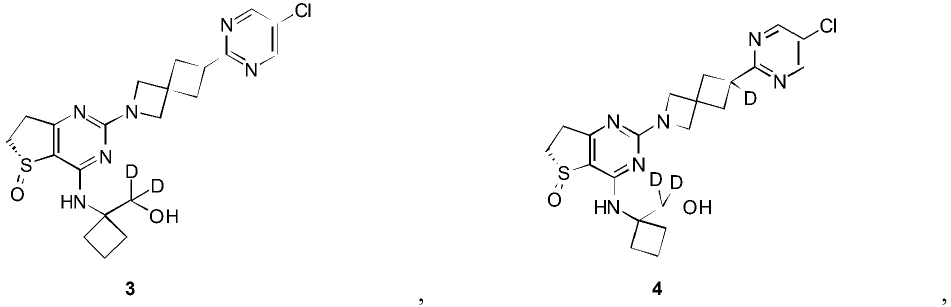

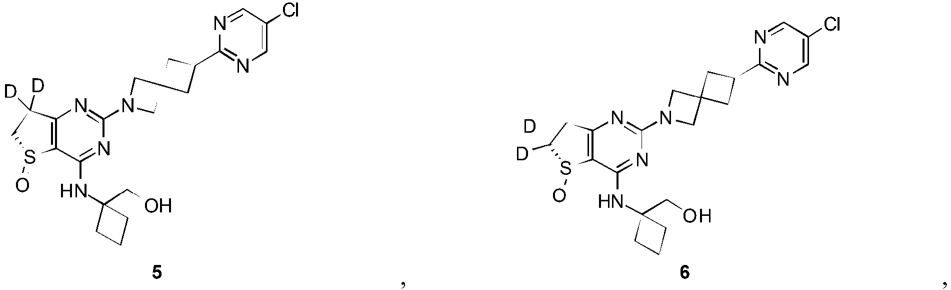

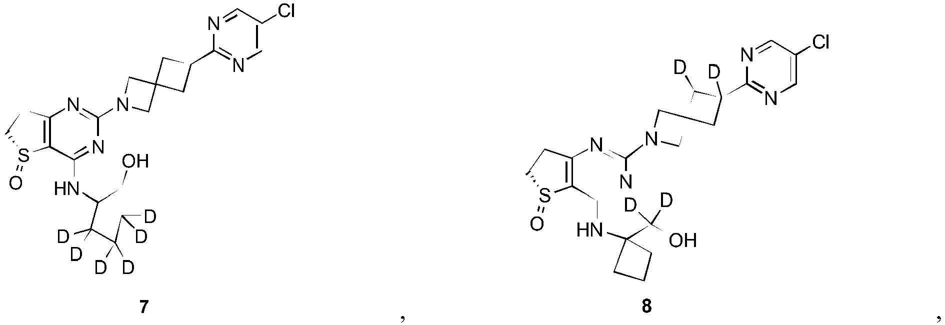

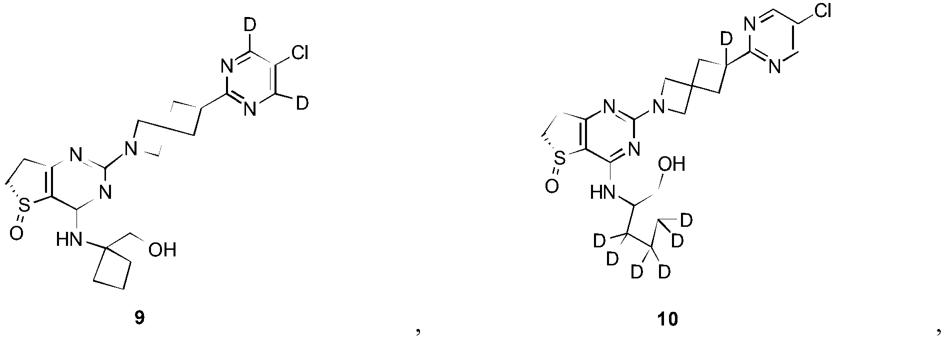

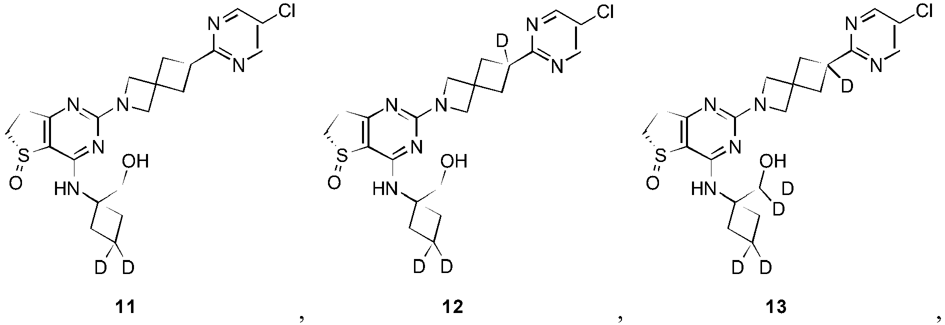

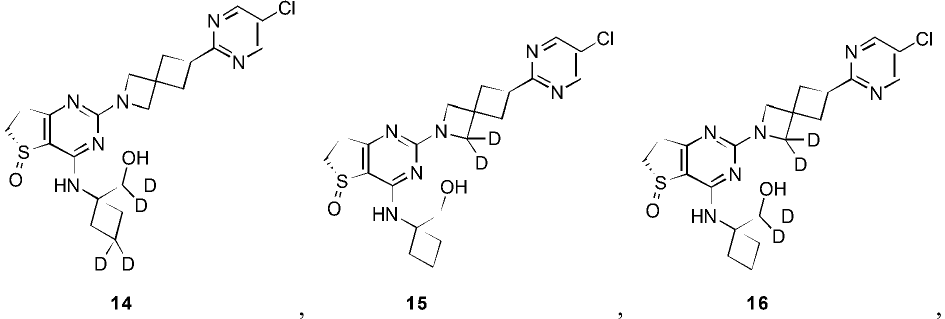

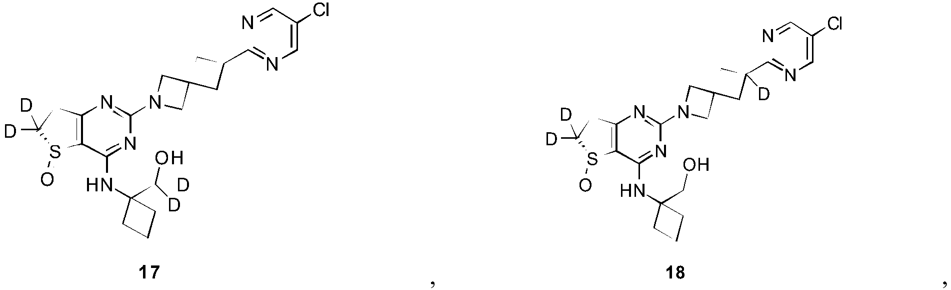

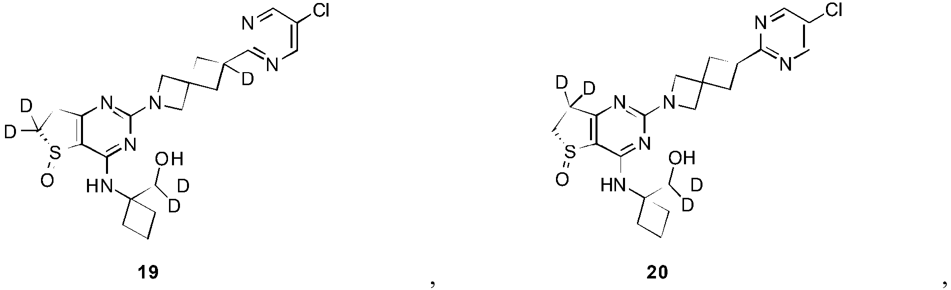

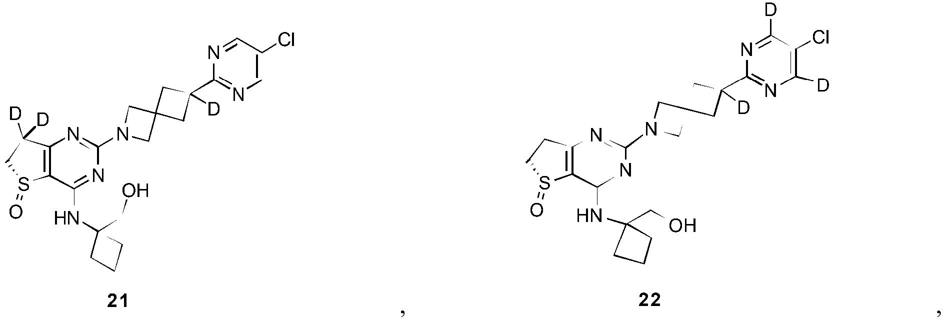

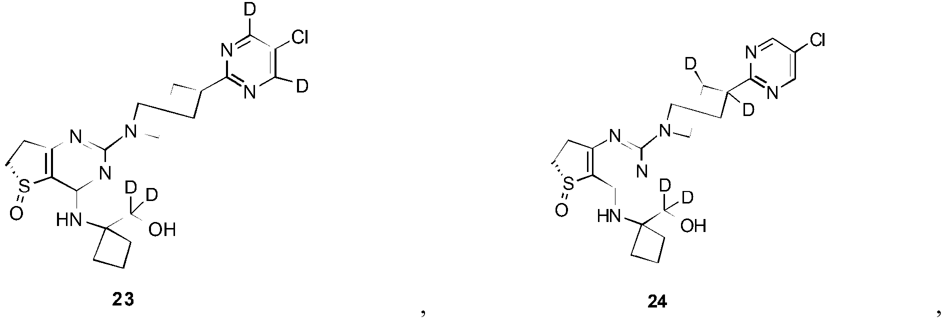

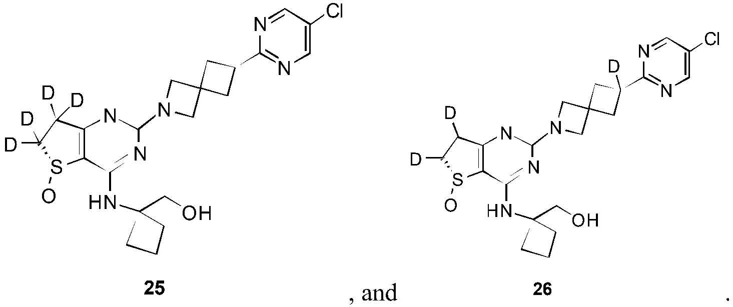

In some embodiments of the present disclosure, the compound represented by Formula I may not be any one of Compound 1 to Compound 26.

In a second aspect, the present disclosure provides a pharmaceutical composition. The pharmaceutical composition includes the compound, or the tautomer, stereoisomer, hydrate, solvate, pharmaceutically acceptable salt or prodrug thereof according to the first aspect of the present disclosure.

In some embodiments of the present disclosure, the pharmaceutical composition further includes a pharmaceutically acceptable carrier or an excipient.

In a second aspect, the present disclosure provides a pharmaceutical composition. The pharmaceutical composition includes the compound represented by Formula I, or the tautomer, stereoisomer, hydrate, solvate, pharmaceutically acceptable salt or prodrug thereof according to the first aspect of the present disclosure; and a pharmaceutically acceptable carrier.

In a third aspect, the present disclosure provides use of the compound, or the tautomer, stereoisomer, hydrate, solvate, pharmaceutically acceptable salt or prodrug thereof according to the first aspect of the present disclosure, or the pharmaceutical composition according to the second aspect of the present disclosure, for inhibiting PDE4B, and/or preventing and/or treating a PDE4B-related disease, and/or avoiding or reducing a gastrointestinal side effect in the treatment of the PDE4B-related disease.

In a fourth aspect, the present disclosure provides use of the compound, or the tautomer, stereoisomer, hydrate, solvate, pharmaceutically acceptable salt or prodrug thereof according to the first aspect of the present disclosure, or the pharmaceutical composition according to the second aspect of the present disclosure, in the preparation of a medicament or formulation for inhibiting PDE4B, and/or preventing and/or treating a PDE4B-related disease.

In a fifth aspect, the present disclosure provides use of the compound, or the tautomer, stereoisomer, hydrate, solvate, pharmaceutically acceptable salt or prodrug thereof according to the first aspect of the present disclosure, or the pharmaceutical composition according to the second aspect of the present disclosure, in the preparation of a medicament or formulation for avoiding or reducing a gastrointestinal side effect in the treatment of a PDE4B-related disease.

In a sixth aspect, the present disclosure provides use of the compound represented by Formula I, or the tautomer, stereoisomer, hydrate, solvate, pharmaceutically acceptable salt or prodrug thereof according to the first aspect of the present disclosure, or the pharmaceutical composition according to the second aspect of the present disclosure. The use includes: inhibiting PDE4B; and/or preventing and/or treating a PDE4B-related disease; and/or preparing a medicament, pharmaceutical composition, or formulation for inhibiting PDE4B, and/or preventing and/or treating a PDE4B-related disease.

In a seventh aspect, the present disclosure provides the compound, or the tautomer, stereoisomer, hydrate, solvate, pharmaceutically acceptable salt or prodrug thereof according to the first aspect of the present disclosure, or the pharmaceutical composition according to the second aspect of the present disclosure, for use in inhibiting PDE4B, and/or preventing and/or treating a PDE4B-related disease, and/or avoiding or reducing a gastrointestinal side effect in the treatment of a PDE4B-related disease.

In an eighth aspect, the present disclosure provides a method for inhibiting PDE4B, or preventing and/or treating a PDE4B-related disease, the method including: administering to a subject in need thereof the compound represented by Formula I, or the tautomer, stereoisomer, hydrate, solvate, pharmaceutically acceptable salt or prodrug thereof according to the first aspect of the present disclosure.

In a ninth aspect, the present invention provides a method for inhibiting PDE4B, and/or preventing and/or treating a PDE4B-related disease, and/or avoiding or reducing a gastrointestinal side effect in the treatment of a PDE4B-related disease, the method including: administering to a subject in need thereof the compound represented by Formula I, or the tautomer, stereoisomer, hydrate, solvate, pharmaceutically acceptable salt or prodrug thereof according to the first aspect of the present disclosure.

According to an embodiment of the present disclosure, in the uses or methods according to the third, fourth, fifth, sixth, seventh, eighth and ninth aspects described above, the PDE4B-related disease includes a respiratory disease, a gastrointestinal disease, an inflammatory disease of joints, skin, or eyes, a cancer, a peripheral or central nervous system disease, an autoimmune disease (such as a diffuse connective tissue disease such as systemic lupus erythematosus, rheumatoid arthritis, multiple sclerosis, dermatomyositis, polymyositis, vasculitis, or Sjögren's syndrome), a transplant rejection, or a disease related to smooth muscle contractility.

Preferably, the respiratory disease is a respiratory or pulmonary disease accompanied by increased mucus production, airway inflammation, and/or obstructive disease.

Preferably, the respiratory disease is COPD, idiopathic pulmonary fibrosis, interstitial lung disease, α1-antitrypsin deficiency, chronic sinusitis, asthma, or chronic bronchitis.

Preferably, the gastrointestinal disease is regional ileitis, ulcerative colitis, or Crohn's disease.

Preferably, the inflammatory disease of the joints, skin, or eyes is rheumatoid arthritis, sarcoidosis, dry eye syndrome, or glaucoma.

Preferably, the cancer is mesothelioma, neuroblastoma, rectal cancer, colon cancer, familial adenomatous polyposis, hereditary non-polyposis colorectal cancer, esophageal cancer, lip cancer, laryngeal cancer, hypopharyngeal cancer, tongue cancer, salivary gland cancer, gastric cancer, adenocarcinoma, medullary thyroid carcinoma, papillary thyroid carcinoma, renal cancer, renal parenchymal cancer, ovarian cancer, cervical cancer, uterine cancer, endometrial cancer, choriocarcinoma, pancreatic cancer, prostate cancer, bladder cancer, testicular cancer, breast cancer, urological cancer, melanoma, brain tumor, lymphoma, head- and-neck, acute lymphoblastic leukemia, chronic lymphocytic leukemia, acute myeloid leukemia, chronic myeloid leukemia, hepatocellular carcinoma, gallbladder cancer, bronchial carcinoma, small cell lung cancer, non-small cell lung cancer, multiple myeloma, basal sarcoma, teratoma, retinoblastoma, choroidal melanoma, seminoma, rhabdomyosarcoma, osteosarcoma, chondrosarcoma, myoma, liposarcoma, fibrosarcoma, Ewing's sarcoma, and plasmacytoma.

Preferably, the peripheral or central nervous system disease is depression, bipolar depression or manic depression, acute and chronic anxiety states, schizophrenia, Alzheimer's disease, Parkinson's disease, acute and chronic multiple sclerosis or acute and chronic pain, and brain damage caused by stroke, hypoxia, or cranio-cerebral trauma.

Additional aspects and advantages of the embodiments of the present disclosure will be provided at least in part in the following description, or will become apparent in part from the following description, or can be learned from the practice of the embodiments of the present disclosure.

### Terms and Definitions

Unless otherwise specified, the definitions of groups and terms recited in the specification and claims including illustrative definitions, exemplary definitions, preferred definitions, definitions recited in tables, and definitions of specific compounds in the examples, etc., may be arbitrarily combined and integrated with one another. The group definitions and compound structures resulting from such combinations and integrations should fall within the scope of the present disclosure.

Unless defined otherwise, all technical and scientific terms herein have the same meaning as commonly understood by one of ordinary skill in the art to which the claimed subject matter belongs. The patents, patent applications, publications cited herein are hereby incorporated by reference in their entireties, unless stated otherwise. When there are multiple definitions for a term, the definition set forth in this section will prevail.

Unless otherwise stated or clearly contradicted by the context, the articles "a", "an", and "the" used herein are intended to include "at least one" or "one or more". Therefore, as used herein, these articles refer to one or more (i.e., at least one) objects. For example, "a component" means one or more components, that is, more than one component may be contemplated for adoption or use in the implementation of the described embodiments.

It should be understood that the foregoing general description and the following detailed description are illustrative and merely for explanation, rather than limiting the subject matter of the present disclosure in any way. In the present disclosure, unless specifically stated otherwise, the use of the singular also includes the plural. It must be noted that, as used in this specification and the claims, the singular forms include plural referents unless the context clearly dictates otherwise. It should also be noted that the term "or" used herein means "and/or" unless stated otherwise. Furthermore, the term "include" used herein, as well as other forms, such as "comprising", "including", and "containing", is not limiting.

Definitions of standard chemical terms may be found in reference document (including "ADVANCED ORGANIC CHEMISTRY 4THED.", Carey and Sundberg, Vols. A(2000) and B(2001), Plenum Press, New York). Unless otherwise indicated, conventional methods in the art, for example, mass spectroscopy, NMR, IR and UV/VIS spectroscopy, and pharmacological methods, are employed. Unless otherwise specifically defined, the terms used in the descriptions relating to analytical chemistry, organic synthetic chemistry, and medicinal and medicinal chemistry are those known in the art. Standard techniques may be used in chemical synthesis, chemical analysis, pharmaceutical preparation, formulation and delivery, and treatment of patients. For example, reactions and purifications can be performed according to the manufacturer's instructions for the kit, or in a manner well known in the art or in accordance with the description of the present disclosure. In general, the techniques and procedures described above may be performed with conventional methods well known in the art according to the description in a number of general and more specific documents cited and discussed throughout the present description. Throughout the description, groups and substituents thereof can be chosen by one skilled in the field to provide stable moieties and compounds.

Generally, the term "substituted" means that one or more hydrogen atoms in a given structure are substituted with specified substituents. Unless otherwise indicated, a substituted group may have a substituent at each substitutable position of the group. When more than one position in a given structural formula can be substituted by one or more substituents for a specified group, the substituents may be identical or different at each substitutable position. Generally, the term "substituted ring" means that hydrogen atom on each ring atom of the ring may be substituted, for example, 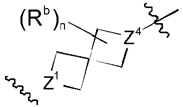 may be

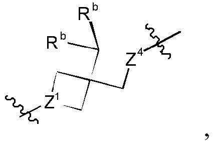

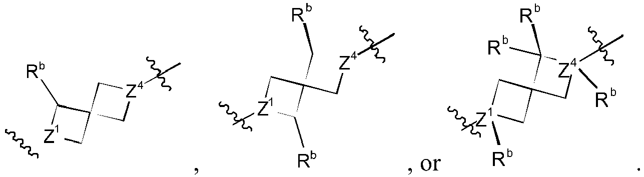

The term "unsubstituted" means that a specified group has no substituents.

As described in the present disclosure, the compounds of the present disclosure may be optionally substituted with one or more substituents, such as the compounds of the general formula set forth above, or specific examples and subclasses in the embodiments and the class of compounds encompassed by the present disclosure. It should be understood that the term "optionally substituted" is interchangeable with the term "substituted or unsubstituted". Generally, the term "optionally", whether preceding the term "substituted" or not, means that one or more hydrogen atoms in a given structure are substituted with specified substituents. Unless otherwise indicated, an optional substituent may have a substituent at any substitutable position of the group. When more than one position in a given structural formula can be substituted by one or more substituents of a specified group, the substituents at each position may be identical or different.

In addition, it should be noted that, unless explicitly stated otherwise, the expressions "each ... is independently", "... are each independently", and "... is independently" used in the present disclosure are interchangeable and shall be construed broadly. They mean that the specific options expressed by the same symbol do not affect each other, whether in different groups or within the same group.

Where a substituent is depicted by a conventional chemical formula, written from left to right, the substituent also encompasses the chemically equivalent substituent that would result from writing the structural formula from right to left. For example, CH₂O is equivalent to OCH₂. As used herein, 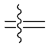 or 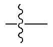 refers to the point of attachment of a group. As used herein, "R₁", "R1", and "R¹" have the same meaning and are interchangeable. For other symbols such as R₂, similar definitions have the same meaning.

The section headings used herein are for organizational purposes only and should not to be construed as limiting the subject matter described. All documents or portions thereof cited in the present disclosure, including but not limited to patents, patent applications, articles, books, manuals, and papers, are incorporated herein by reference in their entireties.

In addition to the foregoing, the following terms, when used in the specification and claims of the present disclosure, have the meanings indicated below, unless otherwise specifically stated.

For the numerical range described in the specification and claims of the present disclosure, when the numerical range is understood as "integers", it should be understood as reciting the two endpoints of the range and all integers in the range. For example, an "integer from 1 to 6" should be understood as reciting the integers 1, 2, 3, 4, 5, and 6; when the numerical range is understood as a "numerical number", it should be understood as reciting the two endpoints of the range, respective integers in the range and respective decimals in the range. For example, a "number from 1 to 10" should be understood as not only reciting the respective integers 1, 2, 3, 4, 5, 6, 7, 8, 9 and 10, but also at least reciting sums of each of the integers and 0.1, 0.2, 0.3, 0.4, 0.5, 0.6, 0.7, 0.8, and 0.9, respectively.

In the present disclosure, "saturated, partially saturated, or unsaturated" includes saturated substituents, substituents completely unsaturated with hydrogen, and substituents partially saturated with hydrogen.

In the present disclosure, the term "halogen", either alone or as part of other substituents, refers to fluorine, chlorine, bromine, or iodine; and preferably, fluorine or chlorine.

As used herein, the term "cyano", either alone or as part of other substituents, refers to -CN.

As used herein, the term "amino", either alone or as part of other substituents, refers to -NH₂.

In the present disclosure, the term "hydroxy" or "hydroxyl", either alone or as part of other substituents, refers to -OH.

In the present disclosure, the term "deuterium", either alone or as part of other substituents, refers to an isotope of hydrogen, also known as heavy hydrogen, with the chemical symbol D or ²H.

The term "alkyl", either alone or as part of other substituents, means a straight or branched hydrocarbon chain group consisting solely of carbon and hydrogen atoms and having, for example, 1 to 6 carbon atoms and connected to the rest of the molecule by a single bond. Examples of alkyl include, but are not limited to, methyl, ethyl, n-propyl, isopropyl, n-butyl, isobutyl, tert-butyl, pentyl, isopentyl, neopentyl, and hexyl. Alkyl may be unsubstituted or substituted with one or more suitable substituents. Alkyl may also be an isotopic isomer of the naturally abundant alkyl enriched in an isotope of carbon and/or hydrogen (i.e., deuterium or tritium).

Unless otherwise stated, a solid wedge bond ( 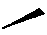 ) and a dashed wedge bond ( 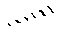 ) are used to denote an absolute configuration of a stereocenter, and a solid straight bond ( 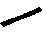 ) and a dashed straight bond ( 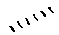 ) are used to denote a relative configuration of the stereocenter. For example: if 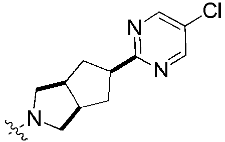 represents one of configurations of 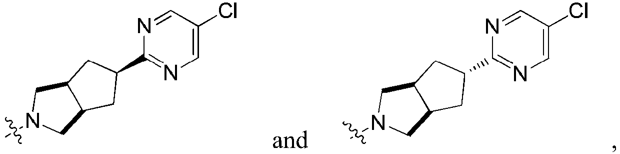 then 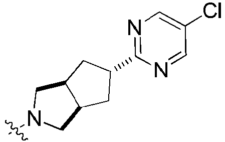 represents the other remaining configuration; and if 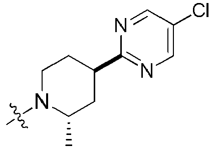 represents one of configurations of 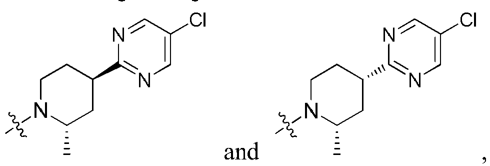 then 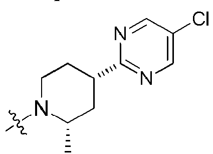 represents the other remaining configuration.

The term "alkyl" refers to a saturated straight or branched monovalent hydrocarbon group of 1 to 6 carbon atoms, or 1 to 4 carbon atoms, or 1 to 3 carbon atoms, in which the alkyl may be independently and optionally substituted with one or more substituents as described in the present disclosure, including, but not limited to, deuterium, amino, hydroxy, cyano, F, Cl, Br, I, mercapto, nitro, oxo (=O), and the like. Examples of alkyl include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), and the like. The term "alkyl" and its prefix "alk-" as used herein both include straight and branched saturated carbon chains.

The term "deuterated alkyl", either alone or as part of other substituents, refers to an alkyl substituted with one or more deuterium atoms, i.e., the hydrogen atom on the alkyl is substituted with deuterium.

The term "deuterated alkoxy", either alone or as part of other substituents, refers to an alkoxy substituted with one or more deuterium atoms, i.e., the hydrogen atoms on the alkoxy is substituted with deuterium.

The term "alkylene", either alone or as part of other substituents, should be interpreted as a straight or branched saturated, unsaturated or partially saturated divalent hydrocarbon group. For example, "C₁₋₆ alkylene" or "C₁₋C₆ alkylene" refers to a straight or branched divalent hydrocarbon group having 1 to 6 carbon atoms, including, but not limited to, methylene, ethylene, propylene, 1-methylpropylene, and butylene.

The term "benzo group", used alone or in combination, refers to the divalent group C₄H₄=, one representation of which is -CH=CH-CH=CH-, which forms a benzene-like ring when ortho-fused to another ring, for example in tetrahydronaphthalene, indole, and the like.

The term "C_{α-β} haloalkyl", either alone or as part of other substituents, refers to the alkyl as defined above, in which any number (at least one) of hydrogen atoms attached to the alkyl chain are substituted with fluorine, chlorine, bromine or iodine.

The term "cycloalkyl", either alone or as part of other substituents, refers to a cyclic alkyl. The term "m- to n-membered cycloalkyl" or "Cₘ to Cₙ cycloalkyl" should be interpreted as a saturated, unsaturated, or partially saturated carbocyclic ring having m to n atoms. For example, "3- to 15-membered cycloalkyl" or "C₃ to C₁₅ cycloalkyl" refers to a cyclic alkyl group containing 3 to 15, 3 to 9, 3 to 6, or 3 to 5 carbon atoms, which may contain 1 to 4 rings. "3- to 10-membered cycloalkyl" contains 3 to 10 carbon atoms, which includes monocyclic, bicyclic, tricyclic, spirocyclic, or bridged rings. Examples of unsubstituted cycloalkyl include, but are not limited to, cyclopropyl, cyclobutyl, cyclopentyl, cyclohexyl, and adamantyl, or a bicyclic hydrocarbon group such as a decalin ring. Cycloalkyl may be substituted with one or more substituents. In some embodiments, cycloalkyl may be a cycloalkyl fused to an aryl or heteroaryl. The terms "cycloalkyl" may be used interchangeably with "carbocyclyl".

The term "heterocycloalkyl", either alone or as part of other substituents, refers to cycloalkyl in which one or more (in some embodiments, 1 to 3) carbon atoms are substituted with heteroatoms such as, but not limited to, N, O, S, and P. The term "m- to n-membered heterocycloalkyl" or "Cₘ₋ₙ heterocycloalkyl" should be interpreted as a saturated, unsaturated or partially saturated ring having m to n atoms, in which the hetero ring atoms are N, O, S, P, and preferably, N, O or S. For example, the term "4- to 8-membered heterocycloalkyl" or "C₄-C₈ heterocycloalkyl" should be interpreted as a saturated, unsaturated or partially saturated ring having 4 to 8 atoms, in which 1, 2, 3, or 4 ring atoms are N, O, S, P, and preferably, N, O, or S. "4- to 10-membered heterocyclyl" refers to a saturated, unsaturated or partially saturated ring having 4 to 10 atoms. In some embodiments, heterocycloalkyl may be a heterocycloalkyl fused to an aromatic ring or heteroaryl ring. When a prefix such as 4- to 8-membered or 4- to 10-membered is used to denote heterocycloalkyl, the number of carbons is intended to include heteroatoms, which includes monocyclic, bicyclic, tricyclic, spirocyclic, or bridged rings. Examples of heterocycloalkyl include pyrrolidinyl, tetrahydrofuranyl, tetrahydropyranyl, tetrahydrothienyl, tetrahydropyridinyl, tetrahydropyrrolyl, azetidinyl, thiazolidinyl, oxazolidinyl, piperidinyl, morpholinyl, thiomorpholinyl, piperazinyl, azepanyl, diazepanyl, oxazepanyl, and the like. The terms "heterocycloalkyl" may be used interchangeably with "heteroalicyclic ring".

The term "alkenyl", either alone or as part of other substituents, refers to a straight or branched monovalent hydrocarbon group of 2 to 40 carbon atoms having at least one carbon-carbon sp² double bond (e.g., C₂-C₆ alkenyl, and further e.g., C₂-C₄ alkenyl), and includes groups having "cis" and "trans" orientations or "E" and "Z" orientations. Examples of alkenyl include, but are not limited to, vinyl and allyl.

The term "alkynyl", either alone or as part of other substituents, refers to a straight or branched monovalent hydrocarbon group of 2 to 40 carbon atoms having at least one carbon-carbon sp triple bond (e.g., C₂-C₆ alkynyl, and further e.g., C₂-C₄ alkynyl). Examples of alkynyl include, but are not limited to, ethynyl and propynyl.

The term "alkoxy", either alone or as part of other substituents, refers to the group -O-R^{Q}, in which R^{Q} is "alkyl" as defined above.

The term "oxo", either alone or as part of other substituents, means that two hydrogen atoms on a methylene group are substituted with oxygen, i.e., the methylene group is substituted with a carbonyl group, represented as =O.

The term "thio", either alone or as part of other substituents, means that two hydrogen atoms on a methylene group are substituted with sulfur, represented as =S.

The term "aromatic ring", either alone or as part of other substituents, refers to a monocyclic or polycyclic carbocyclic ring of 6 to 20 carbon atoms, in which at least one ring is aromatic. When one of the rings is non-aromatic, the group may be linked through the aromatic ring or the non-aromatic ring. Examples of aryl include, but are not limited to: phenyl, naphthyl, tetrahydronaphthyl, 2,3-dihydroindenyl, biphenyl, phenanthryl, anthracenyl and acenaphthyl. The term "aromatic ring" may be used interchangeably with term "aryl".

The term "heteroaromatic ring", either alone or as part of other substituents, refers to a monocyclic or polycyclic carbocyclic ring, in which at least one ring atom is a heteroatom independently selected from oxygen, sulfur, and nitrogen, and the remaining ring atoms are C, and in which at least one ring is aromatic. The group may be a carbon-based group or a heteroatom -based group (i.e., C-linked or N-linked, as long as it is possible). When one of the rings is a non-aromatic ring, the group may be linked through the aromatic ring or the non-aromatic ring. Examples of heteroaryl include, but are not limited to, imidazolyl, acridinyl, carbazolyl, cinnolinyl, quinoxalinyl, pyrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, N-methylpyrrolyl, and tetrahydroquinolinly. The term "heteroaromatic ring" may be used interchangeably with the term "heteroaromatic ring system", "heteroaryl", or "heteroaromatic ring group".

The term "bicyclic", either alone or as part of other substituents, refers to a group having two connected rings. The bicyclic ring may be carbocyclic (all ring atoms are carbon atoms) or heterocyclic (in addition to carbon atoms, ring atoms include, for example, 1, 2, or 3 heteroatoms such as N, O, or S). Both rings may be aliphatic (e.g., decalin and norbornane), aromatic (e.g., naphthalene), or a combination of aliphatic and aromatic (e.g., tetrahydronaphthalene).

Unless otherwise specified, the term "3- to 10-membered heterocycloalkenyl", alone or in combination with other terms, refers to a partially unsaturated cyclic group consisting of 3 to 10 ring atoms containing at least one carbon-carbon double bond, in which 1, 2, 3, or 4 ring atoms are heteroatoms independently selected from O, S and N, and the remainder are carbon atoms, in which the nitrogen atom is optionally quaternized, and the carbon, nitrogen, and sulfur heteroatoms are optionally oxidized (i.e., C(=O), NO, and S(O)p, where p is 1 or 2). It includes monocyclic and bicyclic systems, in which the bicyclic systems include spirocyclic, fused, and bridged rings, and any ring of such systems is non-aromatic. Furthermore, with respect to the "3- to 10-membered heterocycloalkenyl", the heteroatom may occupy the attachment position between heterocycloalkenyl and the rest of the molecule. The 3- to 10-membered heterocycloalkenyl includes 3-, 4-, 5-, 6-, 7-, 8-, 9-, and 10-membered heterocycloalkenyl and the like.

Bicycles include (a) spiro compounds, in which two rings share only a single atom (a spiro atom, which is usually a quaternary carbon). Examples of the spiro compounds include, but are not limited to: 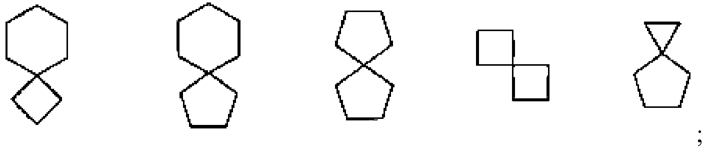 and
further include spirocycloalkyl in which monospirocycloalkyl share a spiro atom with a heterocycloalkyl. Non-limiting examples include: 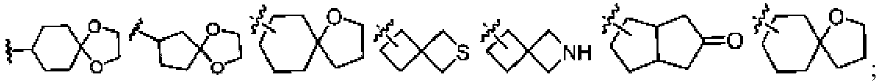
(b) fused rings, i.e., fused bicyclic compounds, in which two rings share two adjacent atoms. That is, the rings share a covalent bond, i.e. bridgehead atoms are directly linked (e.g. α-thujane and decalin). Examples of fused bicyclic rings include, but are not limited to: 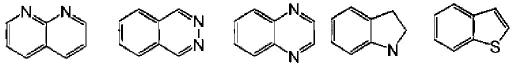 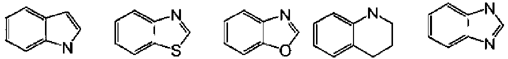 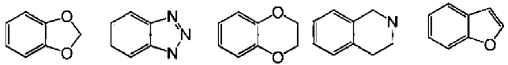 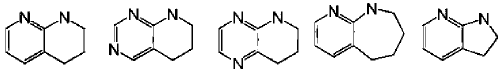 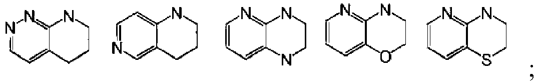
and (c) bridged bicyclic compounds, in which two rings share three or more atoms. and two bridgehead atoms are separated by a bridge containing at least one atom. For example, norbornane, also known as bicyclo[2.2.1]heptane, can be regarded as a pair of cyclopentane rings, each sharing three of their five carbon atoms. Examples of the bridged bicyclic compounds include, but are not limited to:

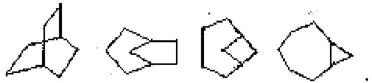

The NR^{f}R^{f} group, either alone or as part of other substituents, may exist in the form of 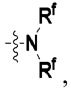 or may also include groups in which two R^{f} groups together form a ring that optionally contains an N, O, or S atom, and may also include the following groups, for example:

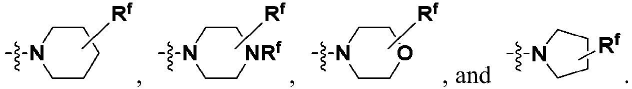

The group N(C_{α-β} alkyl)C_{α-β}alkyl (where α and β are as defined above), either alone or as part of other substituents, includes substituents in which two C_{α-β} alkyl groups together form a ring (optionally containing an N, O, or S atom), and includes groups, for example:

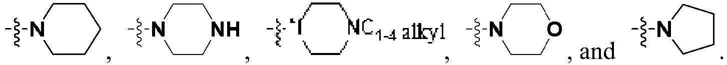

The compound provided according to the present disclosure includes an intermediate that can be used to prepare the compounds according to the present disclosure, which contains reactive functional groups (such as, but not limited to, carboxyl, hydroxyl, and amino moieties), and also includes protected derivatives thereof. "Protected derivatives" are those compounds in which one or more reactive sites are blocked by one or more protecting groups (also referred to as protecting groups). Suitable protecting groups for carboxyl moieties include benzyl, tert-butyl, and the like, as well as isotopes and the like. Suitable protecting groups for amino and amido include acetyl, trifluoroacetyl, tert-butoxycarbonyl, benzyloxycarbonyl, and the like. Suitable protecting groups for hydroxyl moieties include benzyl and the like. Other suitable protecting groups are well-known to those skilled in the art.

In the present disclosure, "optional" or "optionally" means that the subsequently described event or situation may or may not occur, and such a description includes both occurrence and non-occurrence of such event or condition. For example, "optionally substituted aryl" means that the aryl is substituted or unsubstituted, and such an expression includes both substituted and unsubstituted aryl.

In the present disclosure, the term "salt" or "pharmaceutically acceptable salt" includes pharmaceutically acceptable acid addition salts and pharmaceutically acceptable base addition salts. The term "pharmaceutically acceptable" means the compounds, materials, compositions and/or dosage forms that are suitable for use in contact with human and animal tissues without excess toxicity, irritation, allergic reactions or other problems or complications within the scope of reliable medical judgment, and are commensurate with a reasonable benefit/risk ratio.

"Pharmaceutically acceptable acid addition salt" refers to a salt formed with an inorganic acid or organic acid that retains the biological effectiveness of the free base without other adverse effects. "Pharmaceutically acceptable base addition salt" refers to a salt formed with an inorganic base or an organic base that retains the biological effectiveness of the free acid without other adverse effects. In addition to the pharmaceutically acceptable salts, other salts may be contemplated in the present disclosure, and they can serve as intermediates in the purification of compounds or in the preparation of other pharmaceutically acceptable salts, or can be used for identifying, characterizing, or purifying the compounds of the present disclosure.

The term "amine salt" refers to a product obtained by neutralizing an alkyl primary, secondary, or tertiary amine with an acid. The acid includes the inorganic acid or organic acid described in the present disclosure.

The term "stereoisomer" refers to isomers arising from the different spatial arrangements of atoms in a molecule, including a cis-trans isomer, an enantiomer, a diastereoisomer, and a conformational isomer.

According to the selection of the raw materials and methods, the compound of the present disclosure may be present in the form of one or a mixture of possible isomers, for example, in the form of a pure optical isomer or a mixture of isomers such as a mixture of racemate and diastereoisomer, depending on the number of asymmetric carbon atoms. When an optically active compound is described, prefixes *D* and *L,* or *R* and *S* are used to denote an absolute configuration of a molecule in terms of the chiral center (or multiple chiral centers) in the molecule. Prefixes *D* and *L,* or (+) and (-) are symbols used for designating the rotation of plane polarized light caused by a compound, where (-) or *L* indicates that a compound is levorotatory. Compounds with the prefix (+) or *D* are dextrorotatory.

When a bond to a chiral carbon in the formula of the present disclosure is depicted as a straight line, it shall be understood that both (*R*) and (*S*) configurations of the chiral carbon and resulting enantiomerically pure compounds and mixtures thereof, are all encompassed within the scope of the general formula. The racemate or enantiomerically pure compounds of the present disclosure are graphically represented with reference to Maehr, J. Chem. Ed. 1985, 62: 114-120. A wedge bond and a dashed bond are used to denote an absolute configuration of a stereocenter.

The term "tautomer" refers to functional group isomers formed by rapid migration of a certain atom between two positions in a molecule. The compounds of the present disclosure may exhibit tautomerism. The tautomeric compound may be present in two or more interconvertible forms. A prototropic tautomer is formed by migration of a hydrogen atom covalently bonded between two atoms. Tautomer is generally present in an equilibrium state, and separation of a single tautomer usually yields a mixture whose physicochemical properties are consistent with those of a mixture of compounds. The position of equilibrium depends on intramolecular chemical properties. For example, in several aliphatic aldehydes and ketones such as acetaldehyde, ketonic form prevail, while in phenol, an enolic form prevails. The present disclosure encompasses all tautomer of the compound.

The term "solvate" refers to the compound of the present disclosure or a salt thereof includes a stoichiometric or non-stoichiometric solvent bonded therewith through an intermolecular non-covalent force. When the solvent is water, the solvate is a hydrate.

The term "prodrug" refers to a substance that can be converted into the compound of the present disclosure having bioactivity under the physiological conditions or by dissolving in a solvent. The prodrug of the present disclosure is prepared by modifying functional groups in the compound, and the modification can be performed by conventional operations or removed *in vivo* to obtain a parent compound. The prodrug includes a compound formed by linking one hydroxyl group or amino group in the compound of the present disclosure to any group, and when administered to an individual mammal, the prodrug of the compound of the present disclosure is cleaved to form a free hydroxyl group or free amino group.

In the present disclosure, "pharmaceutical composition" refers to a formulation of the compound of the present disclosure and a medium commonly accepted in the art for delivery of a biologically active compound to a mammal (e.g., a human). The medium includes a pharmaceutically acceptable carrier. The purpose of the pharmaceutical composition is to facilitate administration to an organism, facilitate the absorption of the active ingredients, and further promote the exertion of the biological activity.

In the present disclosure, "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier approved by the relevant governmental regulatory authorities as acceptable for human or livestock use.

The term "excipient" refers to pharmaceutically acceptable inert ingredients. Examples of types of the term "excipient" include, but are not limited to, an adhesive, a disintegrant, a lubricant, a glidant, a stabilizer, a filler, a diluent, etc. Excipients can enhance the operating properties of pharmaceutical formulations, that is, improve the flowability and/or adhesion to enable formulation to be more suitable for direct compression.

The term "treatment/treating" refers to therapeutic therapy. When referring to a specific condition, the treatment means: (1) alleviating one or more biological manifestations of a disease or condition; (2) interfering with (a) one or more points in a biological cascade that causes or induce the condition or (b) one or more biological manifestations of the condition, (3) ameliorating one or more symptoms, effects, or side effects associated with the condition, or one or more symptoms, effects, or side effects associated with the condition or treatment thereof, or (4) slowing progression of the condition or one or more biological manifestations of the condition.

The term "prevention/preventing" refers to a reduction of a risk of acquiring or developing a disease or disorder.

The term "patient" refers to any animal, and preferably, a mammal, to which a compound or composition is to be or has been administered according to the embodiments of the present disclosure. The term "mammal" includes any mammal. Examples of mammals include, but are not limited to, cattle, horses, sheep, pigs, cats, dogs, mice, rats, rabbits, guinea pigs, monkeys, humans, and the like, with humans being preferred.

The term "therapeutically effective amount" refers to an amount of a compound that, when administered to a patient, is sufficient to effectively treat a disease or condition described herein. The "therapeutically effective amount" will vary depending on the compound, the condition and its severity, and an age of the patient to be treated, and can be adjusted as needed by those skilled in the art.

For a reaction of each step, a reaction temperature may be appropriately selected based on a solvent, starting materials, reagents, etc., and a reaction duration may also be appropriately selected based on the reaction temperature, solvent, starting materials, reagents, etc. After the reaction of each step is completed, a target compound can be isolated and purified from a reaction system by conventional methods, such as filtration, extraction, recrystallization, washing, and silica gel column chromatography. The target compound may also directly be subjected to the next reaction without the isolation and purification, provided that the next step is not adversely affected.

Without departing from common general knowledge in the art, the above preferred conditions may be combined arbitrarily, thereby obtaining preferred examples of the present disclosure.

### Beneficial Effects

Through extensive and in-depth research, the present inventors have unexpectedly developed a class of deuterated heterocyclic compound and a preparation method and use thereof. The present disclosure provides a compound represented by Formula I, or a tautomer, stereoisomer, hydrates, solvate, pharmaceutically acceptable salt or prodrug thereof. The compound represented by Formula I and the tautomer, stereoisomer, hydrate, solvate, pharmaceutically acceptable salt or prodrug thereof exhibit a significant inhibitory activity against PDE4B, can serve as a selective inhibitor of PDE4B and possess excellent permeability. They exhibit superior pharmacokinetic properties and good druggability. Further, the compounds of the present disclosure exhibit excellent inhibitory activity on TNF-α secreted by lipopolysaccharide (LPS)-stimulated human peripheral blood mononuclear cells (PBMCs). Compared with control compound 1, the compounds of the present disclosure exhibit significant improvements in area under the curve AUC₀₋ₜ and Cₘₐₓ, a lower clearance rate, and superior pharmacokinetic properties. The compounds of the present disclosure have lower toxicity to human hepatocytes and are expected to have better safety for long-term administration. The compounds of the present disclosure exhibit a lower gastrointestinal distribution in mice with a markedly decreased tissue/plasma ratio of the area under the curve. Thus, fewer gastrointestinal side effects such as vomiting and diarrhea are expected upon long-term administration, leading to high safety. In addition, the compounds of the present disclosure can also provide advantageous effects, including high solubility, excellent oral absorption, and a favorable clearance rate.

### DETAILED DESCRIPTION OF THE EMBODIMENTS

The present disclosure is further described below in conjunction with specific embodiments. It should be understood that the following description is only the most preferred embodiment of the present disclosure, and should not be regarded as limiting the protection scope of the present disclosure. On the basis of fully understanding the present disclosure, the experimental methods in the following embodiments for which specific conditions are not specified are generally carried out under conventional conditions or under conditions recommended by the manufacturer. Those skilled in the art can make non-essential changes to the technical solutions of the present disclosure, and such changes should fall within the protection scope of the present disclosure.

### Definitions

### Symbol or unit:

IC₅₀: half maximal inhibitory concentration, which refers to the concentration at which half of the maximum inhibitory effect is achieved.

M: mol/L, for example, n-butyllithium (14.56 mL, 29.1 mmol, 2.5 M n-hexane solution) means a solution of n-butyllithium in n-hexane with a molar concentration of 2.5 mol/L.

N: equivalent concentration, for example, 2N hydrochloric acid means 2 mol/L hydrochloric acid solution.

### Reagents:

DCM: Dichloromethane
DIPEA: N,N-diisopropylethylamine
DMF: N,N-dimethylformamide
TFA: trifluoroacetic acid
THF: Tetrahydrofuran
S-(-)-BINOL: S-1,1'-bi-2-naphthol
Ti(OiPr)₄: titanium tetraisopropoxide

### Intermediate A1: Preparation of (5R)-2-chloro-4-((1-(hydroxymethyl)cyclobutyl)amino)-5-oxo-6,7-dihydrothieno[3,2-d]pyrimidine

The synthetic route of **Intermediate A1** is as follows:

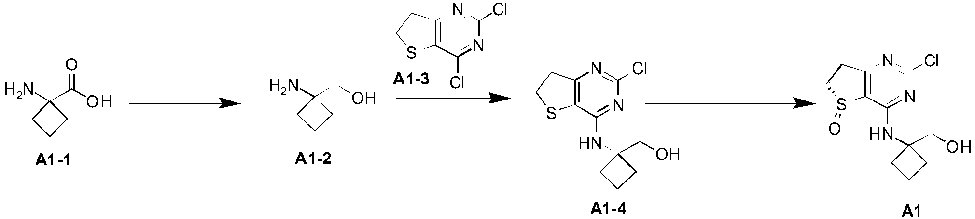

### Step 1: Synthesis of 1-aminocyclobutyl)methanol (Intermediate A1-2)

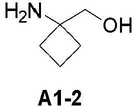

1-aminocyclobutanecarboxylic acid (15 g, 130.3 mmol) was dissolved in tetrahydrofuran (300 mL) at room temperature, and lithium aluminum hydride (2.5 M in tetrahydrofuran, 104 mL, 260 mmol) was added dropwise at 0°C under an argon atmosphere with stirring. After the dropwise addition was completed, the reaction solution was slowly heated to room temperature and stirred for 16 hours under an argon atmosphere. The reaction was quenched with solid sodium sulfate decahydrate in an ice bath, dried over anhydrous sodium sulfate, and filtered. The filter cake was rinsed with ethyl acetate. The combined filtrate was concentrated at room temperature to obtain (1-aminocyclobutyl)methanol **(Intermediate A1-2)** (12 g, yield 90%).

### Step 2: Synthesis of: (1-((2-chloro-6,7-dihydrothieno[3,2-d]pyrimidin-4-yl)amino)cyclobutyl)methanol (Intermediate A1-4)

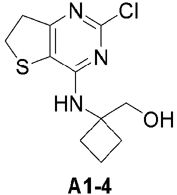

Intermediate A1-2 (3 g, 29.7 mmol), Intermediate A1-3 (2.1 g, 29.7 mmol), and triethylamine (9 g, 100 mmol) were added to acetonitrile (100 mL), and the mixture was stirred at 75°C for 12 hours. The reaction solution was concentrated, and purified by silica gel column chromatography (petroleum ether: ethyl acetate (V/V)=5:1 to 1:1, gradient elution) to obtain (1-((2-chloro-6,7-dihydrothieno[3,2-d]pyrimidin-4-yl)amino)cyclobutyl)methanol **(Intermediate A1-4)** (3.5 g, yield 43%).

### Step 3: Synthesis of: (5R)-2-chloro-4-((1-(hydroxymethyl)cyclobutyl)amino)-5-oxo-6,7-dihydrothieno[3,2-d]pyrimidine (Intermediate A1)

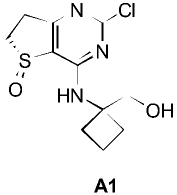

Under a nitrogen atmosphere at 21°C, Intermediate A1-4 (2.7 g, 10 mmol), S-(-)-BINOL (0.28 g, 1 mmol), dichloromethane (80 mL), Ti(OiPr)₄ (1.4 mL, 0.5 mmol), and water (0.18 mL, 10 mmol) were added to a flask and stirred for 1 hour. At 21°C, tert-butyl hydroperoxide (70% in water, 1.5 mL, 11 mmol) was added, and the mixture was stirred at room temperature for 1.5 hours. The reaction solution was concentrated, and purified by silica gel column chromatography (dichloromethane: methanol (V/V)=10:1, gradient elution) to obtain (5R)-2-chloro-4-((1-(hydroxymethyl)cyclobutyl)amino)-5-oxo-6,7-dihydrothio[3,2-d]pyrimidine **(Intermediate A1)** (2.5 g, yield 87%).

### Intermediate 2: 6-(5-chloropyrimidin-2-yl)-2-azaspiro[3.3]heptane (Intermediate B1)

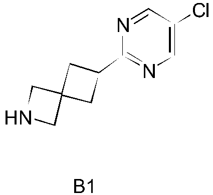

The synthetic route is as follows:

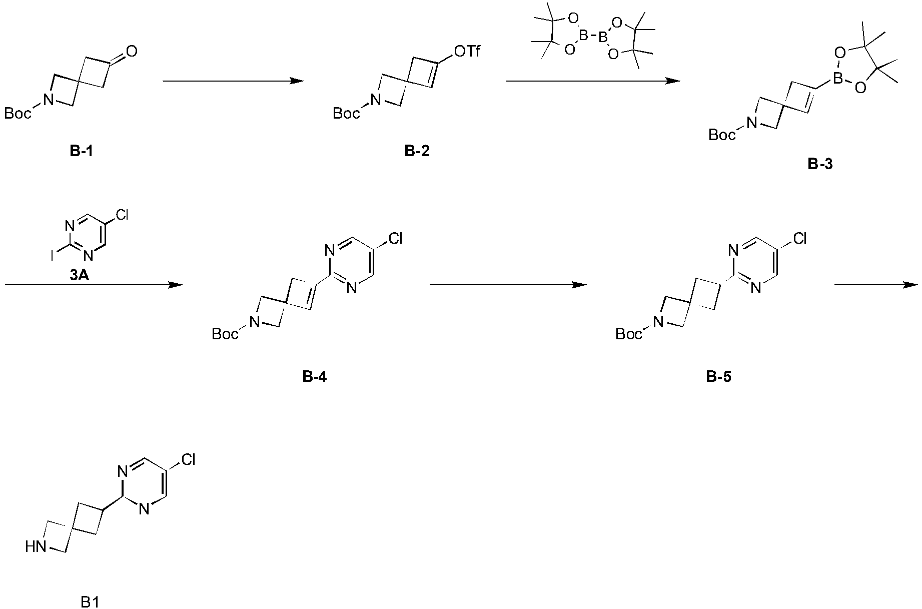

### Step 1: Synthesis of tert-butyl 6-(((trifluoromethyl)sulfonyl)oxy)-2-azaspiro[3.3]hept-5-ene-2-carboxylate (Intermediate B-2)

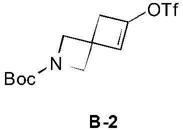

Tert-butyl 6-oxo-2-azaspiro[3.3]heptane-2-carboxylate (Compound 16-1) (5.00 g, 23.7 mmol) was dissolved in tetrahydrofuran (80 mL), and then lithium bis(trimethylsilyl)amide (4.75 g, 28.4 mmol) was added dropwise at -70°C. The mixture was reacted at 25°C for 1 hour. N-phenylbis(trifluoromethanesulfonimide) (9.30 g, 26.0 mmol) was dissolved in tetrahydrofuran (10 mL) and added dropwise to the reaction solution at -78°C. Then, the mixture was heated to 25°C and reacted for 2 hours. The reaction solution was quenched with ammonium chloride (100 mL). Then, the mixture was extracted three times with 300 mL of ethyl acetate each time. The organic phase was washed with saturated brine (200 mL), dried over anhydrous sodium sulfate, filtered, concentrated and purified by silica gel column chromatography (petroleum ether: ethyl acetate (V/V)=1:0 to 20:1) to obtain compound tert-butyl 6-(((trifluoromethyl)sulfonyl)oxy)-2-azaspiro[3.3]hept-5-ene-2-carboxylate **(Intermediate B-2)** (4.20 g, yield 43.1%).

### Step 2: Synthesis of tert-butyl 6-(4,4,5,5-tetramethyl-1,3,2-dioxaborolan-2-yl)-2-azaspiro[3.3]hept-5-ene-2-carboxylate (Intermediate B-3)

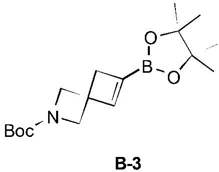

Tert-butyl 6-(((trifluoromethyl)sulfonyl)oxy)-2-azaspiro[3.3]hept-5-ene-2-carboxylate (Compound 16-2) (700 mg, 2.04 mmol), bis(pinacolato)diboron (569 mg, 2.24 mmol), and 1,1'-bis(diphenylphosphino)ferrocene palladium(II) dichloride (47.6 mg, 102 µmol), and potassium acetate (600 mg, 6.12 mmol) were dissolved in 1,4-dioxane (10 mL), and then reacted at 100°C under a nitrogen atmosphere for 10 hours. The reaction solution was filtered with diatomaceous earth and concentrated to obtain tert-butyl 6-(4,4,5,5-tetramethyl-1,3,2-dioxaborolan-2-yl)-2-azaspiro[3.3]hept-5-ene-2-carboxylate **(Intermediate B-3)** (1.00 g, crude product).
LC-MS, M/Z (ESI): 266.2[M-56+H]

### Step 3: Synthesis of tert-butyl 6-(5-chloropyrimidin-2-yl)-2-azaspiro[3.3]hept-5-ene-2-carboxylate (Intermediate B-4)

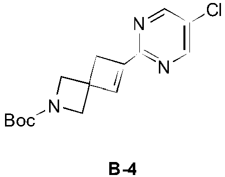

Tert-butyl 6-(4,4,5,5-tetramethyl-1,3,2-dioxaborolan-2-yl)-2-azaspiro[3.3]hept-5-ene-2-carboxylate (Compound 16-3) (800 mg, 2.49 mmol) and 5-chloro-2-iodopyrimidine (718 mg, 2.99 mmol) were dissolved in 1,4-dioxane (20 mL) and water (4 mL), and potassium carbonate (860 mg, 6.23 mmol) and 1,1'-bis(diphenylphosphino)ferrocene palladium(II) dichloride (182 mg, 249 µmol) were added. The mixture was reacted at 90°C under a nitrogen atmosphere for 5 hours. The reaction solution was diluted with water (50 mL), then extracted three times with ethyl acetate (150 mL). The organic phase was washed with saturated brine (100 mL), dried over anhydrous sodium sulfate, filtered, concentrated, and purified by silica gel column chromatography (petroleum ether: ethyl acetate (V/V)=1:0 to 5:1) to obtain Compound tert-butyl 6-(5-chloropyrimidin-2-yl)-2-azaspiro[3.3]hept-5-ene-2-carboxylate **(Intermediate B-4)** (312 mg, yield 41.7%). LC-MS, M/Z (ESI): 252.2 [M-55]⁺. ¹ H NMR (400 MHz, CDCl₃) δ=8.65 (s, 2H), 6.92 (s, 1H), 4.15 (d, 4H), 3.07 (s, 2H), 1.46 (s, 9H).

### Step 4: Synthesis of tert-butyl 6-(5-chloropyrimidin-2-yl)-2-azaspiro[3.3]heptane-2-carboxylate (Intermediate B-5)

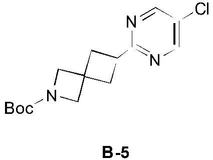

Tert-butyl 6-(5-chloropyrimidin-2-yl)-2-azaspiro[3.3]hept-5-ene-2-carboxylate (Compound 16-4) (400 mg, 1.30 mmol) was dissolved in methanol (10 mL), and tris(triphenylphosphine)rhodium(I) chloride (120 mg, 130 µmol) was added under a nitrogen atmosphere. The suspension was purged three times with nitrogen and hydrogen, respectively, and then reacted at 50°C under a hydrogen pressure of 50 psi for 16 hours. The reaction solution was filtered, concentrated, and purified by silica gel column chromatography (petroleum ether: ethyl acetate (V/V)=10:0 to 5:1) to obtain Compound tert-butyl 6-(5-chloropyrimidin-2-yl)-2-azaspiro[3.3]heptane-2-carboxylate **(Intermediate B-5)** (360 mg, 94.0%). LC-MS, M/Z (ESI): 209.2 [M-100]⁺.

### Step 5: Synthesis of 6-(5-chloropyrimidin-2-yl)-2-azaspiro[3.3]heptane (Intermediate B1)

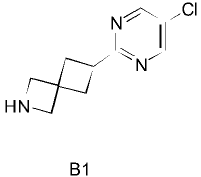

Tert-butyl 6-(5-chloropyrimidin-2-yl)-2-azaspiro[3.3]heptane-2-carboxylate (Compound 16-5) (360 mg, 1.16 mmol) was dissolved in methanol (10 mL), and then a solution of then hydrogen chloride gas in methanol (4M, 5 mL) was added. The mixture was reacted at 25°C for 5 hours. The reaction solution was concentrated to obtain Compound 6-(5-chloropyrimidin-2-yl)-2-azaspiro[3.3]heptane **(Intermediate B1)** (172 mg, yield 54.1%). LC-MS, M/Z (ESI): 210.2 [M+H]⁺.

### Synthetic route 1:

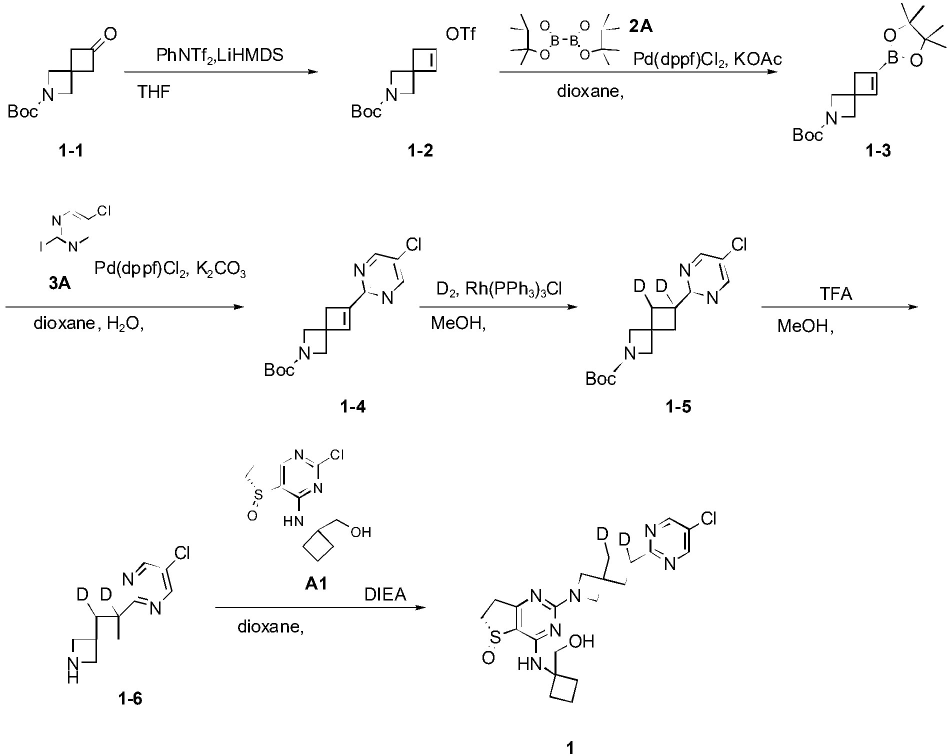

### Example 1: Preparation of Target Compound 1

### (5R)-2-(6-(5-Chloropyrimidin-2-yl)-2-azaspiro[3.3]heptan-2-yl-5,6-d2)-4-((1-(hydroxymethyl)cyclobutyl)amino)-6,7-dihydrothieno[3,2-d]pyrimidine 5-oxide (Target Compound 1)

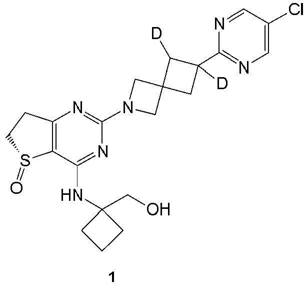

The synthetic route of Target Compound 1 is as follows:

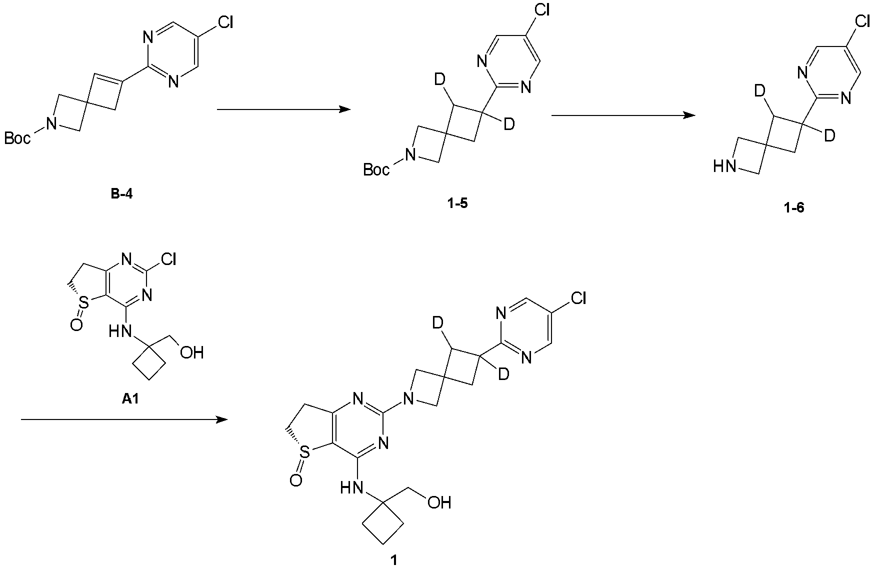

### Step 1: Synthesis of tert-butyl 6-(5-chloropyrimidin-2-yl)-2-azaspiro[3.3]heptane-2-carboxylate-5,6-d2 (1-5)

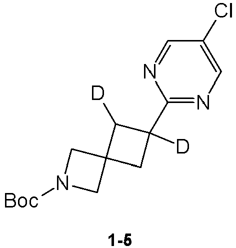

Tert-butyl 6-(5-chloropyrimidin-2-yl)-2-azaspiro[3.3]hept-5-ene-2-carboxylate (B-4) (6.80 g, 20.7 mmol) was dissolved in deuterated methanol-d4 (50.0 mL), and tris(triphenylphosphine)rhodium(I) chloride (3.84 g, 4.15 mmol) was added. The mixture was purged three times with inert gas and deuterium gas, respectively. The reaction mixture was stirred at 50°C under a deuterium gas atmosphere of 50 psi for 20 hours. After the reaction was completed, the reaction solution was filtered and concentrated. The crude product was purified by silica gel column chromatography (petroleum ether: ethyl acetate (V/V)=1:0 to 5:1) to obtain Compound tert-butyl 6-(5-chloropyrimidin-2-yl)-2-azaspiro[3.3]heptane-2-carboxylate-5,6-d2(1-5) (5.16 g, yield 79.5%). LC-MS, M/Z (ESI): 312.3 [M+H]⁺.

### Step 2: Synthesis of 6-(5-chloropyrimidin-2-yl)-2-azaspiro[3.3]heptane-5,6-d2 (1-6)

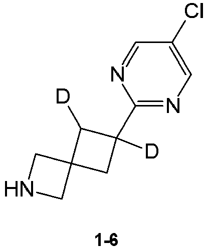

Tert-butyl 6-(5-chloropyrimidin-2-yl)-2-azaspiro[3.3]heptane-2-carboxylate-5,6-d2 (5) (1.01 g, 3.24 mmol) was dissolved in dichloromethane (8.00 mL), and then trifluoroacetic acid (2.00 mL) was added at 0°C. The reaction mixture was stirred at 25°C for 1 hour. After the reaction was completed, the reaction solution was concentrated and evaporated to dryness to obtain Compound 6-(5-chloropyrimidin-2-yl)-2-azaspiro[3.3]heptane-5,6-d2 **(1-6)** (1.00 g, crude product), which was used directly in the next step. LC-MS, M/Z (ESI): 212.2 [M+H]⁺.

### Step 3: Synthesis of: (5R)-2-(6-(5-chloropyrimidin-2-yl)-2-azaspiro[3.3]heptan-2-yl-5,6-d2)-4-((1-(hydroxymethyl)cyclobutyl)amino)-6,7-dihydrothieno[3,2-d]pyrimidine 5-oxide (Target Compound 1)

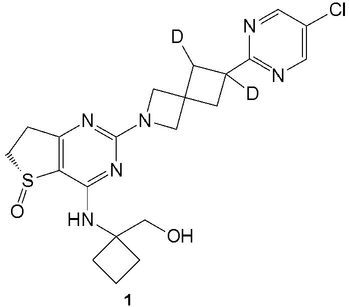

6-(5-chloropyrimidin-2-yl)-2-azaspiro[3.3]heptane-5,6-d2(1-6) (960 mg, 2.95 mmol) was dissolved in 1,4-dioxane (30.0 mL), and then N,N-diisopropylethylamine (1.90 g, 14.7 mmol, 2.57 mL) and (R)-2-chloro-4-((1-(hydroxymethyl)cyclobutyl)amino)-6,7-dihydrothieno[3,2-d]pyrimidine 5-oxide (Intermediate A1) (805 mg, 2.80 mmol) were added. The reaction mixture was slowly heated to 100°C and stirred for 1 hour. After the reaction was completed, the reaction solution was concentrated and evaporated to dryness. The crude product was first purified by silica gel column chromatography (dichloromethane: methanol (V/V)=1:0 to 10:1), and then purified by high-performance liquid chromatography (column: Waters Xbridge 150×25 mm×5 µm; solvent: A=water+ammonia (0.5%), B=acetonitrile; gradient: 20% to 50%, 11 minutes) to obtain Compound (5R)-2-(6-(5-chloropyrimidin-2-yl)-2-azaspiro[3.3]heptan-2-yl-5,6-d2)-4-((1-(hydroxymethyl)cyclobutyl)amino)-6,7-dihydrothieno[3,2-d]pyrimidine 5-oxide **(Compound 1)** (500 mg, yield 36.4%). LC-MS, M/Z (ESI): 463.3 [M+H]⁺. ¹H NMR (400 MHz, CDCl₃) *δ* 8.63 (s, 2H), 5.89 (s, 1H), 5.20-5.71 (m, 1H), 4.08-4.30 (m, 4H), 3.85 (s, 2H), 3.55-3.65 (m, 1H), 3.36-3.47 (m, 1H), 2.96-3.09 (m, 2H), 2.58-2.69 (m, 3H), 2.34 (br d, 2H), 2.10-2.23 (m, 2H), 1.88-2.03 (m, 2H)

### Example 2: Synthesis of Compound 2

### 2-(6-(5-chloropyrimidin-2-yl)-6-deuterio-2-azaspiro[3.3]heptan-2-yl)-4-((1-(hydroxymethyl)cyclobutyl)amino)-6,7-dihydrothieno[3,2-d]pyrimidine 5-oxide (Target Compound 2)

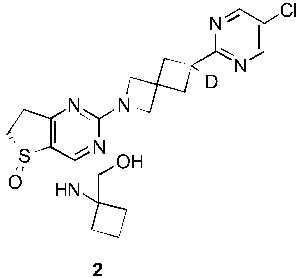

The synthetic route of Compound **2** is as follows:

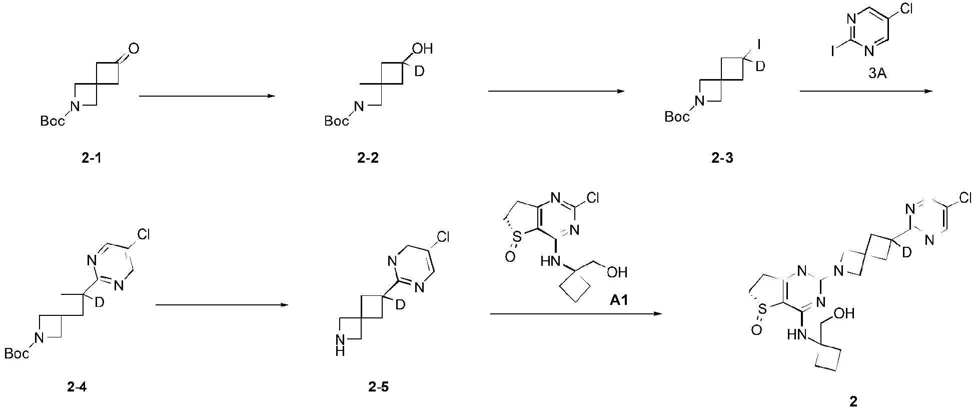

### Step 1: Tert-butyl 6-deuterio-6-hydroxy-2-azaspiro[3.3]heptane-2-carboxylate (Compound 2-2)

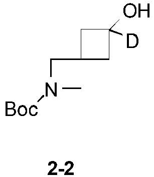

In a 100 mL single-necked flask, tert-butyl 6-oxo-2-azaspiro[3.3]heptane-2-carboxylate (1.0 g, 47.3 mmol) was dissolved in methanol (10 mL), and sodium borodeuteride (397 mg, 94.6 mmol) was added slowly in portions at room temperature. The reaction system was stirred at room temperature for 1 hour. The reaction apparatus was removed, and aqueous NH₄Cl solution (10 mL) was slowly added. The mixture was extracted twice with ethyl acetate (10 mL×2). The organic phase was combined, dried over anhydrous sodium sulfate and filtered. The resulting mother liquor was concentrated and evaporated to dryness to obtain target compound tert-butyl 6-deuterio -2-azaspiro[3.3]heptane-2-carboxylate (Compound **2-2**) (1.0 g, yield 99.6%).

### Step 2: Tert-butyl 6-deuterio-6-iodo-2-azaspiro[3.3]heptane-2-carboxylate (Compound 2-3)

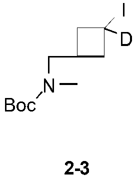

In a 100 mL single-necked flask, Compound **2-2** (1.0 g, 47.1 mmol) was dissolved in Toluene (10.0 mL). Then, triphenylphosphine (1.83 g, 70.7 mmol), imidazole (477 mg, 70.7 mmol), and elemental iodine (1.43 g, 56.5 mmol) were added to the reaction solution. The reaction system was heated to 120°C and stirred for 1 hour. The reaction solution was cooled, then filtered with diatomaceous earth and rinsed with ethyl acetate (20.0 mL). The mother liquor was concentrated and evaporated to dryness for sample loading, and then purified by silica gel column chromatography (petroleum ether: ethyl acetate (V/V)=1:0 to 20:1) to obtain Target Compound tert-butyl 6-deuterio-6-iodo-2-azaspiro[3.3]heptane-2-carboxylate (Compound **2-3**) (1.0 g, yield 65.8%).

### Step 3: Tert-butyl 6-(5-chloropyrimidin-2-yl)-6-deuterio-2-azaspiro[3.3]heptane-2-carboxylate (Compound 2-4)

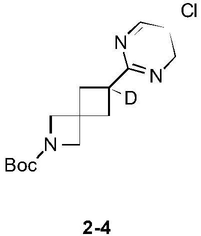

In a 250.0 mL three-necked flask, Compound **2-3** (6.0 g, 186 mmol) and zinc powder (1.2 g, 372 mmol) were dissolved in a mixed solvent of DMAC (30.0 mL) and THF (30.0 mL). The system was purged with N₂ atmosphere three times, and then trimethylchlorosilane was slowly added to the reaction solution (200 mg, 37.2 mmol). The reaction system was stirred at room temperature for half an hour. 5-Chloro-2-iodopyrimidine (2.23 g, 186 mmol) and Pd(dppf)Cl₂ (336 mg, 9.3 mmol) dissolved in DMAC (30.0 mL) and THF (300.0 mL) were added to the reaction solution. After the addition was completed, the system was heated to 90°C and stirred overnight. The reaction solution was cooled and then filtered with diatomaceous earth. A saturated NH₄Cl solution (50.0 mL) was added to the mother liquor. The mixture was extracted with ethyl acetate (50.0 mL). The resulting organic phase was washed with saturated brine (50.0 mL), dried over anhydrous sodium sulfate and then filtered. The mother liquor was concentrated and evaporated to dryness for sample loading, and then purified by silica gel column chromatography (petroleum ether: ethyl acetate (V/V)=30:1 to 10:1) to obtain Target Compound tert-butyl 6-(5-chloropyrimidin-2-yl)-6-deuterio-2-azaspiro[3.3]heptane-2-carboxylate (Compound 2-4) (3.7 g yield 65%).

### Step 4: 6-(5-Chloropyrimidin-2-yl)-6-deuterio-2-azaspiro[3.3]heptane hydrobromide (Compound 2-5)

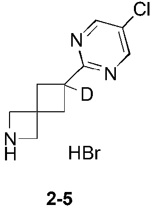

Compound 2-4 (1.7 g, 54.8 mmol) was dissolved in DCM (20.0 mL) in a 10.0 mL single-necked flask, and boron tribromide (1.6 g, 65.7 mmol) was slowly added to the reaction solution. The reaction system was stirred at room temperature for 2 hours. The reaction solution was directly concentrated and evaporated to dryness to obtain Target Compound 6-(5-chloropyrimidin-2-yl)-6-deuterio-2-azaspiro[3.3]heptane hydrobromide (Compound 2-5).

### Step 5: 2-(6-(5-chloropyrimidin-2-yl)-6-deuterio-2-azaspiro[3.3]heptan-2-yl)-4-((1-(hydroxymethyl)cyclobutyl)amino)-6,7-dihydrothieno[3,2-d]pyrimidine 5-oxide

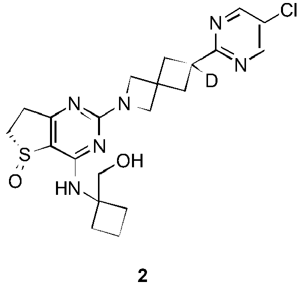

In a 10 mL single-necked flask, Compound 2-5 (1.15 g, 55.3 mmol) was dissolved in a mixed solvent of THF (20.0 mL) and H₂O (10.0 mL), and then DIEA (2.56 g, 276.5 mmol) was added to the reaction solution. The mixture was stirred for ten minutes, and then (5R)-2-chloro-4-((1-(hydroxymethyl)cyclobutyl)amino)-6,7-dihydrothieno[3,2-d]pyrimidine 5-oxide 6A (683 mg, 33.2 mmol) was added to the reaction solution. The system was heated to 85°C and stirred overnight. After the reaction solution was cooled, H₂O was added to the reaction solution. The mixture was extracted twice with DCM (20.0 mL). The organic phase was combined, dried over anhydrous sodium sulfate (20.0 g) and filtered. The mother liquor was concentrated and evaporated to dryness for sample loading, and then purified by silica gel column chromatography to obtain Target Compound (R)-2-(6-(5-chloropyrimidin-2-yl)-6-deuterio-2-azaspiro[3.3]heptan-2-yl)-4-((1-(hydroxymethyl)cyclobutyl)amino)-6,7-dihydrothieno[3,2-d]pyrimidine 5-oxide (Target Compound **2**) (604 mg, yield 55%). LC-MS, M/Z (ESI): 462.39 (M+1).

¹H NMR (400 MHz, CDCl₃) δ 8.61 (s, 2H), 5.86 (s, 1H), 5.46 (d, 1H), 4.17 (d, 4H), 3.83 (s, 2H), 3.64-3.52 (m, 1H), 3.45-3.35 (m, 1H), 3.01 (dt, 2H), 2.63 (q, 4H), 2.33 (d, 2H), 2.23-2.10 (m, 2H), 2.05-1.84 (m, 2H).

### Example 3: Preparation of Target Compound 3

### (R)-2-(6-(5-chloropyrimidin-2-yl)-2-azaspiro[3.3]heptan-2-yl)-4-((1-(hydroxymethyl-d2)cyclobutyl)amino)-6,7-dihydrothieno[3,2-d]pyrimidine 5-oxide (Target Compound 3)

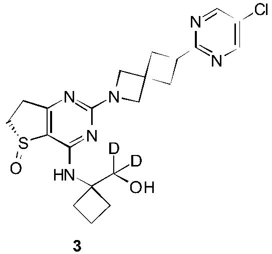

The synthetic route of Target Compound 3 is as follows:

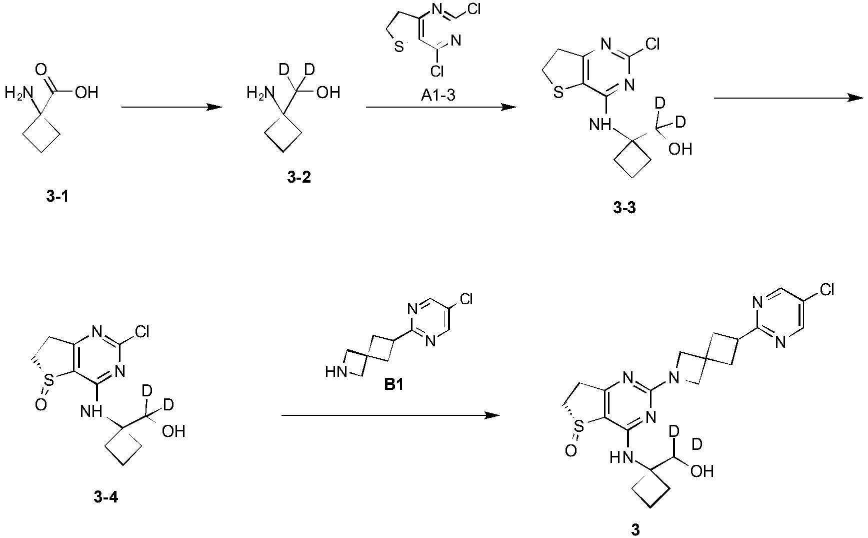

### Step 1: Synthesis of (1-aminocyclobutyl)methan-d2-ol (Compound 3-2)

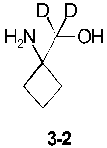

1-aminocyclobutanecarboxylic acid (Compound **3-1**) (15.0 g, 130 mmol) was dissolved in anhydrous tetrahydrofuran (500 mL) and cooled to 0°C in an ice-water bath, and lithium aluminum deuteride (9.89 g, 260 mmol) was carefully added. The mixture was reacted at 25°C for 10 hours. The reaction solution was cooled to 0°C, and sodium sulfate decahydrate (20.0 g, 65.1 mmol) was carefully and slowly added under a nitrogen stream to quench the reaction. The mixture was dried over anhydrous sodium sulfate, filtered, and concentrated under reduced pressure, to obtain a yellow oily compound (1-aminocyclobutyl)methane-d2-ol (Compound 3-2) (14.5 g, crude product, directly used in the next step).

### Step 2: Synthesis of: (1-((2-chloro-6,7-dihydrothieno[3,2-d]pyrimidin-4-yl)amino)cyclobutyl)methan-d2-ol (Compound 3-3)

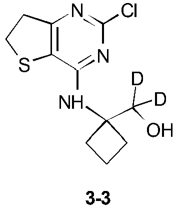

2,4-dichloro-6,7-dihydrothieno[3,2-d]pyrimidine (Compound A1-3) (22.0 g, 104 mmol) and (1-aminocyclobutyl)methan-d2-ol (12.9 g, 125 mmol) were dissolved in acetonitrile (400 mL), and triethylamine (31.7 g, 313 mmol, 43.6 mL) was added. The mixture was reacted at 80°C for 10 hours. The reaction solution was added to a saturated sodium bicarbonate solution (400 mL), extracted three times with ethyl acetate (900 mL), washed with saturated brine (1000 mL), dried over sodium sulfate, and concentrated. The crude product was purified by silica gel column chromatography (petroleum ether: ethyl acetate (V/V)=5:1 to 1:1) to obtain Target Compound (1-((2-chloro-6,7-dihydrothieno[3,2-d]pyrimidin-4-yl)amino)cyclobutyl)methan-d2-ol (Compound **3-3**) (6.00 g, yield 21.4%).

### Step 3: (R)-2-chloro-4-((1-(hydroxymethyl-d2)cyclobutyl)amino)-6,7-dihydrothieno[3,2-d]pyrimidine 5-oxide (Compound 3-4)

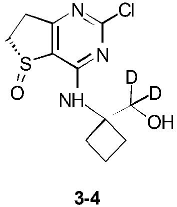

(1-((2-Chloro-6,7-dihydrothieno[3,2-d]pyrimidin-4-yl)amino)cyclobutyl)methan-d2-ol (3-3) (5.00 g, 18.0 mmol), S-(-)-1,1'-Bi-2-naphthol (515 mg, 1.80 mmol) were dissolved in dichloromethane (50 mL), and titanium tetraisopropoxide (255 mg, 900 µmol, 265 µL) and water (324 mg, 18.0 mmol, 324 µL) were added. The mixture was reacted at 20°C for 1 hour. After cooling to 0°C, 70% aqueous tert-butanol hydroperoxide solution (2.43 g, 18.9 mmol, 2.59 mL, 70%) was added. The mixture was reacted at 25°C for 1.5 hours. The solid product precipitated in the reaction solution was filtered, and the filter cake was washed twice with ethyl acetate (10 mL) and dried, to obtain Target Compound (R)-2-chloro-4-((1-(hydroxymethyl-d2)cyclobutyl)amino)-6,7-dihydrothieno[3,2-d]pyrimidine 5-oxide (Target Compound **3-**4) (4.60 g, yield 88.5%).

### Step 4: Synthesis of (R)-2-(6-(5-chloropyrimidin-2-yl)-2-azaspiro[3.3]heptan-2-yl)-4-((1-(hydroxymethyl-d2)cyclobutyl)amino)-6,7-dihydrothieno[3,2-d]pyrimidine 5-oxide (Target Compound 3)

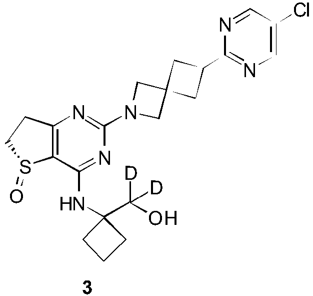

6-(5-chloropyrimidin-2-yl)-2-azaspiro[3.3]heptane hydrobromide (Intermediate B1) (1.20 g, 4.13 mmol) and N,N-diisopropylethylamine (2.23 g, 17.2 mmol, 3.00 mL) were added to a solution of (R)-2-chloro-4-((1-(hydroxymethyl-d2)cyclobutyl)amino)-6,7-dihydrothieno[3,2-d]pyrimidine 5-oxide (64.4 mg, 219 µmol) in 1,4-dioxane (30 mL). The mixture was slowly heated to 100°C for 3 hours. The reaction solution was concentrated and evaporated to dryness. The crude product was purified by silica gel column chromatography (dichloromethane: methanol (V/V)=1:0 to 10:1) to obtain Target Compound (R)-2-(6-(5-chloropyrimidin-2-yl)-2 -azaspiro[3.3]heptan-2-yl)-4-((1-(hydroxymethyl-d2)cyclobutyl)amino)-6,7-dihydrothieno[3,2-d]pyrimidin-5-oxide (Compound **3**) (1.00 g, yield 60.8%).
LC-MS, M/Z (ESI): 463.2 [M+H]⁺
¹HNMR (400 MHz, DMSO_d₆) *δ*=8.62 (s, 2H), 6.25 (br s, 1H), 4.95-5.82 (m, 1H), 3.99-4.39 (m, 4H), 3.57-3.75 (m, 2H), 3.33-3.44 (m, 1H), 2.94-3.12 (m, 2H), 2.65 (br m, 4H), 2.15-2.34 (m, 4H), 1.82-1.97 (m, 2H).

### Example 4: Synthesis of Compound 4

### 2-[6-(5-chloropyrimidin-2-yl)-6-deuterio-2-azaspiro[3.3]heptan-2-yl]-4-(((dideuterohydroxymethyl)cyclobutyl)amino)-6,7-dihydrothieno[3,2-d]pyrimidine-5-oxide (Compound 4)

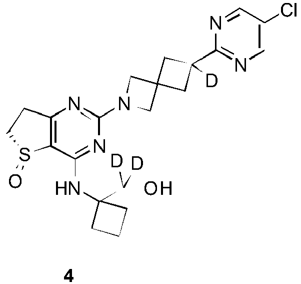

The synthetic route is as follows:

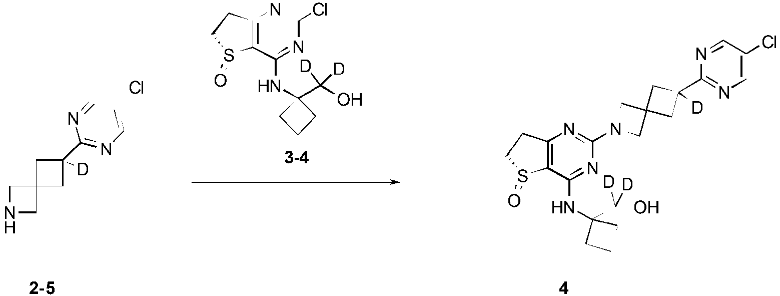

In a 100 mL single-necked flask, a bromine compound of Compound **2-5** (700 mg, 3.35 mmol) was dissolved in a mixed solvent of THF (5.0 mL) and H₂O (5.0 mL). Then, DIEA (2.14 g, 16.75 mmol) was added to the reaction solution. After stirring for ten minutes, Compound 3-4 (574 mg, 2.1 mmol) was added to the reaction solution. The system was heated to 85°C and stirred overnight. After the reaction solution was cooled, H₂O (10.0 mL) was added to the reaction solution. The mixture was extracted twice with DCM (10.0 mL). The organic phase was combined, dried over anhydrous sodium sulfate (10.0 g) and filtered. The mother liquor was concentrated and evaporated to dryness for sample loading, and then purified by silica gel column chromatography to obtain Target Compound (R)-2-[6-(5-chloropyrimidin-2-yl)-6-deuterio-2-azaspiro[3.3]heptan-2-yl]-4-(((dideuterohydroxymethyl)cyclobutyl)amino)-6,7-dihydrothieno[3,2-d]pyrimidine 5-oxide (Compound **4**) (740 mg, yield 80%).
LC-MS, M/Z (ESI): 464.2 (M+1).
¹H NMR (400 MHz, CDCl₃) δ 8.61 (s, 2H), 5.87 (s, 1H), 5.42 (s, 1H), 4.17 (d, 4H), 3.64-3.53 (m, 1H), 3.44-3.35 (m, 1H), 3.07-2.94 (m, 2H), 2.63 (q, 4H), 2.32 (d, 2H), 2.23-2.10 (m, 2H), 2.03-1.82 (m, 2H).

### Example 5: Synthesis of Compound 7

### (R)-2-(6-(5-chloropyrimidin-2-yl)-2-azaspiro[3.3]heptan-2-yl)-4-((1-(hydroxymethyl)cyclobutyl-2,2,3,3,4,4-d6)amino)-6,7-dihydrothieno[3,2-d]pyrimidine 5-oxide (Target Compound 7)

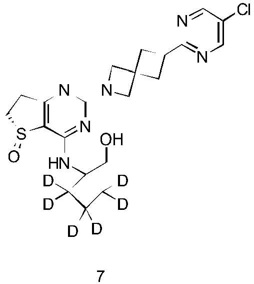

The synthetic route of Target Compound 7 is as follows:

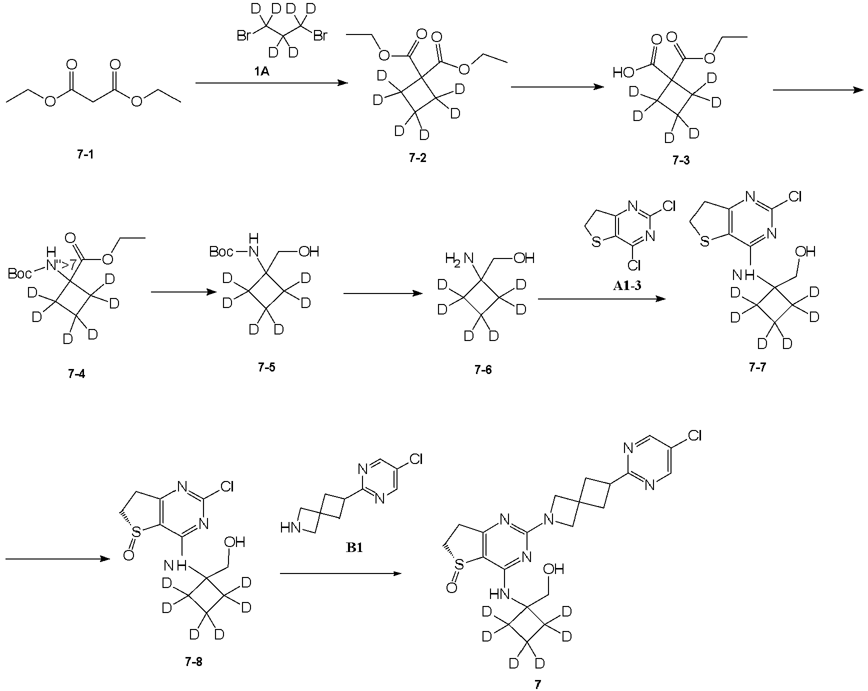

### Step 1: Synthesis of diethyl cyclobutane-1,1-dicarboxylate-d6 (7-2)

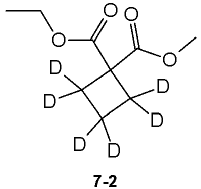

Diethyl malonate **(7-1**) (1.54 g, 9.62 mmol, 1.46 mL) and 1,3-dibromopropane-1,1,2,2,3,3-d6 (**1A**) (2.00 g, 9.62 mmol) were dissolved in anhydrous tert-butanol (10.0 mL). The mixture was cooled to 0°C in an ice-water bath, and then sodium tert-butoxide (1.00 M, 21.1 mL, 2.20 eq) was added. The reaction mixture was stirred at 90°C for 4 hours. After the reaction was completed, the reaction solution was concentrated and evaporated to dryness and diluted with water (10.0 mL). The mixture was extracted with dichloromethane (10.0 mL). The organic phase was combined, washed with saturated brine (10.0 mL), dried over anhydrous sodium sulfate, filtered, and concentrated. The crude product was purified by silica gel column chromatography (petroleum ether: ethyl acetate (V/V)=1/0 to 10/1), to obtain Compound diethyl cyclobutane-1,1-dicarboxylate-d6 **(7-2)** (950 mg, yield 47.8%). ¹H NMR (400 MHz, CDCl₃) *δ* 4.10-4.28 (m, 4H), 1.24 (t, 6H).

### Step 2: Synthesis of 1-(ethoxycarbonyl)cyclobutane-1-carboxylic acid-2,2,3,3,4,4-d6 (7-3)

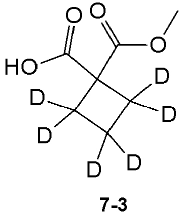

Diethyl cyclobutane-1,1-dicarboxylate-d6 **(7-2)** (950 mg, 4.61 mmol) was dissolved in ethanol (6.00 mL). The mixture was cooled to 0°C in an ice-water bath. Aqueous potassium hydroxide solution (1.80 M, 2.81 mL) was carefully added. The reaction mixture was stirred at 0°C for 5 hours and then stirred at 25°C for 35 hours. After the reaction was completed, the reaction solution was concentrated and evaporated to dryness and diluted with water (5.00 mL). Then, the pH was adjusted to 2 with 1.00 M dilute hydrochloric acid. Then, the mixture was extracted three times with ethyl acetate (30.0 mL). The organic phase was combined, washed with saturated brine (10.0 mL), dried over anhydrous sodium sulfate, filtered, and concentrated and evaporated to dryness, to obtain Compound 1-(ethoxycarbonyl)cyclobutane-1-carboxylic acid-2,2,3,3,4,4-d6 **(7-3)** (470 mg, yield 57.2%).

### Step 3: Synthesis of ethyl 1-((tert-butoxycarbonyl)amino)cyclobutane-1-carboxylate-2,2,3,3,4,4-d6 (7-4)

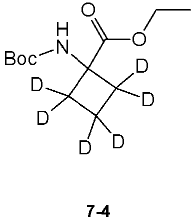

1-(ethoxycarbonyl)cyclobutane-1-carboxylic acid-2,2,3,3,4,4-d6 **(7-3)** (470 mg, 2.64 mmol) was dissolved in anhydrous tert-butanol (10.0 mL), and diphenylphosphoryl azide (762 mg, 2.77 mmol, 597 µL) and triethylamine (374 mg, 2.90 mmol, 505 µL) were added. The reaction mixture was stirred at 25°C for 0.5 hours and then stirred at 100°C for 4 hours. After the reaction was completed, the reaction solution was diluted with water (10.0 mL). Then, the mixture was extracted three times with ethyl acetate(30.0 mL). The organic phase was combined, washed with 5% aqueous citric acid solution (20.0 mL), saturated sodium bicarbonate (20.0 mL), and brine (30.0 mL), respectively, dried over anhydrous sodium sulfate, filtered, and concentrated. The crude product was purified by thin-layer silica gel chromatography (petroleum ether: ethyl acetate (V/V)=10:1), to obtain Compound ethyl 1-((tert-butoxycarbonyl)amino)cyclobutane-1-carboxylate-2,2,3,3,4,4-d6 **(7-4)** (200 mg, yield 30.4%).

### Step 4: Synthesis of tert-butyl (1-(hydroxymethyl)cyclobutyl-2,2,3,3,4,4-d6) carbamate (7-5)

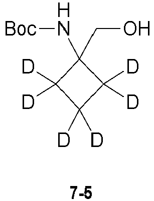

Ethyl 1-((tert-butoxycarbonyl)amino)cyclobutane-1-carboxylate-2,2,3,3,4,4-d6 (7-**4**) (200 mg, 802 µmol) was dissolved in anhydrous tetrahydrofuran (10.0 mL). The mixture was cooled to 0°C in an ice-water bath. Lithium borohydride solution (2.00 M, 1.60 mL) was carefully added. The reaction mixture was stirred at 0°C for 0.5 hours and then stirred at 25°C for 1.5 hours. After the reaction was completed, the reaction solution was cooled to 10°C. 1M citric acid (5.00 mL) was carefully and slowly added under a nitrogen stream to quench the reaction. Then, the mixture was extracted three times with ethyl acetate (30.0 mL). The organic phase was combined, washed with saturated sodium bicarbonate (20.0 mL) and brine (30.0 mL), dried over anhydrous sodium sulfate, filtered, and concentrated and evaporated to dryness, to obtain Compound tert-butyl (1-(hydroxymethyl)cyclobutyl-2,2,3,3,4,4-d6) carbamate **(7-5)** (200 mg, crude product), which was used directly in the next step.

### Step 5: Synthesis of: (1-aminocyclobutyl-2,2,3,3,4,4-d6)methanol hydrochloride (7-6)

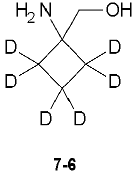

Tert-butyl (1-(hydroxymethyl)cyclobutyl-2,2,3,3,4,4-d6) carbamate **(7-5)** (200 mg, 964 µmol) was dissolved in anhydrous dioxane (10.0 mL), and dioxane hydrochloride solution (2.00 M, 9.65 mL) was carefully added. The reaction mixture was stirred at 25°C for 1 hour. After the reaction was completed, the reaction solution was concentrated and evaporated to dryness, to obtain Compound (1-aminocyclobutyl-2,2,3,3,4,4-d6) methanol hydrochloride **(7-6)** (200 mg, crude product), which was used directly in the next step.

### Step 6: Synthesis of: (1-((2-chloro-6,7-dihydrothieno[3,2-d]pyrimidin-4-yl)amino)cyclobutyl-2,2,3,3,4,4-d6)methanol (7-7)

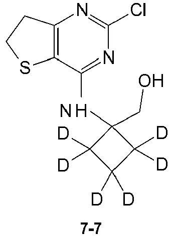

(1-aminocyclobutyl-2,2,3,3,4,4-d6) methanol hydrochloride **(7-6)** (200 mg, 1.39 mmol, HCl) and 2,4-dichloro-6,7-dihydrothieno[3,2-d]pyrimidine **(A1-3)** (288 mg, 1.39 mmol) were dissolved in acetonitrile (10.0 mL), and triethylamine (704 mg, 6.96 mmol, 968 µL) was added. The reaction mixture was stirred at 80°C for 2 hours. After the reaction was completed, the reaction solution was diluted with water (10.0 mL). Then, the mixture was extracted three times with ethyl acetate (30.0 mL). The organic phase was combined, washed with saturated brine (30.0 mL), dried over sodium sulfate, and concentrated. The crude product was purified by silica gel column chromatography (petroleum ether: ethyl acetate (V/V)=5:1 to 1:1), to obtain Compound (1-((2-chloro-6,7-dihydrothieno[3,2-d]pyrimidin-4-yl)amino)cyclobutyl-2,2,3,3,4,4-d6)methanol **(7-7)** (70.0 mg, yield 16.2%). LC-MS, M/Z (ESI): 278.0 [M+H]⁺.

### Step 7: Synthesis of (R)-2-chloro-4-((1-(hydroxymethyl)cyclobutyl-2,2,3,3,4,4-d6)amino)-6,7-dihydrothieno[3,2-d]pyrimidine 5-oxide (7-8)

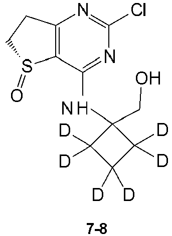

(1-((2-chloro-6,7-dihydrothieno[3,2-d]pyrimidin-4-yl)amino)cyclobutyl-2,2,3,3,4,4-d6)methanol **(7-7)** (60.0 mg, 194 µmol) and (S)-(-)-1,1'-bi-2-naphthol (11.1 mg, 38.8 µmol) were dissolved in dichloromethane (10.0 mL), and then titanium tetraisopropoxide (5.52 mg, 19.4 µmol, 5.74 µL) and water (3.50 mg, 194 µmol, 3.50 µL) were added. The reaction mixture was stirred at 20°C for 1 hour and then cooled to 0°C. 70% aqueous tert-butyl hydroperoxide solution (26.2 mg, 204 µmol, 27.9 µL, 70.0%) was added, and the reaction mixture was stirred at 25°C for 1.5 hours. After the reaction was completed, saturated aqueous sodium sulfite solution (10.0 mL) was added to the reaction solution to quench the reaction. Then, the mixture was concentrated and evaporated to dryness. The crude product was purified by silica gel column chromatography (dichloromethane:methanol (V/V)=1:0 to 10:1), to obtain Compound (R)-2-chloro-4-((1-(hydroxymethyl)cyclobutyl-2,2,3,3,4,4-d6)amino)-6,7-dihydrothieno[3,2-d]pyrimidine 5-oxide **(7-8)** (40.0 mg, yield 70.0%). LC-MS, M/Z (ESI): 294.0 [M+H]⁺.

### Step 8: Synthesis of (R)-2-(6-(5-Chloropyrimidin-2-yl)-2-azaspiro[3.3]heptan-2-yl)-4-((1-(hydroxymethyl)cyclobutyl-2,2,3,3,4,4-d6)amino)-6,7-dihydrothieno[3,2-d]pyrimidine 5-oxide (Target Compound 7)

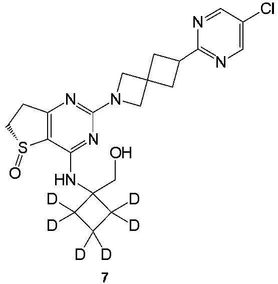

(R)-2-chloro-4-((1-(hydroxymethyl)cyclobutyl-2,2,3,3,4,4-d6)amino)-6,7-dihydrothieno[3,2-d]pyrimidine 5-oxide **(7-8)** (40.0 mg, 136 µmol) was dissolved in 1,4-dioxane (5.00 mL) solution, and then 6-(5-chloropyrimidin-2-yl)-2-azaspiro[3.3]heptane hydrobromide **(B1)** (47.4 mg, 163 µmol, HBr) and N,N-diisopropylethylamine (87.9 mg, 680 µmol, 118 µL) were added. The reaction mixture was stirred at 100°C for 2 hours. After the reaction was completed, the reaction solution was concentrated and evaporated to dryness. The crude product was purified by high-performance liquid chromatography (column: Boston Green ODS 150×30 mm×5 µm; solvent: A=water+0.05 volume formic acid (99%), B=acetonitrile; gradient: 15% to 45%, 11 minutes), to obtain Compound (R)-2-(6-(5-chloropyrimidin-2-yl)-2-azaspiro[3.3]heptan-2-yl)-4-((1-(hydroxymethyl)cyclobutyl-2,2,3,3,4,4-d6)amino)-6,7-dihydrothieno[3,2-d]pyrimidine 5-oxide (Compound 7) (17.0 mg, yield 27.6%).

LC-MS, M/Z (ESI): 467.3 [M+H]⁺. ¹H NMR (400 MHz, CDCl₃) *δ* 8.63 (s, 2H), 5.81 (s, 1H), 4.01-4.37 (m, 4H), 3.85 (s, 2H), 3.54-3.74 (m, 2H), 3.35-3.54 (m, 1H), 2.88-3.17 (m, 2H), 2.55-2.77 (m, 4H)

Example 6: The synthesis of each of the following compounds was carried out with reference to the preparation method of Example 1:

| Structure | Name | LCMS | H-NMR |
|---|---|---|---|
| 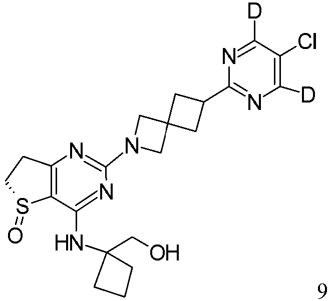 | (R)-2-(6-(5-chloro(4,6-d2)pyrimidin-2-yl)-2-azaspiro[3.3]hept-2-yl)-4-((1-(hydroxymethyl)cyclobutyl )amino)-6,7-dihydrothieno[3,2-d]pyrimidin-5-oxide | 463 | ¹H NMR (400 MHz, DMSO*_d₆*) *δ* = 7.33 (s, 1H), 4.87 (m, 1H), 4.12 (s, 2H), 3.95 (s, 2H), 3.64-3.72 (m, 3H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H). |
| 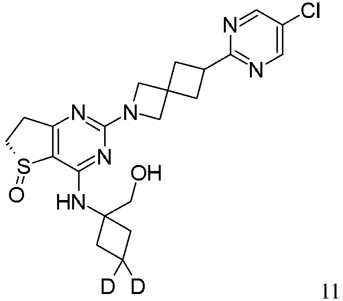 | (R)-2-(6-(5-chloropyrimidin-2-yl)-2-azaspiro[3.3]hept-2-yl)-4-((1-(hydroxymethyl)cyclobutyl -3,3-d₂)amino)-6,7-dihydrothieno[3,2-d]pyrimidin-5-oxide | 463 | ¹H NMR (400 MHz, DMSO_*d₆*) *δ* = 8.87 (s, 2H), 7.33 (s, 1H), 4.87 (m, 1H), 4.12 (s, 2H), 3.95 (s, 2H), 3.64-3.72 (m, 3H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H). |
| 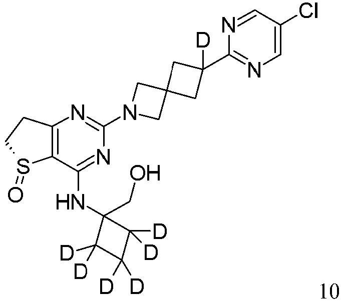 | (R)-2-(6-(5-chloropyrimidin-2-yl)-6-deutero-2-azaspiro[3.3]hept-2-yl)-4-((1-(hydroxymethyl)cyclobutyl -2,2,3,3,4,4-d₆)amino)-6,7-dihydrothieno[3,2-d]pyrimidin-5-oxide | 468 | LC-MS, M/Z (ESI): 467.3 [M+H]⁺. ¹H NMR (400 MHz, CDCl₃) *δ* 8.63 (s, 2H), 5.81 (s, 1H), 4.01-4.37 (m, 4H), 3.85 (s, 2H), 3.54-3.74 (m, 2H), 2.88-3.17 (m, 2H), 2.55-2.77 (m, 4H). |
| 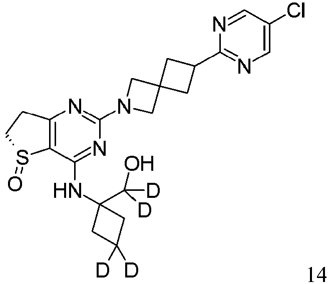 | (R)-2-(6-(5-chloropyrimidin-2-yl)-2-azaspiro[3.3]hept-2-yl)-4-((1-(hydroxymethyl-d₂)cyclobutyl-3,3-d₂)amino)-6,7-dihydrothieno[3,2-d]pyrimidin-5-oxide | 465 | ¹H NMR (400 MHz, DMSO*_d₆*) *δ* = 8.87 (s, 2H), 7.33 (s, 1H), 4.87 (m, 1H), 4.12 (s, 2H), 3.64-3.72 (m, 3H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H) |
| 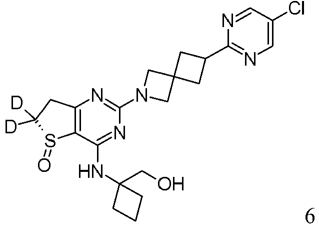 | (R)-2-(6-(5-chloropyrimidin-2-yl)-2-azaspiro[3.3]heptan-2-yl)-4-((1-(hydroxymethyl)cyclobutyl )amino)-(6,6-d₂)-6,7-dihydrothieno[3,2-d]pyrimidin-5-oxide | 463 | ¹H NMR (400 MHz, DMSO_d*₆*) *δ* = 8.87 (s, 2H), 7.33 (s, 1H), 4.87 (m, 1H), 4.12 (s, 2H), 3.64-3.72 (m, 3H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H). |
| 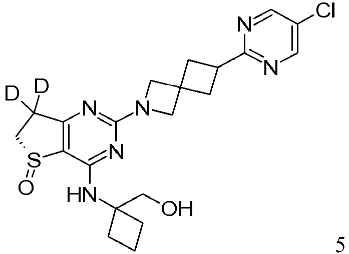 | (R)-2-(6-(5-chloropyrimidin-2-yl)-2-azaspiro[3.3]heptan-2-yl)-4-((1-(hydroxymethyl)cyclobutyl )amino)-(7,7-d₂)-6,7-dihydrothieno[3,2-d]pyrimidin-5-oxide | 463 | ¹H NMR (400 MHz, DMSO*_d₆*) *δ* = 8.87 (s, 2H), 7.33 (s, 1H), 4.87 (m, 1H), 4.12 (s, 2H), 3.95 (s, 2H), 3.64-3.72 (m, 1H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H). |
| 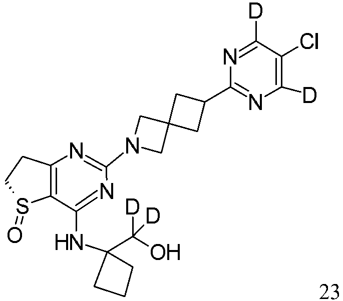 | (R)-2-(6-(5-chloro(4,6-d₂)pyrimidin-2-yl)-2-azaspiro[3.3]hept-2-yl)-4-((1-(hydroxymethyl-d₂)cyclobutyl)amino)-6,7-dihydrothieno[3,2-d]pyrimidin-5-oxide | 465 | ¹H NMR (400 MHz, DMSO*_d₆*) *δ* = 7.33 (s, 1H), 4.87 (m, 1H), 3.95 (s, 2H), 3.64-3.72 (m, 3H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H). |
| 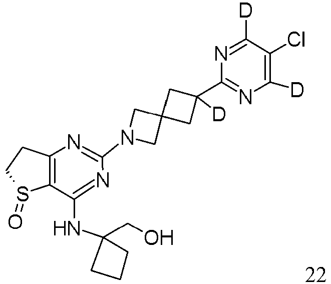 | (R)-2-(6-(5-chloro(4,6-d₂)pyrimidin-2-yl)-6-deutero-2-azaspiro[3.3]hept-2-yl)-4-((1-(hydroxymethyl)cyclobutyl )amino)-6,7-dihydrothieno[3,2-d]pyrimidin-5-oxide | 464 | ¹H NMR (400 MHz, DMSO_*d₆*) *δ* = 7.33 (s, 1H), 4.87 (m, 1H), 4.12 (s, 2H), 3.95 (s, 2H), 3.64-3.72 (m, 2H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H). |
| 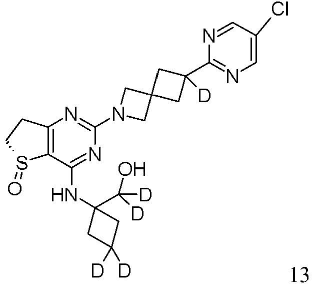 | (R)-2-(6-(5-chloropyrimidin-2-yl)-6-deutero-2-azaspiro[3.3]hept-2-yl)-4-((1-(hydroxymethyl-d₂)cyclobutyl-3,3-d₂)amino)-6,7-dihydrothieno[3,2-d]pyrimidin-5-oxide | 466 | ¹H NMR (400 MHz, DMSO_*d₆*) *δ* = 8.87 (s, 2H), 7.33 (s, 1H), 4.87 (m, 1H), 4.12 (s, 2H), 3.64-3.72 (m, 2H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H). |
| 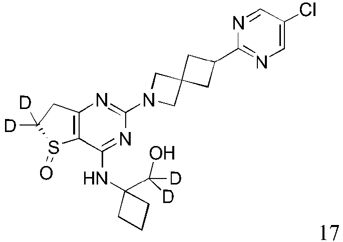 | (R)-2-(6-(5-chloropyrimidin-2-yl)-2-azaspiro[3.3]hept-2-yl)-4-((1-(hydroxymethyl-d₂)cyclobutyl)amino)-(6,6-d₂)-6,7-dihydrothieno[3,2-d]pyrimidin-5-oxide | 465 | ¹H NMR (400 MHz, DMSO_*d₆*) *δ* = 8.87 (s, 2H), 7.33 (s, 1H), 4.87 (m, 1H), 3.64-3.72 (m, 3H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H). |
| 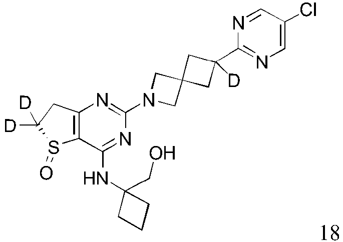 | (R)-2-(6-(5-chloropyrimidin-2-yl)-6-deutero-2-azaspiro[3.3]hept-2-yl)-4-((1-(hydroxymethyl)cyclobutyl )amino)-(6,6-d₂)-6,7-dihydrothieno[3,2-d]pyrimidin-5-oxide | 464 | ¹H NMR (400 MHz, DMSO_*d₆*) *δ* = 8.87 (s, 2H), 7.33 (s, 1H), 4.87 (m, 1H), 4.12 (s, 2H), 3.64-3.72 (m, 2H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H). |
| 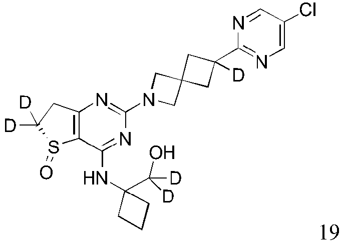 | (R)-2-(6-(5-chloropyrimidin-2-yl)-6-deutero-2-azaspiro [3.3]hept-2-yl)-4-((1-(hydroxymethyl-d₂)cyclobutyl)amino)-(6,6-d₂)-6,7-dihydrothieno[3,2-d]pyrimidin-5-oxide | 466 | ¹H NMR (400 MHz, DMSO_*d₆*) *δ* = 8.87 (s, 2H), 7.33 (s, 1H), 4.87 (m, 1H), 3.64-3.72 (m, 2H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H). |

### Synthesis of Control Compound 1:

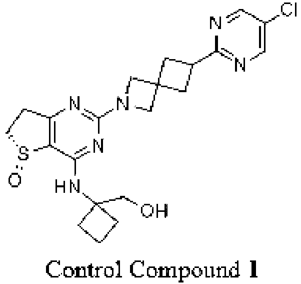

The synthetic route is as follows:

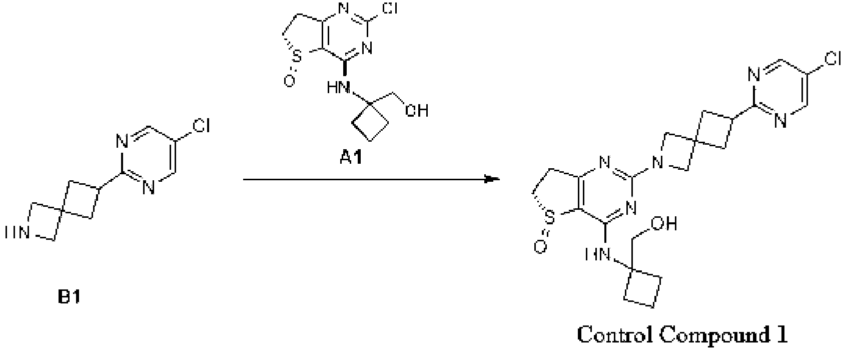

### Step 1: Synthesis of: (R)-2-((6-(5-chloropyrimidin-2-yl)- 2-azaspiro[3.3]heptan-2-yl))-4-((1-(hydroxymethyl)cyclobutyl)amino)-6,7-dihydrothieno[3,2-d]pyrimidine 5-oxide (Control Compound 1)

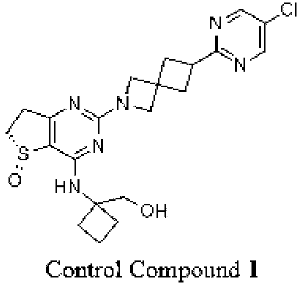

6-(5-chloropyrimidin-2-yl)-2-azaspiro[3.3]heptane (Intermediate B1) (60.0 mg, 219 umol) and N,N-diisopropylethylamine (141 mg, 1.10 mmol) were added to a solution of (R)-2-chloro-4-((1-(hydroxymethyl)cyclobutyl)amino)-6,7-dihydrothieno[3,2-d]pyrimidine 5-oxide (64.4 mg, 219 µmol) in 1,4-dioxane (10 mL). The mixture was slowly heated to 100°C and reacted for 3 hours. The mixture was extracted three times with dichloromethane (60 mL). The organic phase was combined, dried over sodium sulfate and concentrated. The crude product was purified by reversed-phase high-performance liquid chromatography (column: Waters Xbridge 150×25 mm 5 µm; solvent: A=water+0.05% ammonia solution, B=acetonitrile; gradient (acetonitrile): 18% to 48%, 9 minutes), to obtain Compound (R)-2-((6-(5-chloropyrimidin-2-yl)-2-azaspiro[3.3]heptan-2-yl)-4-((1-(hydroxymethyl)cyclobutyl)amino)-6,7-dihydrothieno[3,2-d]pyrimidine 5-oxide (Control Compound **1**). LC-MS, M/Z (ESI): 461.3 [M+H]⁺. ¹H NMR (400 MHz, DMSO_*d₆*) *δ*=8.87 (s, 2H), 7.33 (s, 1H), 4.87 (m, 1H), 4.12 (s, 2H), 3.95 (s, 2H), 3.64-3.72 (m, 3H), 3.36-3.42 (m, 1H), 3.16-3.23 (m, 1H), 2.82-2.94 (m, 2H), 2.58-2.65 (m, 3H), 2.37 (br d, 2H), 2.30 (br s, 1H), 2.10-2.15 (m, 2H), 1.71-1.82 (m, 2H).

### Synthesis of Control Compound 2:

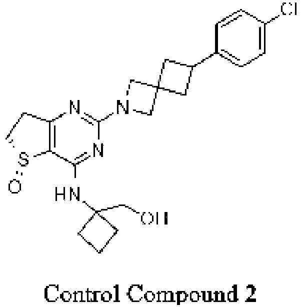

The synthetic route is as follows:

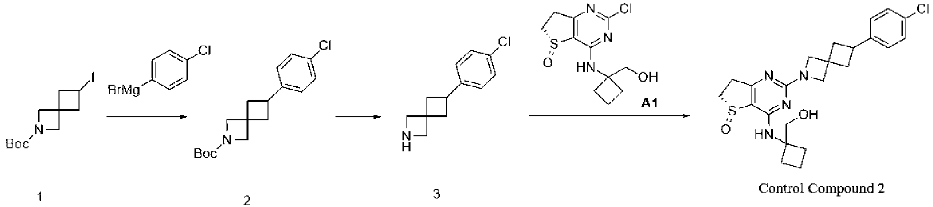

### Step 1: Synthesis of tert-butyl 6-iodo-2-azaspiro[3.3]heptane-2-carboxylate

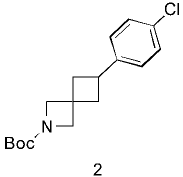

Tert-butyl 6-iodo-2-azaspiro[3.3]heptane-2-carboxylate (10 g, 30.9 mmol), 4-chlorophenylmagnesium bromide (25.8 mL, 62.0 mmol, 2.4 M in THF), and Pd(dppf)Cl₂ (2.2 g, 3.1 mmol) were sequentially added to 100 mL of anhydrous THF. The mixture was reacted at 50°C under a nitrogen atmosphere for 16 hours. After the reaction system was cooled to room temperature, the mixture was concentrated. The resulting crude product was purified by silica gel column chromatography (PE/EA=6/1) to obtain tert-butyl 6-iodo-2-azaspiro[3.3]heptane-2-carboxylate (8.1 g, yellow oil).

### Step 2: Synthesis of 6-(4-chlorophenyl)-2-azaspiro[3.3]heptane

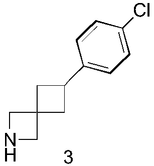

Tert-butyl 6-iodo-2-azaspiro[3.3]heptane-2-carboxylate (4.5 g, 14.6 mmol) and BBr₃ (5.5 g, 22 mmol) were sequentially added to 100 mL of dry DCM. The reaction mixture was reacted at room temperature for 4 hours and concentrated to obtain 6.1 g of crude product as a light yellow oil, which was used directly in the next step.

### Step 3: (R)-2-(6-(4-chlorophenyl)-2-azaspiro[3.3]heptan-2-yl)-4-((1-(hydroxymethyl-d₂)cyclobutyl)amino)-6,7-dihydrothieno[3,2-d]pyrimidine 5-oxide (Control Compound 2)

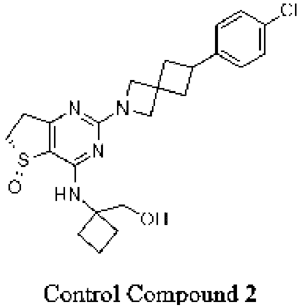

Intermediate A1 (3.7 g, 13.1 mmol), 6-(4-chlorophenyl)-2-azaspiro[3.3]heptane (6.1 g crude product, 14.6 mmol), and DIPEA (9.4 g, 73 mmol) were sequentially added to a THF/water (40/20 mL) mixed solvent system. The reaction mixture was reacted at 85°C for 2 hours, cooled to room temperature, and concentrated. The crude product was quickly purified by silica gel column chromatograph to obtain the crude product, followed by Pre-HPLC purification and lyophilization, to obtain (R)-2-(6-(4-chlorophenyl-2-yl)-2-azaspiro[3.3]heptan-2-yl)-4-((1-(hydroxymethyl-d₂)cyclobutyl)amino)-6,7-dihydrothieno[3,2-d]pyrimidine 5-oxide (Control Compound 2) (1.2 g, two-step yield 17.9%). LC-MS, M/Z (ESI): 459 [M+H]⁺. ¹H NMR (400 MHz, CDCl₃) δ 7.25 (t, 2H), 7.09 (d, 2H), 6.08 (s, 1H), 5.55 (t, 1H), 4.25 (s, 2H), 4.04 (s, 2H), 3.81 (d, 2H), 3.65-3.53 (m, 1H), 3.39 (dt, 2H), 3.09-2.94 (m, 2H), 2.62 (t, J=9.8 Hz, 2H), 2.30 (t, 4H), 2.24-2.09 (m, 2H), 2.03-1.81 (m, 2H).

### Test Example 1: Enzyme Activity Inhibition Assay of Test Compounds Against PDE4B2 and PDE4D2

Inhibitory activities of the compounds of the present disclosure against PDE4B2 and PDE4D2 can be detected using AMP-Glo^{™} Assay kit (Promega, V5011). First, all compounds were prepared as stock solution at 20 or 25 mM in DMSO, followed by 3-fold serial dilution in DMSO on a compound plate to obtain 200×working solutions. Meanwhile, 200×Rolipram (2 mM) was prepared as a positive control, and 100% DMSO was served as a blank control. The compound plate was centrifuged at 1,000 rpm for 1 minute. 20 nL of the compound working solution was transferred to a 384-well assay plate (Greiner, 784075) using Echo 550, and the plate was sealed and then centrifuged at 1,000 rpm for 1 minute. PDE4B2 enzyme (BPS bioscience, 60042) and PDE4D2 enzyme (BPS bioscience, 60048) were diluted to 2×working solutions using ice-cold PDE assay buffer. 2 µL of 2×PDE4B2 (6.4 pg/µL) or 2×PDE4D2 (4 pg/µL) working solution was added to each well of the 384-well assay plate, and the plate was sealed and then equilibrated at room temperature for 10 minutes. Cyclic-3',5'-AMP (sigma, A6882) was diluted to a 2×working solution using PDE assay buffer, and 2 µL of 2×Cyclic-3',5'-AMP (2 µM) working solution was added to each well of the 384-well assay plate, followed by incubation at room temperature for 60 minutes. AMP was detected using the AMP-Glo^{™} Assay kit (Promega, V5011): 4 µL of AMP-Glo Reagent I was added to each well of the 384-well assay plate, followed by incubation at room temperature for 60 min; then 8 µL of AMP Detection Reagent was added to each well of the 384-well assay plate, followed by incubation at room temperature for 60 minutes. RLU (relative light unit) signals were read using an Envision 2105 instrument. An inhibition rate of the test compounds at different concentrations was calculated as follows: Inhibition (%)=(1-(RLU compound - RLU positive control)/(RLU blank control - RLU positive control))×100%. The experimental results were imported into GraphPad Prism software, and the IC₅₀ value of each compound was calculated by curve fitting. The results demonstrated that the PDE4B2 IC₅₀ value of each compound in the present disclosure was lower than its PDE4D2 IC₅₀ value, indicating significant selectivity. This example exemplarily presents the data of some compounds, as detailed in Table 1.

**[Table 1] IC₅₀ Values of Compounds**

| Compound number | PDE4B2 IC₅₀ (nM) | PDE4D2 IC₅₀ (nM) |
|---|---|---|
| Compound 2 | 23.9 | 50.2 |
| Compound 3 | 14.5 | 45.1 |
| Compound 4 | 17.5 | 42.7 |

The experimental results showed that the compounds of the present disclosure exhibited significant inhibitory activity against PDE4B and can act as a selective inhibitor of PDE4B.

### Test Example 2: Pharmacokinetic Study

Pharmacokinetic studies were conducted on miniature pigs, and male miniature pigs weighing 10 kg to 12 kg was used. Three miniature pigs in each group were fasted and administered with the compound via oral gavage at 10 mg/kg. Blood samples were collected before the administration, and 15 minutes, 30 minutes, 1 hour, 2 hours, 4 hours, 8 hours, and 24 hours after the administration. Three additional miniature pigs in each group were administered with the compound via intravenous injection at 3 mg/kg. Blood samples were collected before the administration, and 5 minutes, 15 minutes, 30 minutes, 1 hour, 2 hours, 4 hours, 8 hours, and 24 hours after the administration. Whole blood samples were placed in EDTA-K₂ tubes for anticoagulation, then centrifuged (at 1,500g to 1,600 g) at 4 °C for 10 minutes to separate plasma. All the samples were stored at -80°C until analysis. 20 µL of plasma collected at each time point was mixed with 400 µL of acetonitrile-methanol aqueous solution containing internal standard. After vortex mixing, the mixture was centrifuged at 3,700 rpm for 10 minutes. 50 µL of the supernatant was added to 100 µL of water, followed by vortex-mixing. An appropriate amount of the mixed solution was subjected to LC-MS/MS analysis. The main plasma pharmacokinetic parameters were calculated using a noncompartmental model.

The experimental results showed that the compounds of the present disclosure exhibited excellent pharmacokinetic properties.

### Test example 3: Caco-2 Cell Permeability Assay

Caco-2 cells were seeded onto a 96-well Transwell plate at a density of 1×10⁵ cells/cm², and the culture medium was refreshed every 4 to 5 days until a confluent cell monolayer was formed on Day 28. Integrity of the Caco-2 cell monolayer was verified using HBSS solution containing 100 µM Lucifer Yellow. Then, the following assays were then carried out in the presence and absence of Elacridar (10 µM). For A-to-B (Apical-to-Basolateral) group, the test compound was added to a donor side of the 96-well Transwel1 plate at a final concentration of 2 µM, while buffer was added to a receiver side of the 96-well Transwel1 plate. Then, the plates were incubated in a CO₂ incubator at 37 °C with 5% CO₂ and saturated humidity for 2 hours. At an end of the incubation, samples were collected from both the donor side and the receiver side simultaneously. All the samples were mixed with acetonitrile containing the internal standard, centrifuged at 3,200g for 10 minutes, and the supernatants were collected. Then, a concentration of the compounds was detected by LC-MS/MS. The assay was carried out at the same conditions in B-to-A (Basolateral-to-Apical) group. Finally, Papp (10⁻⁶cm/sec) and an efflux rate were calculated in accordance with the following equations: 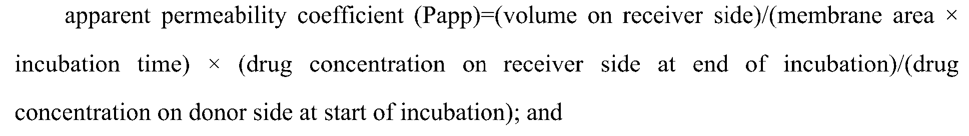 apparent permeability coefficient (Papp)=(volume on receiver side)/(membrane area × incubation time) × (drug concentration on receiver side at end of incubation)/(drug concentration on donor side at start of incubation);and$efflux rate \left(ER\right) = Papp \left({}_{B - A}\right) / Papp \left({}_{A - B}\right) .$

The experimental results showed that the compounds of the present disclosure exhibited excellent permeability.

### Test Example 4: Inhibition Assay of Test Compounds on LPS-induced TNF-α Secretion by Human PBMCs

Fresh blood samples were diluted with an equal volume of PBS (BI, 02-024-01ACS) for later use. 15 mL of Lymphoprep (stem cell, 7851) was added into a 50 mL centrifuge tube, and 30 mL of diluted blood sample was slowly layered onto the Lymphoprep without disturbing the interface. The mixture was centrifuged at 1,000 g for 25 minutes at room temperature with the brake off. The buffy coat containing PBMCs was collected into a new 50 mL centrifuge tube, washed twice with 50 mL of DPBS, and centrifuged at 350 g for 10 minutes. The supernatant was discarded. The cells were resuspended in complete RPMI 1640 medium and adjusted to a density of 1.5×10⁶ cells/mL. A 96-well cell culture plate was taken, and 100 µL of the PBMC suspension was added to each well. The test compounds were prepared as 4× working solutions at a series of concentrations using complete RPMI 1640 medium, and 50 µL of the compound working solution was added to the corresponding wells. Meanwhile, an LPS control group (without the compounds) and a DMSO control group (without LPS and the compound) were set. After the plate was incubated at 37°C in a CO₂ incubator for 1 hour, 50 µL of LPS (at a final concentration 0.1 µg/mL) was added to the corresponding wells, and the plate was further incubated in an incubator for 4 hours. After the completion of the incubation, the supernatant was collected by centrifugation. The expression of TNF-α was measured using a Human TNF-α ELISA Kit (Cat: 555212) from BD Biosciences. An inhibition rate of the test compounds on the LPS-induced TNF-α secretion by the human PBMCs was calculated. Based on the inhibition rates at different concentrations of the compounds, IC₅₀ values for the inhibition of the TNF-α secretion by the LPS-stimulated human PBMCs were measured by curve fitting using GraphPad Prism 8 software. This example exemplarily represents the data of some of the compounds, as detailed in Table 2. 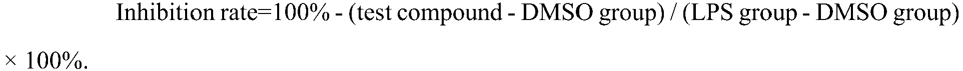 Inhibition rate=100% - (test compound - DMSO group) / (LPS group - DMSO group) × 100%.

**[Table 2] Inhibition Results of Compounds on LPS-induced TNF-α Secretion by Human PBMCs**

| Compound Number | IC₅₀ (nM) |
|---|---|
| Compound 3 | 41.4 |
| Compound 2 | 57.8 |
| Control Compound 1 | 63.4 |

The test results showed that compared to Control Compound 1, the compounds of the present disclosure exhibited superior inhibitory activity on the LPS-induced TNF-α secretion by the human PBMCs; and Compound 2 and Compound 3 of the present disclosure exhibited superior inhibitory activity on the LPS-induced TNF-α secretion by the human PBMCs, and in particular, Compound 3 exhibited a significant improvement in the inhibitory effect on the LPS-induced TNF-α secretion by human PBMCs, indicating stronger antiinflammatory activity.

### Test Example 5: Pharmacokinetic Study

For the pharmacokinetic study in monkeys, male cynomolgus monkeys weighing 5 kg to 7 kg were used and fasted overnight. Three cynomolgus monkeys were administered with the compound via oral gavage at 10 mg/kg. Blood samples were collected before the administration, and 15 minutes, 30 minutes, 1 hour, 2 hours, 4 hours, 8 hours, and 24 hours after the administration. Whole blood samples were placed in EDTA K₂ tubes for anticoagulation, and then centrifuged (at 1,500 g to 1,600 g) at 4°C for 10 minutes to separate plasma. All the samples were stored at -80°C until analysis. 20 µL of plasma collected at each time point was mixed with 400 µL of acetonitrile-methanol solution containing internal standard. After vortex mixing, the mixture was centrifuged at 3,700 rpm for 10 minutes. 50 µL of the supernatant was added to 100 µL of water, followed by vortex-mixing. An appropriate amount of the mixed solution was subjected to LC-MS/MS analysis. The main plasma pharmacokinetic parameters were calculated using a noncompartmental model.

**[Table 3] Pharmacokinetic Results in Cynomolgus Monkeys After Oral Gavage Administration**

| Compound Number | Pharmacokinetic Parameters in Cynomolgus Monkeys | | |
|---|---|---|---|
| | Oral Gavage Administration (10 mg/kg) | | |
| | Cₘₐₓ (ng/mL) | AUC₀₋ₜ (h·ng/mL) | CL/F (mL/h/kg) |
| Compound 3 | 2,426 | 19,549 | 506 |
| Control Compound 1 | 2,061 | 9,793 | 1,006 |

PK properties were evaluated in monkeys, which have metabolic characteristics closer to humans. The results showed that the compounds of the present disclosure exhibited significantly improved area under the curve AUC₀₋ₜ and Cₘₐₓ, as well as lower clearance rate, indicating superior pharmacokinetic properties. The area under the curve AUC₀₋ₜ of Compound 3 of the present disclosure was twice that of Control Compound 1, with a markedly increased Cₘₐₓ, better oral absorption, and lower clearance rate. The Compound 3 of the present disclosure exhibited superior pharmacokinetic properties, with an expected lower dosage and reduced side effects.

### Test Example 6: In Vitro Toxicity Evaluation of Compounds on Human HepG2 Cells

HepG2 cells in a logarithmic growth phase were trypsinized and resuspended in MEM complete medium containing 10% FBS. 10,000 cells were seeded in each well of a 96-well plate (no cells were seeded in the blank control group) with a seeding volume of 100 µL/well. The plates were incubated at 37°C in a CO₂ incubator for 24 hours.

The test compounds were prepared as 50 mM stock solutions in DMSO, serially diluted to appropriate concentrations in DMSO, and further 3-fold serially diluted with cell culture medium to obtain 2× working solutions. 100 µL of the test compound working solution was added to the corresponding wells of the 96-well plate (n=2). Meanwhile, a blank control group (cell culture medium containing 0.5% DMSO) and a DMSO control group (cell culture medium containing 0.5% DMSO) were set. After the plate was incubated at 37°C in a CO₂ incubator for 72 hours, 100 µL of the supernatant was aspirated from each well and discarded. Then, 50 µL of CTG (Promega, G9243) was added to each well. The plate was shaken on a microplate shaker for 5 minutes, allowed to stand for 10 min, and the luminescence value was read using a microplate reader. The inhibition rate of the test compounds on the human HepG2 cells was calculated, and the IC₅₀ values of the compound on human HepG2 cells were fitted based on the inhibition rate of the compound at different concentrations. Inhibition rate=100%-(test compound-blank control group)/(DMSO group-blank control group) × 100%.

**Table 4. In vitro Toxicity Evaluation Results of Compounds on HepG2 cells**

| Test Compound | IC₅₀ (µM) | Test Compound | IC₅₀ (Mm) |
|---|---|---|---|
| Compound 1 | >200 | Compound 11 | >200 |
| Compound 2 | >200 | Compound 13 | >200 |
| Compound 3 | >200 | Compound 14 | >200 |
| Compound 4 | >200 | Compound 17 | >200 |
| Compound 5 | >200 | Compound 18 | >200 |
| Compound 6 | >200 | Compound 19 | >200 |
| Compound 7 | >200 | Compound 23 | >200 |
| Compound 9 | >200 | Compound 22 | >200 |
| Compound 10 | >200 | Control Compound 2 | 31.2 |

The test results showed that the inhibitory effect of Compound 3 of the present disclosure on the human HepG2 cells was significantly weaker than that of Control Compound 2. Thus, the compounds of the present disclosure exhibited lower toxicity to human hepatocytes and were expected to have better safety for long-term administration.

### Test Example 7: Mouse Tissue Distribution Assay

For the mouse tissue distribution assay, ICR mice weighing 20 g to 25 g were used and fasted overnight. 8 male mice and 4 female mice were administered via oral gavage at 10 mg/kg. Blood samples as well as tissue samples including stomach, small intestine, and large intestine were collected at 30 minutes, 1 hour, 4 hours, and 8 hours after the administration. Two male mice and one female mouse were used at each time point. Whole blood samples were placed in EDTA-K₂ tubes for anticoagulation, and then centrifuged (at 1,500 g to 1,600 g) at 4°C for 10 minutes to separate plasma. All plasma and tissue samples were stored at -80°C until analysis. Compound concentrations in plasma and homogenate samples (stomach, small intestine, and large intestine) from mice were measured by LC-MS/MS, and area under the concentration-time curve and tissue/plasma area under the curve ratio were calculated based on the compound concentrations at different time points.

**[Table 5] Results of Mouse Tissue Distribution Assay**

| Tissue | Area under the concentration-time curve AUC₍₀₋ₜ₎ (h*ng/ml or h*ng/g) | | | |
|---|---|---|---|---|
| | Compound 3 | | Control Compound 1 | |
| | | tissue/plasma ratio | | tissue/plasma ratio |
| Plasma | 15,998 | - | 12,487 | - |
| Stomach | 48,722 | 3.05 | 222,882 | 17.85 |
| Small Intestine | 44,273 | 2.77 | 61,044 | 4.89 |
| Large Intestine | 42,506 | 2.66 | 105,410 | 8.44 |

The test results showed that the compounds of the present disclosure exhibited lower gastrointestinal distribution in the mice, with significantly reduced tissue/plasma area under the curve ratio, and are expected to have fewer gastrointestinal side effects such as vomiting and diarrhea for long-term administration, indicating high safety.

Although embodiments of the present disclosure have been shown and described, it would be appreciated that the above embodiments are exemplary and cannot be construed to limiting the present disclosure, and those skilled in the art can make changes, modifications, alternatives, and alterations to the above embodiments by without departing from scope of the present disclosure.

## Claims

1. A compound represented by Formula I, or a tautomer, stereoisomer, hydrate, solvate, pharmaceutically acceptable salt or prodrug thereof: 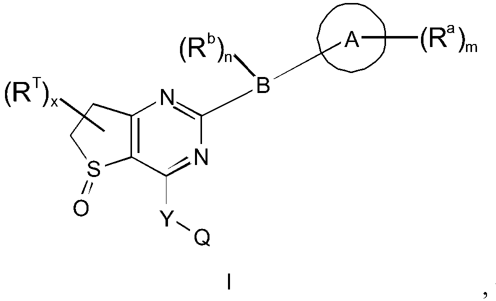 wherein:
ring A is a 5- to 10-membered aromatic ring or a 5- to 10-membered heteroaromatic ring;
each R^{T} is independently H, halogen, hydroxyl, deuterium, C₁₋₆ alkyl, C₁₋₆ alkoxy, C₃-₆ cycloalkyl, deuterated C₃-₆ cycloalkyl, deuterated C₁₋₆ alkyl, or deuterated C₁₋₆ alkoxy, wherein the C₁₋₆ alkyl, the C₁₋₆ alkoxy, the C₃-₆ cycloalkyl, the deuterated C₃-₆ cycloalkyl, the deuterated C₁₋₆ alkyl, and the deuterated C₁₋₆ alkoxy are optionally substituted with one or more substituents of halogen, hydroxyl, amino, nitro, cyano, or carbonyl, and wherein when a plurality of substituents are present, the plurality of substituents are identical or different;
B is 3- to 10-membered heterocycloalkyl, 3- to 10-membered heterocycloalkenyl, or 3- to 10-membered cycloalkyl;
R^{a} and R^{b} are each independently H, deuterium, halogen, hydroxyl, amino, nitro, cyano, carbonyl, oxo, carboxyl, C₁₋₆ alkyl, C₃₋₈ cycloalkyl, deuterated C₁₋₆ alkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, deuterated C₁₋₆ alkoxy, or 3- to 10-membered heterocycloalkyl, wherein the C₁₋₆ alkyl, the C₃₋₈ cycloalkyl, the deuterated C₁₋₆ alkyl, the C₂₋₆ alkenyl, the C₂₋₆ alkynyl, the C₁₋₆ haloalkyl, the C₃₋₈ halocycloalkyl, the C₁₋₆ hydroxyalkyl, the C₁₋₆ alkylcarbonyl, the C₁₋₆ alkoxy, and the C₁₋₆ haloalkoxy are optionally substituted with one or more R^{c}, wherein the one or more R^{c} are each deuterium, halogen, hydroxyl, amino, nitro, cyano, carbonyl, oxo, carboxyl, C₁₋₆ alkyl, deuterated C₁₋₆ alkyl, C₂₋₆ alkynyl, C₁₋₆ haloalkyl, C₁₋₆ hydroxyalkyl, C₁₋₆ alkylcarbonyl, C₁₋₆ alkoxy, or C₁₋₆ haloalkoxy, and wherein when a plurality of substituents R^{c} are present, the plurality of substituents R^{c} are identical or different;
Y is NR¹, O, S, or C(R¹)₂;
each R¹ is independently H, deuterium, C₁₋₁₀ alkyl, or C₂₋₆ alkenyl, wherein the C₁₋₁₀ alkyl and the C₂₋₆ alkenyl are optionally substituted with one or more R^{d}, wherein the one or more R^{d} are each deuterium, halogen, C₁-₃ haloalkyl, C₁₋₆ alkoxy, C₁₋₆ haloalkoxy, -COO-C₁-₆ alkyl, - C(O)NR^{f}R^{f}, 5- to 8-membered aromatic ring, -het¹, or monocyclic- or bicyclic- C₅₋₈-cycloalkyl, wherein each R^{f} is hydrogen or C₁₋₆ alkyl, and wherein het¹ represents a 5- to 8-membered monocyclic or bicyclic, saturated or unsaturated heterocyclic ring comprising one, two, three, or four heteroatoms independently selected from N, S, and O;
Q is 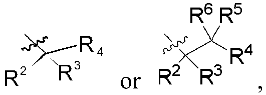 wherein:
R² and R³ as a pair as well as R⁵ and R⁶ as a pair each independently forms, together with a carbon atom attached thereto, a saturated or partially saturated 3-, 4-, 5-, or 6-membered monocyclic ring, wherein the 3-, 4-, 5-, or 6-membered monocyclic ring comprises zero, one, two, or three heteroatoms selected from N, O, or S, and wherein the 3-, 4-, 5-, or 6-membered monocyclic ring is further substituted with g R²³, wherein the g R²³ are at least one of H, deuterium, halogen, hydroxyl, amino, cyano, carbonyl, oxo, carboxyl, C₁₋₆ alkyl, C₂₋₆ alkenyl, C₂₋₆ alkynyl, deuterated C₁₋₆ alkyl, C₁₋₆ haloalkyl, C₁₋₆ hydroxyalkyl, C₁₋₆ alkylcarbonyl, C₁₋₆ alkoxy, deuterated C₁₋₆ alkoxy, C₁₋₆ haloalkoxy, -COO-C₁₋₆ alkyl, or -C(O)NR^{f}R^{f}, wherein R^{f} is hydrogen or C₁₋₆ alkyl; or
R⁵ and R⁶ are each independently H, deuterium, halogen, C₁₋₆ alkyl, deuterated C₁₋₆ alkyl, C₂₋₆ alkenyl, C₁₋₆ alkoxy, or deuterated C₁₋₆ alkoxy, wherein the C₁₋₆ alkyl, the deuterated C₁₋₆ alkyl, the C₂₋₆ alkenyl, the C₁₋₆ alkoxy, and the deuterated C₁₋₆ alkoxy are optionally substituted with one or more R^{d}, wherein the one or more R^{d} are each deuterium, halogen, C₁-₃ haloalkyl, C₁₋₆ alkoxy, C₁₋₆ haloalkoxy, -COO-C₁₋₆ alkyl, -C(O)NR^{f}R^{f}, -OC(O)NR^{f}R^{f}, 5- to 8-membered aromatic ring, -het², or monocyclic- or bicyclic-C₅₋₈-cycloalkyl, wherein R^{f} is hydrogen or C₁₋₆ alkyl, and wherein het² represents a 5- to 8-membered monocyclic or bicyclic, saturated or unsaturated heterocyclic ring comprising one, two, three, or four heteroatoms independently selected from N, S and O; and
R⁴ is H, deuterium, halogen, hydroxyl, amino, nitro, cyano, carbonyl, oxo, carboxyl, C₁₋₆ alkyl, C₃₋₈ cycloalkyl, deuterated C₁₋₆ alkyl, C₂₋₆ alkenyl, C₂₋₆ alkynyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, deuterated C₁₋₆ alkoxy, 3- to 10-membered heterocycloalkyl, wherein the C₁₋₆ alkyl, the C₃₋₈ cycloalkyl, the deuterated C₁₋₆ alkyl, the C₂₋₆ alkenyl, the C₂₋₆ alkynyl, the C₁₋₆ haloalkyl, the C₃₋₈ halocycloalkyl, the C₁₋₆ hydroxyalkyl, the C₁₋₆ alkylcarbonyl, the C₁₋₆ alkoxy, the deuterated C₁₋₆ alkoxy, the C₁₋₆ haloalkoxy, and the 3- to 10-membered heterocycloalkyl are optionally substituted with one or more R^{g}, wherein the one or more R^{g} are at least one of deuterium, halogen, hydroxyl, amino, nitro, cyano, carbonyl, oxo, carboxyl, C₁₋₆ alkyl, deuterated C₁₋₆ alkyl, C₂₋₆ alkynyl, C₁₋₆ haloalkyl, C₁₋₆ hydroxyalkyl, C₁₋₆ alkylcarbonyl, C₁₋₆ alkoxy, or C₁₋₆ haloalkoxy, and wherein when a plurality of substituents R^{g} are present, the plurality of substituents R^{g} are identical or different;
x is 1, 2, 3, or 4;
m and n are each independently 1, 2, 3, 4, 5, 6, 7, or 8;
g is 1, 2, 3, 4, 5, 6, 7, or 8, and
wherein the compound represented by Formula I satisfies the following condition:
at least one of R^{T}, R^{a}, R^{b}, R¹, R², R³, R²³, R⁴, R⁵, or R⁶ is deuterium, deuterated C₁₋₆ alkyl, or deuterated C₁₋₆ alkoxy.

2. The compound according to claim 1, wherein:
the ring A is selected from a 5- to 9-membered heteroaromatic ring; and/or
the heteroaromatic ring comprises one or two heteroatoms; and/or
the heteroatom is selected from N or O; and/or
the ring A is a pyridine ring, a pyrimidine ring, a pyrazine ring, a pyridazine ring, or benzoxazolyl; and/or
the ring A is 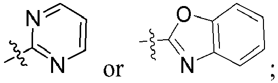 and/or
the structural unit 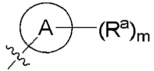 is 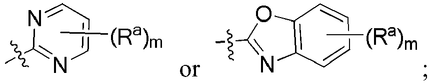 and/or
R^{a} is H, deuterium, F, Cl, C₁-₃ alkyl, deuterated C₁-₃ alkyl, or C₁-₃ haloalkyl; and/or
R^{a} is H, deuterium, F, Cl, CH₃, CD₃, or halogen-substituted CH₃; and/or
R^{a} is H, deuterium, F, Cl, CH₃, CD₃, or F-substituted CH₃; and/or
R^{a} is H, deuterium, CD₃, F, Cl, or CHF₂; and/or
the structural unit 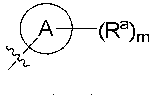 is 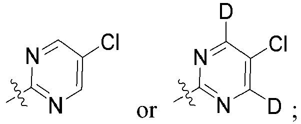 and/or
when Y is NR¹ or CHR¹, R¹ is selected from H, deuterium, or C₁₋₆ alkyl; and/or
Y is -NH-.

3. The compound according to claim 1, wherein:
B is selected from 3- to 10-membered heterocycloalkyl or 3- to 10-membered heterocycloalkenyl; and/or
the 3- to 10-membered heterocycloalkyl is monocyclic, fused bicyclic, bridged bicyclic, or spiro bicyclic; and/or
the 3- to 10-membered heterocycloalkyl further comprises one to three heteroatoms selected from N, O, and S; and/or
the 3- to 10-membered heterocycloalkenyl is monocyclic or fused bicyclic; and/or
the 3- to 10-membered heterocycloalkenyl further comprises one to three heteroatoms selected from N, O, and S; and/or
B is 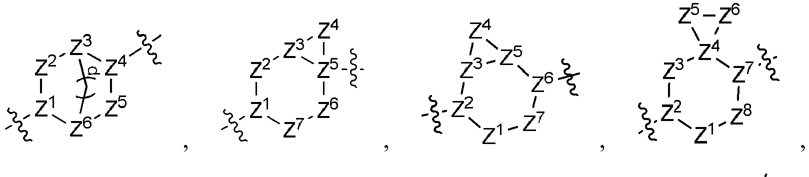 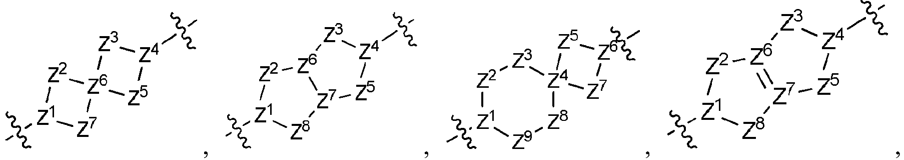 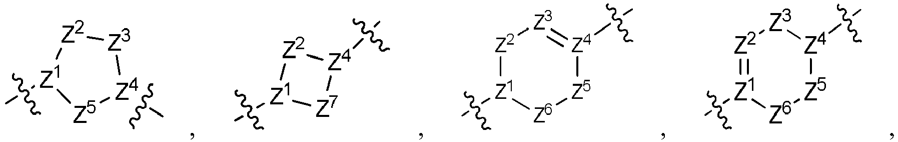 or 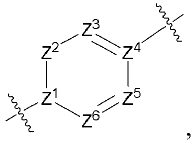 where:
Z¹, Z², Z³, Z⁴, Z⁵, Z⁶, Z⁷, Z⁸, and Z⁹ are each independently N, NH, CH₂, CH, C, -NH-CH₂-, or -CH₂-CH₂-, and p is 0, 1, or 2; and/or 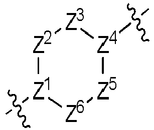 is 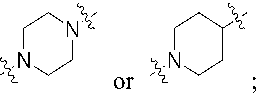 and/or 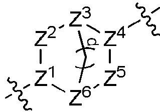 is 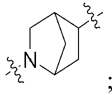 and/or 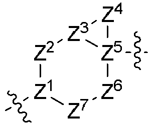 is 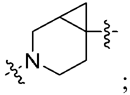 and/or 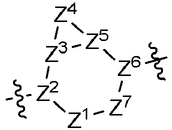 is 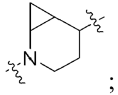 and/or 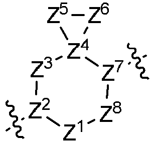 is 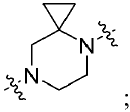 and/or 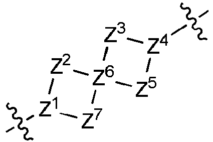 is 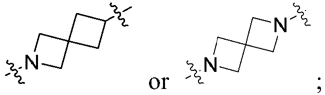 and/or 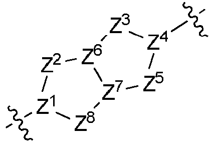 is 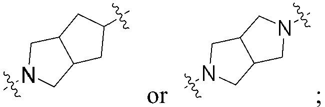 and/or 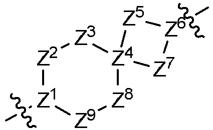 is 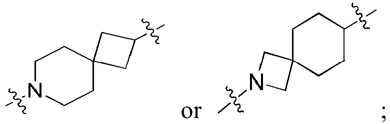 and/or 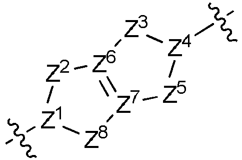 is 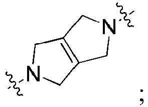 and/or 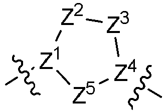 is 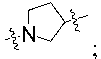 and/or 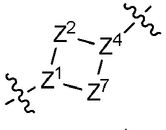 is 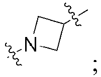 and/or 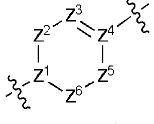 is 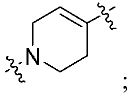 and/or 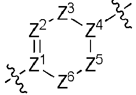 is 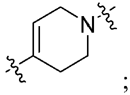 and/or 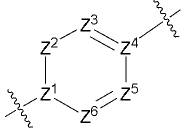 is 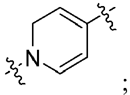 and/or
moiety 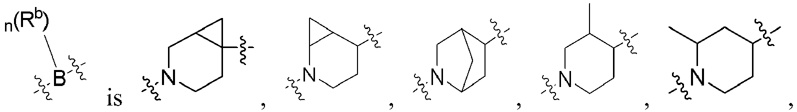 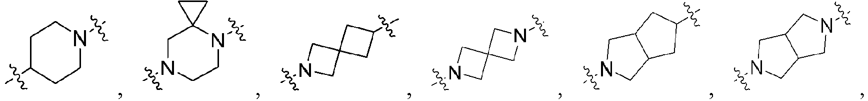 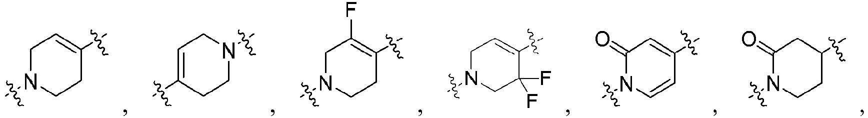 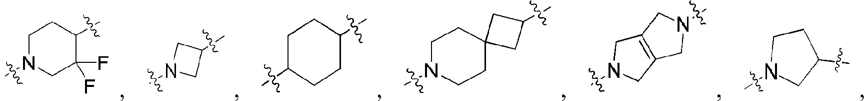 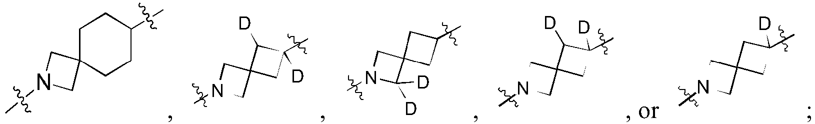 and/or
the moiety 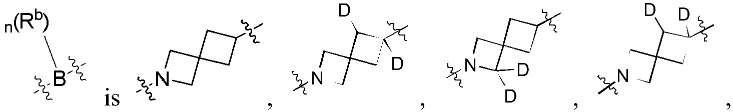 or

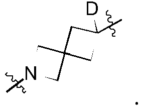

4. The compound according to claim 1, wherein:
each R^{b} is independently H, deuterium, C₁₋₆ alkyl, deuterated C₁₋₆ alkyl, halogen, C₃₋₈ cycloalkyl, or oxo; and/or
each R^{b} is independently H, deuterium, methyl, F, deuterated C₁-₃ alkyl, cycloethyl, or oxo; and/or
each R^{b} is independently H, deuterium, methyl, F, cycloethyl, or oxo; and/or
each R^{b} is independently H or deuterium; and/or
the number of R^{c} is 0, 1, 2, or 3; and/or
each R^{c} is independently deuterium, halogen, oxo, deuterated C₁₋₆ alkyl, C₁₋₆ alkyl, or C₁₋₆ haloalkyl; and/or
each R^{c} is independently deuterium, halogen, oxo, deuterated C₁-₃ alkyl, C₁-₃ alkyl, or C₁-₃ haloalkyl; and/or
the number of R^{g} is 0, 1, 2, or 3; and/or
each R^{g} is independently deuterium, halogen, hydroxyl, cyano, deuterated C₁₋₆ alkyl, C₁₋₆ alkyl, or C₁₋₆ haloalkyl; and/or
each R^{g} is independently deuterium, halogen, oxo, deuterated C₁-₃ alkyl, C₁-₃ alkyl, or C₁-₃ haloalkyl.

5. The compound according to claim 1, wherein:
each R^{T} is independently H, deuterium, deuterated C₁-₃ alkyl, or deuterated C₁-₃ alkoxy; and/or
each R^{T} is independently H or deuterium; and/or
each R⁴ is independently H, deuterium, halogen, hydroxyl, amino, nitro, cyano, carboxyl, C₁₋₆ alkyl, C₃₋₈ cycloalkyl, deuterated C₁₋₆ alkyl, C₁₋₆ haloalkyl, C₃₋₈ halocycloalkyl, C₁₋₆ hydroxyalkyl, C₁₋₆ alkylcarbonyl, C₁₋₆ alkoxy, C₁₋₆ haloalkoxy, deuterated C₁₋₆ alkoxy, or 3- to 10-membered heterocycloalkyl, wherein the C₁₋₆ alkyl, the C₃₋₈ cycloalkyl, the deuterated C₁₋₆ alkyl, the C₁₋₆ haloalkyl, the C₃₋₈ halocycloalkyl, the C₁₋₆ hydroxyalkyl, the C₁₋₆ alkylcarbonyl, the C₁₋₆ alkoxy, the C₁₋₆ haloalkoxy, the deuterated C₁₋₆ alkoxy, and the 3- to 10-membered heterocycloalkyl are optionally substituted with one or more R^{g}, wherein the one or more R^{g} are at least one of deuterium, halogen, hydroxyl, amino, nitro, cyano, carbonyl, oxo, carboxyl, C₁₋₆ alkyl, deuterated C₁₋₆ alkyl, C₂₋₆ alkynyl, C₁₋₆ haloalkyl, C₁₋₆ hydroxyalkyl, C₁₋₆ alkylcarbonyl, C₁₋₆ alkoxy, or C₁₋₆ haloalkoxy, and wherein when a plurality of substituents R^{g} are present, the plurality of substituents R^{g} are identical or different; and/or
each R⁴ is independently H, deuterium, hydroxyl, C₁₋₆ alkyl, C₃₋₈ cycloalkyl, deuterated C₁₋₆ alkyl, C₁₋₆ haloalkyl, C₃₋₈ halocycloalkyl, or C₁₋₆ hydroxyalkyl; and/or
each R⁴ is independently H, deuterium, or hydroxyl; and/or
R⁵ and R⁶ are each independently H, deuterium, C₁₋₆ alkyl, deuterated C₁₋₆ alkyl, C₂₋₆ alkenyl, C₁₋₆ alkoxy, or deuterated C₁₋₆ alkoxy, wherein the C₁₋₆ alkyl and the C₂₋₆ alkenyl are optionally substituted with one or more R^{d}, wherein the one or more R^{d} are each deuterium, halogen, C₁-₃ fluoroalkyl, C₁₋₆ alkoxy, C₁₋₆ haloalkoxy, or -COO-C₁₋₆ alkyl; and/or
R⁵ and R⁶ are each independently H, deuterium, C₁₋₆ alkyl, deuterated C₁₋₆ alkyl; and/or
R⁵ and R⁶ are each independently H or deuterium; and/or
Q is 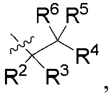 and R⁴ is hydroxyl; and/or
Q is 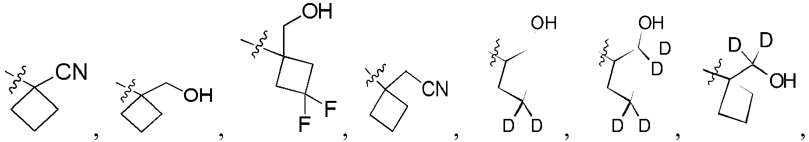 or 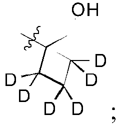 and/or
Q is 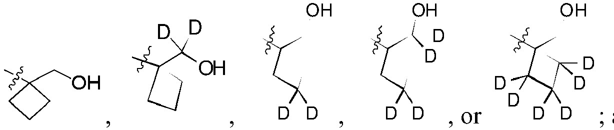 and/or 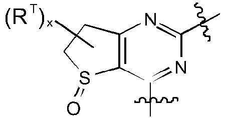 is

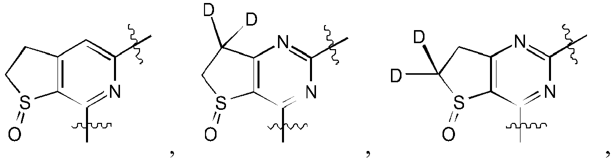

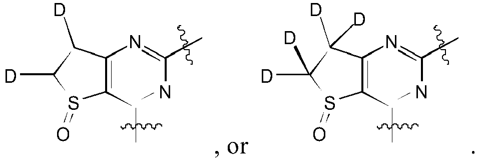

6. The compound according to claim 1, wherein the compound has a structure represented by Formula I-A or Formula I-B: 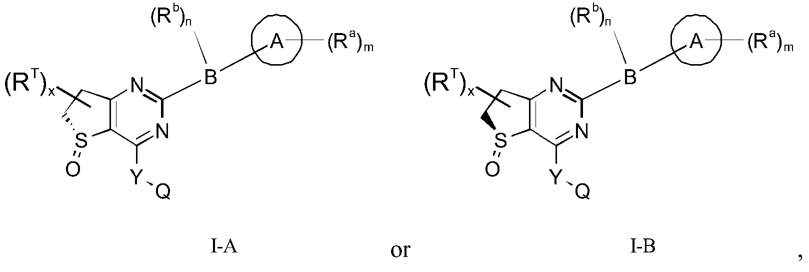 wherein: ring A, B, R^{a}, R^{b}, R^{T}, Y, Q, m, n, and x are as defined in claim 1.

7. The compound according to claim 1, wherein the compound has a structure represented by Formula I-1: 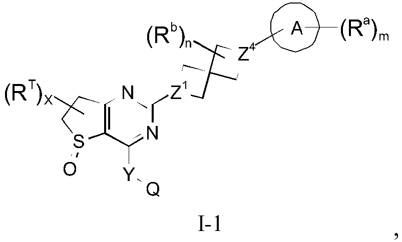 wherein:
Z¹ and Z⁴ are independently CH or N; and
ring A, R^{a}, R^{b}, R^{T}, Y, Q, m, n, and x are as defined in claim 1.

8. The compound according to claim 1, wherein the compound has a structure represented by Formula I-1A: 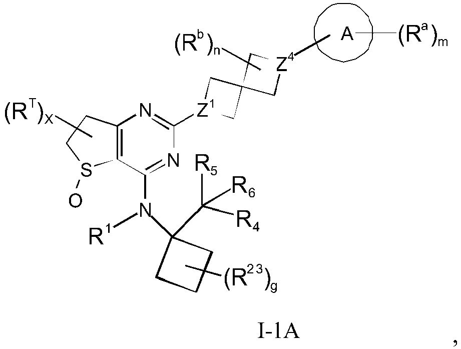 wherein:
Z¹ and Z² are independently CH or N; and
ring A, R^{a}, R^{b}, R^{T}, R¹, R²³, R⁴, R⁵, R⁶, m, n, and x are as defined in claim 1.

9. The compound according to claim 1, wherein the compound has a structure represented by Formula I-1B: 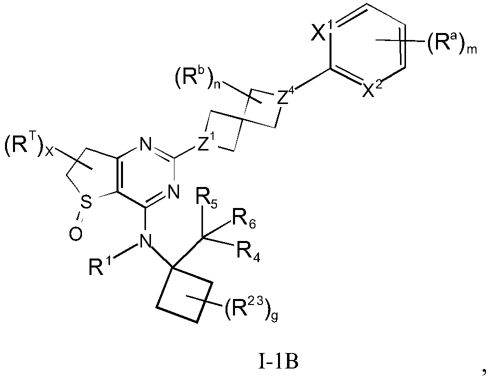 wherein:
X¹ and X² are each independently CH or N;
Z¹ and Z⁴ are each independently N or CH; and
R^{a}, R^{b}, R^{T}, R¹, R²³, R⁴, R⁵, R⁶, m, n, g, and x are as defined in claim 1.

10. The compound according to claim 1, wherein the compound has a structure represented by Formula I-1B1, Formula I-1B2, or Formula I-1B3: 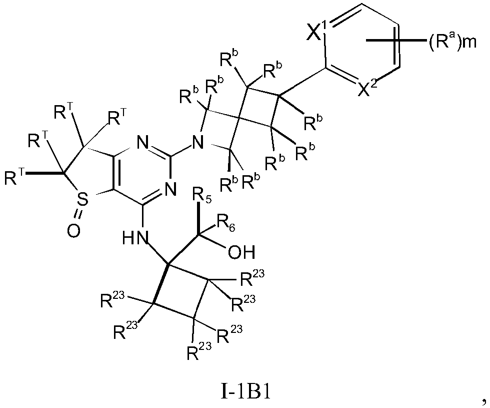 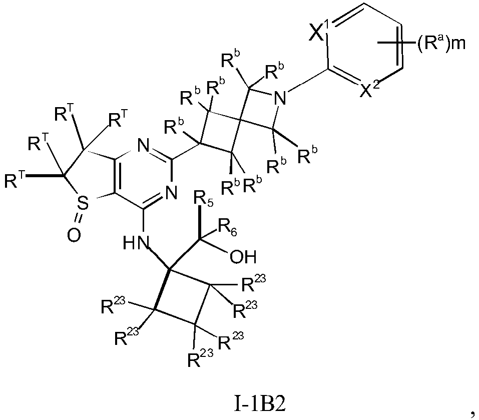 or 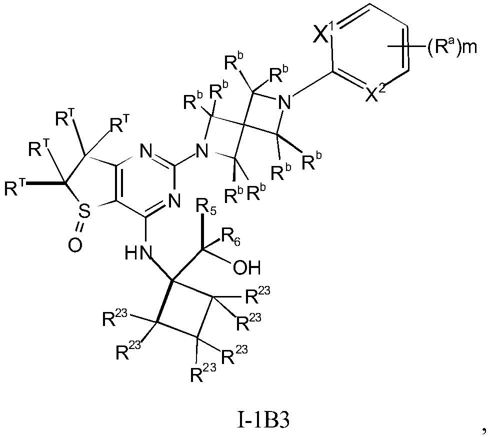 wherein:
X¹ and X² are each independently CH or N;
each R^{a} is independently H, halogen, hydroxyl, deuterium, C₁₋₆ alkyl, C₁₋₆ alkoxy, deuterated C₁₋₆ alkyl, or deuterated C₁₋₆ alkoxy;
each R^{T} is independently H, halogen, hydroxyl, deuterium, C₁₋₆ alkyl, C₁₋₆ alkoxy, deuterated C₁₋₆ alkyl, or deuterated C₁₋₆ alkoxy;
each R²³ is independently H, halogen, hydroxyl, deuterium, C₁₋₆ alkyl, C₁₋₆ alkoxy, deuterated C₁₋₆ alkyl, or deuterated C₁₋₆ alkoxy;
R⁵ and R⁶ are each independently H, deuterium, halogen, C₁₋₆ alkyl, deuterated C₁₋₆ alkyl, C₁₋₆ alkoxy, or deuterated C₁₋₆ alkoxy; and
each R^{b} is independently H, halogen, hydroxyl, deuterium, C₁₋₆ alkyl, C₁₋₆ alkoxy, deuterated C₁₋₆ alkyl, or deuterated C₁₋₆ alkoxy,
wherein at least one of R^{T}, R^{a}, R^{b}, R²³, R⁵, or R⁶ is deuterium, deuterated C₁₋₆ alkyl, or deuterated C₁₋₆ alkoxy.

11. The compound according to claim 10, wherein the compound has a structure represented by Formula I-1B4' or I-1B5': 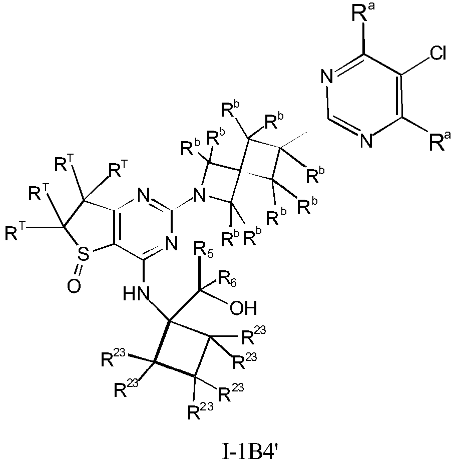 or 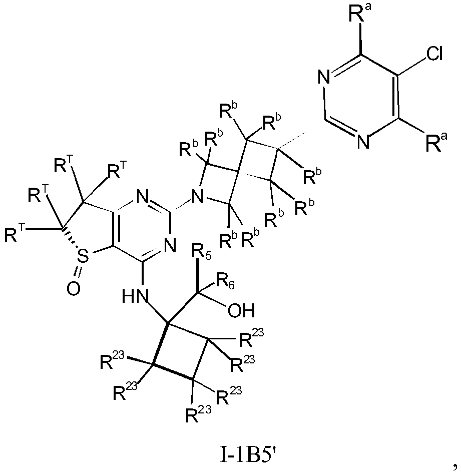 wherein:
each R^{a} is independently H, halogen, hydroxyl, deuterium, C₁₋₆ alkyl, C₁₋₆ alkoxy, deuterated C₁₋₆ alkyl, or deuterated C₁₋₆ alkoxy;
each R^{T} is independently H, halogen, hydroxyl, deuterium, C₁₋₆ alkyl, C₁₋₆ alkoxy, deuterated C₁₋₆ alkyl, or deuterated C₁₋₆ alkoxy;
each R²³ is independently H, halogen, hydroxyl, deuterium, C₁₋₆ alkyl, C₁₋₆ alkoxy, deuterated C₁₋₆ alkyl, or deuterated C₁₋₆ alkoxy;
R⁵ and R⁶ are each independently H, deuterium, halogen, C₁₋₆ alkyl, deuterated C₁₋₆ alkyl, C₁₋₆ alkoxy, deuterated C₁₋₆ alkoxy; and
each R^{b} is independently H, halogen, hydroxyl, deuterium, C₁₋₆ alkyl, C₁₋₆ alkoxy, deuterated C₁₋₆ alkyl, or deuterated C₁₋₆ alkoxy,
wherein at least one of R^{T}, R^{a}, R^{b}, R²³, R⁵, or R⁶ is deuterium, deuterated C₁₋₆ alkyl, or deuterated C₁₋₆ alkoxy.

12. The compound according to claim 11, wherein the compound has a structure represented by Formula I-1B4 or I-1B5: 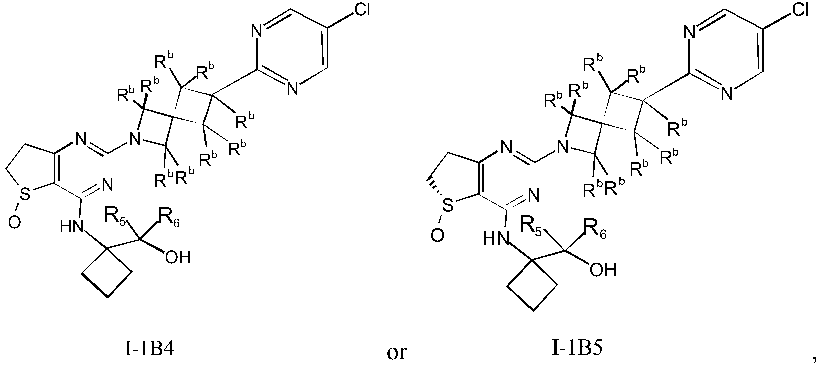 wherein:
R⁵ and R⁶ are each independently H, deuterium, halogen, C₁₋₆ alkyl, deuterated C₁₋₆ alkyl, C₁₋₆ alkoxy, or deuterated C₁₋₆ alkoxy; and
each R^{b} is independently H, halogen, hydroxyl, deuterium, C₁₋₆ alkyl, C₁₋₆ alkoxy, deuterated C₁₋₆ alkyl, or deuterated C₁₋₆ alkoxy,
wherein at least one of R^{b}, R⁵, and R⁶ is deuterium, deuterated C₁₋₆ alkyl, or deuterated C₁₋₆ alkoxy.

13. The compound according to claim 10, wherein:
each R^{T} is independently H or deuterium; and/or
at least one of R^{T} substituents is deuterium, and the remaining of R^{T} substituents are each H; and/or
each R²³ is independently H or deuterium; and/or
at least one of R²³ substituents is deuterium, and the remaining of R²³ substituents are each H; and/or
R⁵ and R⁶ are independently H or deuterium; and/or
at least one of R⁵ and R⁶ is deuterium, and the other one of R⁵ and R⁶ is H or deuterium; and/or
each R^{b} is independently H or deuterium; and/or
at least one of R^{b} is deuterium, and the remainder of R^{b} are each H; and/or
each R^{a} is independently H, deuterium, Cl or F; and/or
at least one of R^{a} is deuterium, and the remainder of R^{a} are each H, Cl, or F.

14. The compound according to claim 1, wherein the compound has any one of the following structures:

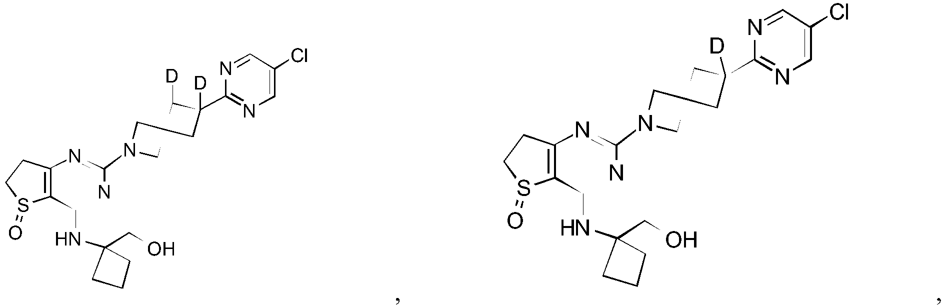

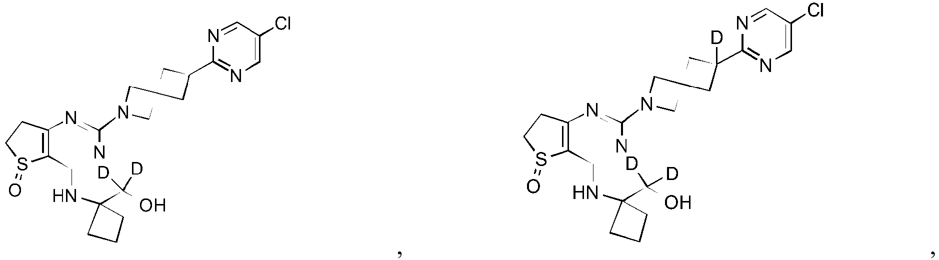

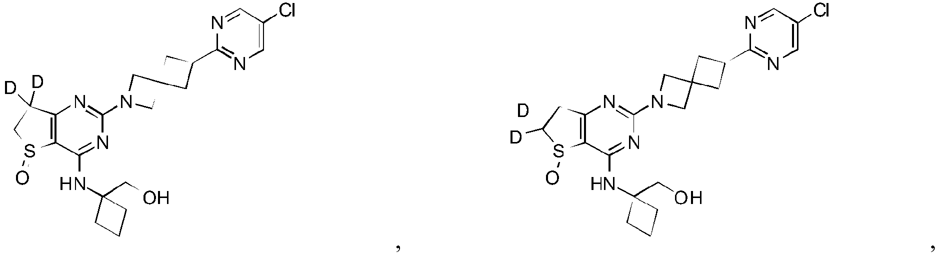

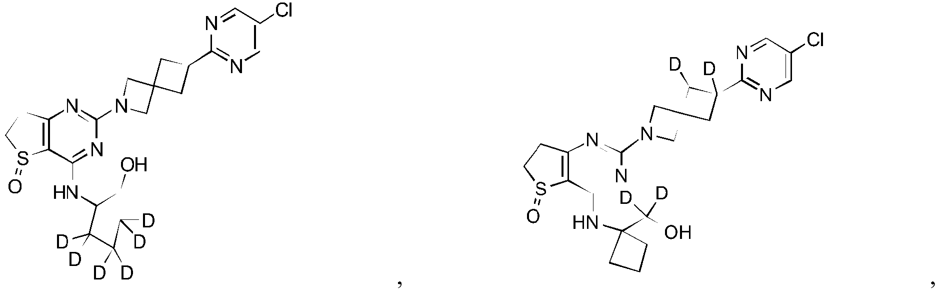

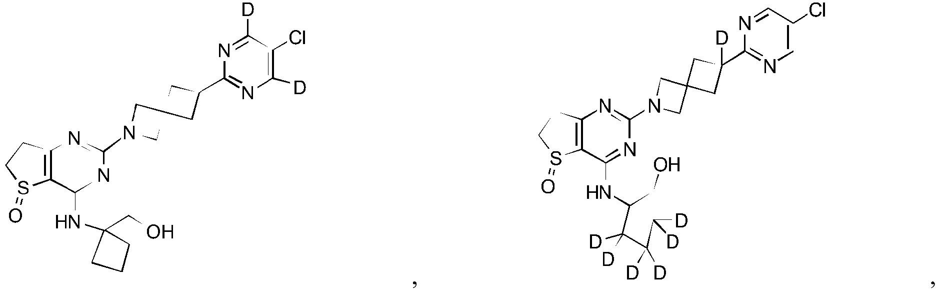

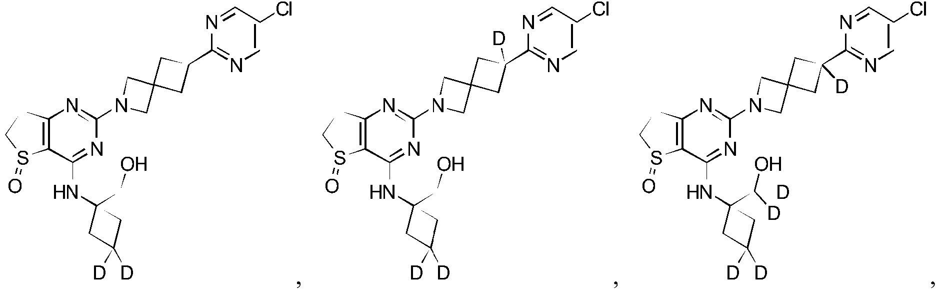

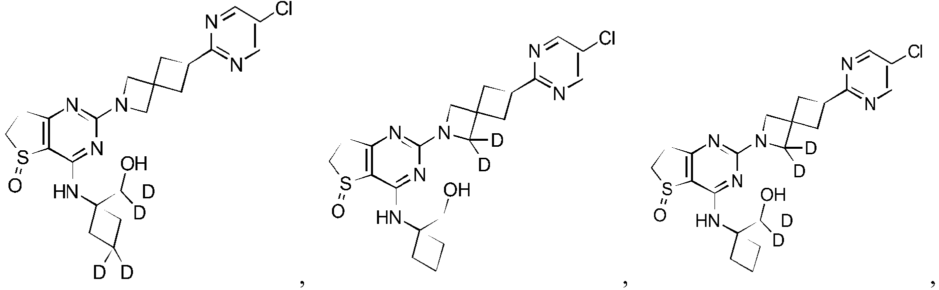

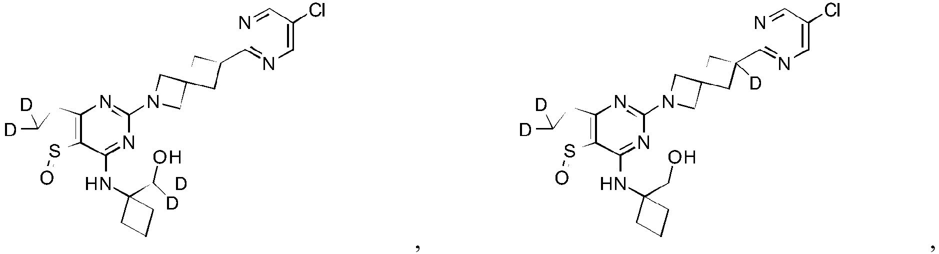

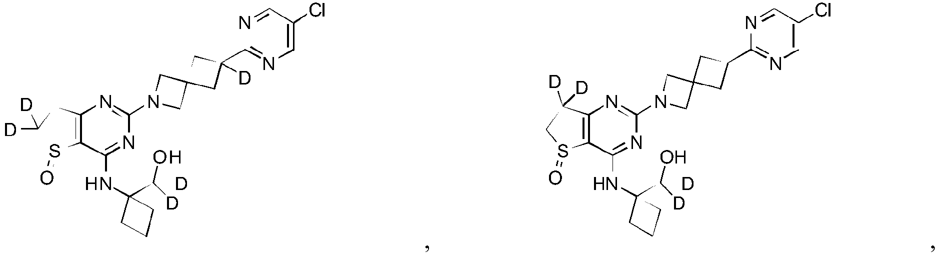

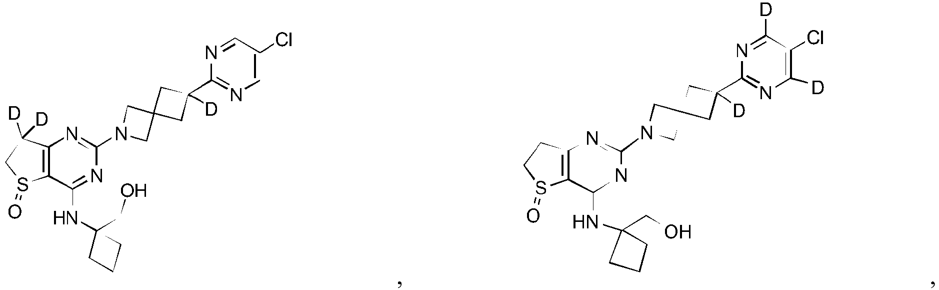

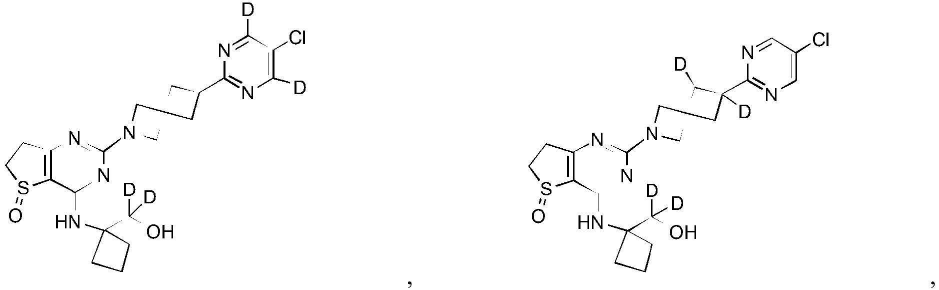

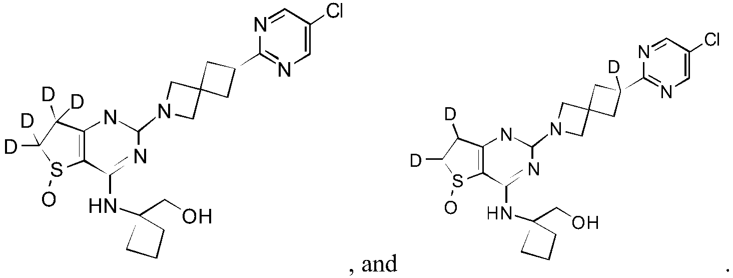

15. The compound according to claim 14, wherein the compound has any one of the following structures:

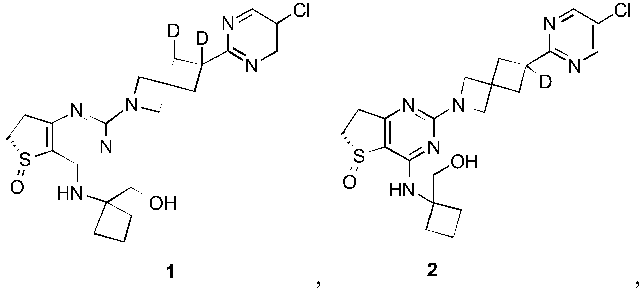

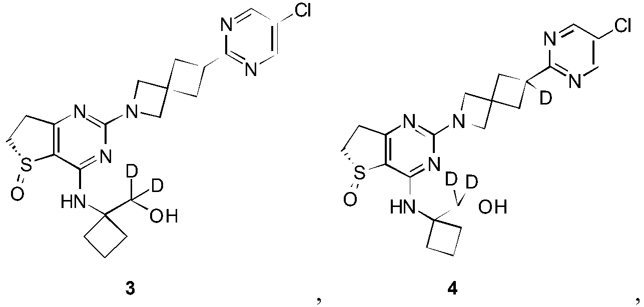

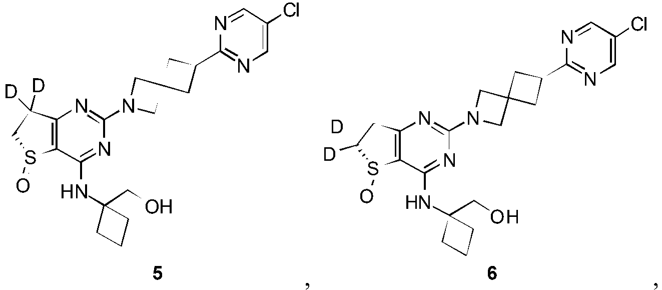

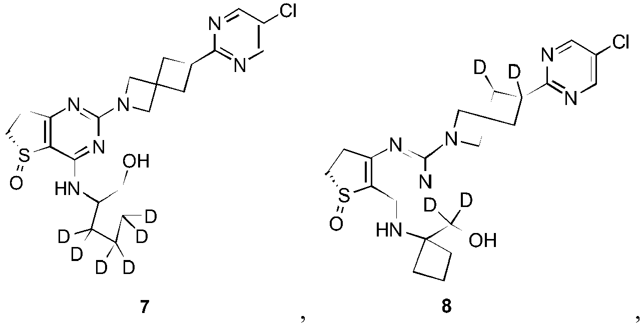

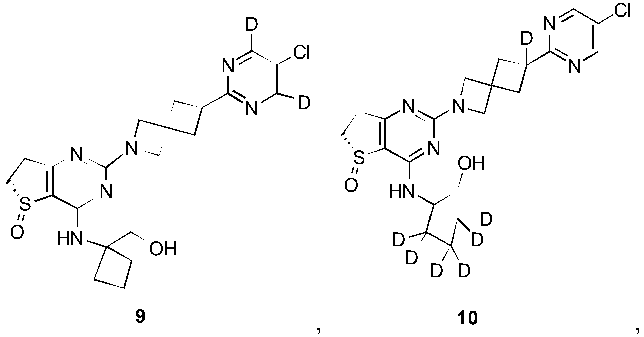

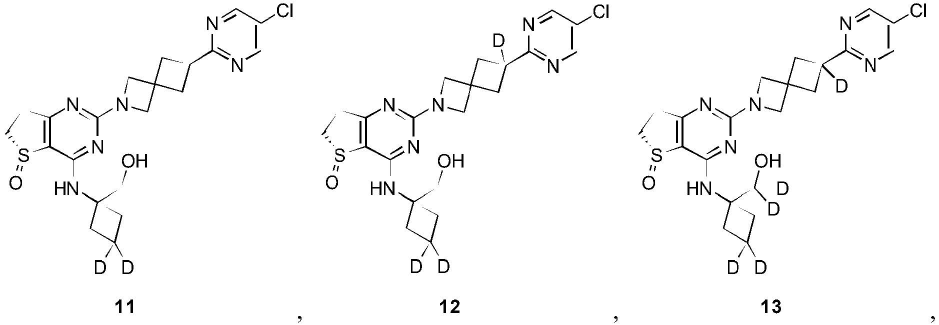

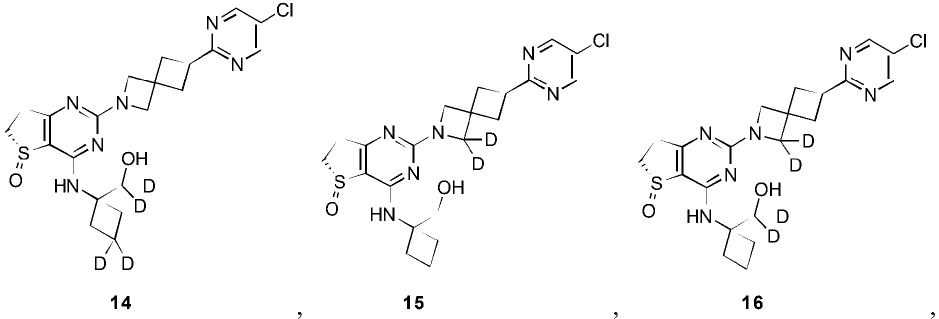

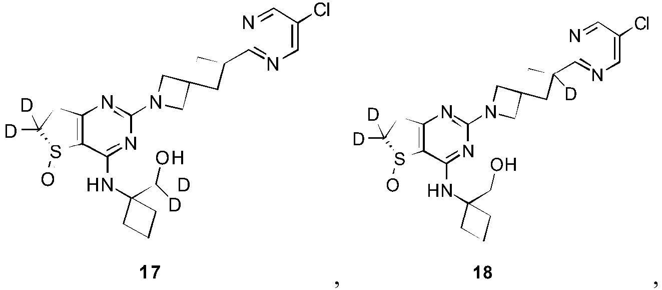

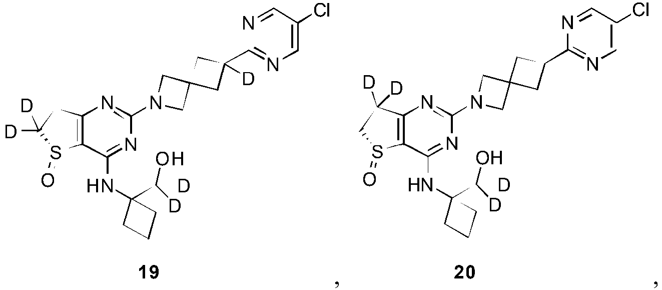

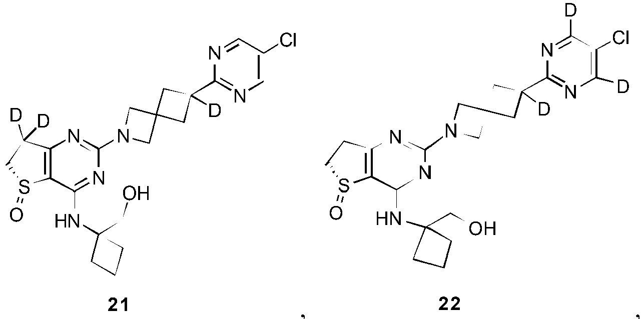

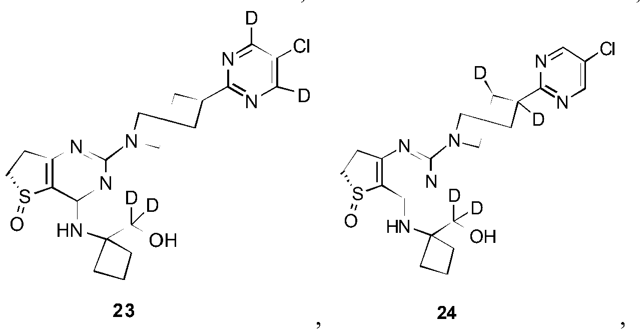

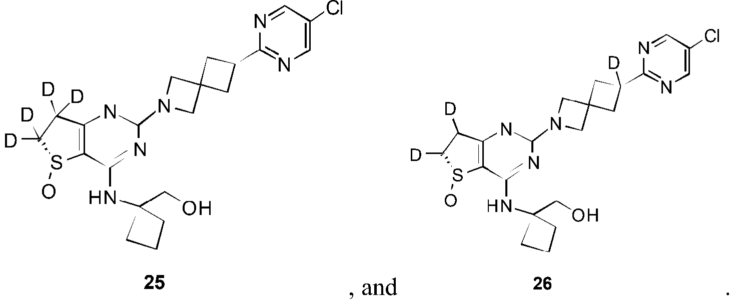

16. A pharmaceutical composition, comprising:
the compound, or the tautomer, stereoisomer, hydrate, solvate, pharmaceutically acceptable salt or prodrug thereof according to any one of claims 1 to 15; and/or
a pharmaceutically acceptable carrier or excipient.

17. Use of the compound, or the tautomer, stereoisomer, hydrate, solvate, pharmaceutically acceptable salt or prodrug thereof according to any one of claims 1 to 15, or the pharmaceutical composition according to claim 16 in the preparation of a medicament or a formulation for:
inhibiting PDE4B; and/or
preventing and/or treating a PDE4B-related disease; and/or
avoiding or reducing a gastrointestinal side effect in the treatment of a PDE4B-related disease.

18. The compound, or the tautomer, stereoisomer, hydrate, solvate, pharmaceutically acceptable salt or prodrug thereof according to any one of claims 1 to 15, or the pharmaceutical composition according to claim 16, for use in inhibiting PDE4B, and/or preventing and/or treating a PDE4B-related disease, and/or avoiding or reducing a gastrointestinal side effect in the treatment of a PDE4B-related disease.

19. A method for preventing and/or treating a PDE4B-related disease, and/or avoiding or reducing a gastrointestinal side effect in the treatment of a PDE4B-related disease, the method comprising:
administrating to a subject in need thereof the compound, or the tautomer, stereoisomer, hydrate, solvate, pharmaceutically acceptable salt or prodrug thereof according to any one of claims 1 to 15, or the pharmaceutical composition according to claim 16.

20. The use according to any one of claims 17 to 18 or the method according to claim 19, wherein the PDE4B-related disease comprises a respiratory disease, a gastrointestinal disease, an inflammatory disease of joints, skin, or eyes, a cancer, a peripheral or central nervous system disease, an autoimmune disease, a transplant rejection, or a disease related to smooth muscle contractility,
optionally, the respiratory disease is a respiratory or pulmonary disease accompanied by increased mucus production, airway inflammation, and/or obstructive disease;
optionally, the respiratory disease is COPD, idiopathic pulmonary fibrosis, interstitial lung disease, α1-antitrypsin deficiency, chronic sinusitis, asthma, or chronic bronchitis;
optionally, the autoimmune disease is a diffuse connective tissue disease such as systemic lupus erythematosus, rheumatoid arthritis, multiple sclerosis, dermatomyositis, polymyositis, vasculitis, or Sjögren's syndrome;
optionally, the gastrointestinal disease is regional ileitis, ulcerative colitis, or Crohn's disease;
optionally, the inflammatory disease of the joints, skin, or eyes is rheumatoid arthritis, sarcoidosis, dry eye syndrome, or glaucoma;
optionally, the cancer is mesothelioma, neuroblastoma, rectal cancer, colon cancer, familial adenomatous polyposis, hereditary non-polyposis colorectal cancer, esophageal cancer, lip cancer, laryngeal cancer, hypopharyngeal cancer, tongue cancer, salivary gland cancer, gastric cancer, adenocarcinoma, medullary thyroid carcinoma, papillary thyroid carcinoma, renal cancer, renal parenchymal cancer, ovarian cancer, cervical cancer, uterine cancer, endometrial cancer, choriocarcinoma, pancreatic cancer, prostate cancer, bladder cancer, testicular cancer, breast cancer, urological cancer, melanoma, brain tumor, lymphoma, headand-neck cancer, acute lymphoblastic leukemia, chronic lymphocytic leukemia, acute myeloid leukemia, chronic myeloid leukemia, hepatocellular carcinoma, gallbladder cancer, bronchial carcinoma, small cell lung cancer, non-small cell lung cancer, multiple myeloma, basal sarcoma, teratoma, retinoblastoma, choroidal melanoma, seminoma, rhabdomyosarcoma, osteosarcoma, chondrosarcoma, myoma, liposarcoma, fibrosarcoma, Ewing's sarcoma, and plasmacytoma; and
optionally, the peripheral or central nervous system disease is depression, bipolar depression or manic depression, acute and chronic anxiety states, schizophrenia, Alzheimer's disease, Parkinson's disease, acute and chronic multiple sclerosis or acute and chronic pain, and brain damage caused by stroke, hypoxia, or cranio-cerebral trauma.
